# EUROPEAN PATENT SPECIFICATION

(11) **EP 1 853 312 B1**
(45) Date of publication and mention of the grant of the patent: **10.12.2014**
(21) Application number: 06736808.4
(22) Date of filing: 02.03.2006
(51) Int. Cl.: A61K 39/395, A61P 35/00, A61P 37/02, A61K 38/17, C07K 16/28, C07K 14/715

(54) **METHODS AND COMPOSITIONS FOR MODULATING TWEAK AND FN14 ACTIVITY**
VERFAHREN UND ZUSAMMENSETZUNGEN ZUR MODULIERUNG VON TWEAK- UND FN14-AKTIVITÄT
METHODES ET COMPOSITIONS DE MODULATION DE L'ACTIVITE DE TWEAK ET DE FN14

(30) Priority: 07.03.2005 US 659339 P
(43) Date of publication of application: 14.11.2007
(73) Proprietor: Genentech, Inc., South San Francisco CA 94080-4990 (US)
(72) Inventor: ASHKENAZI, Avi, J., San Mateo, California 94402 (US); MAECKER, Heather, Palo Alto, California 94303 (US)
(74) Representative: Kiddle, Simon John
(86) International application number: PCT/US2006/007547
(87) International publication number: WO 2006/096487

(56) References cited:
- WO-A-01/53486
- WO-A2-2006/052926
- US-A1- 2002 015 703
- US-A1- 2004 253 610
- US-B1- 6 207 642
- KADUKA Y ET AL: "TWEAK mediates anti-tumor effect of tumor-infiltrating macrophage" BIOCHEMICAL AND BIOPHYSICAL RESEARCH COMMUNICATIONS, ACADEMIC PRESS INC. ORLANDO, FL, US, vol. 331, no. 2, 3 June 2005 (2005-06-03), pages 384-390, XP004867567 ISSN: 0006-291X
- MAECKER HEATHER ET AL: "TWEAK attenuates the transition from innate to adaptive immunity" CELL, vol. 123, no. 5, December 2005 (2005-12), pages 931-944, XP002403317 ISSN: 0092-8674
- MICHAELSON JENNIFER S ET AL: "Therapeutic targeting of TWEAK/Fnl4 in cancer: exploiting the intrinsic tumor cell killing capacity of the pathway", RESULTS AND PROBLEMS IN CELL DIFFERENTIATION, SPRINGER VERLAG, NEW YORK, NY, US LNKD- DOI:10.1007/400_2008_18, 1 January 2009 (2009-01-01), pages 145-160, XP008119975, ISSN: 0080-1844
- BURKLY ET AL: "TWEAKing tissue remodeling by a multifunctional cytokine: Role of TWEAK/Fn14 pathway in health and disease", CYTOKINE, ACADEMIC PRESS LTD, PHILADELPHIA, PA, US LNKD- DOI:10.1016/J.CYTO.2007.09.007, vol. 40, no. 1, 5 November 2007 (2007-11-05), pages 1-16, XP022344466, ISSN: 1043-4666
- NAKAYAMA M ET AL: "Fibroblast growth factor-inducible 14 mediates multiple pathways of TWEAK-induced cell death", THE JOURNAL OF IMMUNOLOGY, THE AMERICAN ASSOCIATION OF IMMUNOLOGISTS, US, vol. 170, no. 1, 1 January 2003 (2003-01-01), pages 341-348, XP002356992, ISSN: 0022-1767

## Description

### RELATED APPLICATIONS

This application claims priority to US provisional application no. 60/659,339 filed March 7, 2005.

### FIELD OF THE INVENTION

The present invention provides agonists and antagonists which modulate the activity of TWEAK and TWEAK receptor. More particularly, the invention provides methods, compositions and kits which may be employed to modulate the activity of TWEAK and/or TWEAK receptor on immune cells and for the treatment of disorders such as cancer and immune-related diseases.

### BACKGROUND OF THE INVENTION

Various ligands and receptors belonging to the tumor necrosis factor (TNF) superfamily have been identified in the art. Included among such ligands are tumor necrosis factor-alpha ("TNF-alpha"), tumor necrosis factor-beta ("TNF-beta" or "lymphotoxin-alpha"), lymphotoxin-beta ("LT-beta"), CD30 ligand, CD27 ligand, CD40 ligand, OX-40 ligand, 4-1BB ligand, LIGHT, Apo-1 ligand (also referred to as Fas ligand or CD95 ligand), Apo-2 ligand (also referred to as Apo2L or TRAIL), TWEAK (also referred to as Apo-3 ligand), APRIL, OPG ligand (also referred to as RANK ligand, ODF, or TRANCE), and TALL-1 (also referred to as BlyS, BAFF or THANK) (See, e.g., Ashkenazi, Nature Review, 2:420-430 (2002); Ashkenazi and Dixit, Science, 281:1305-1308 (1998); Ashkenazi and Dixit, Curr. Opin. Cell Biol., 11:255-260 (2000); Golstein, Curr. Biol., 7:750-753 (1997); Wallach, Cytokine Reference, Academic Press, 2000, pages 377-411; Locksley et al., Cell, 104:487-501 (2001); Gruss and Dower, Blood, 85:3378-3404 (1995); Schmid et al., Proc. Natl. Acad. Sci., 83:1881 (1986); Dealtry et al., Eur. J. Immunol., 17:689 (1987); Pitti et al., J. Biol. Chem., 271:12687-12690 (1996); Wiley et al., Immunity, 3:673-682 (1995); Browning et al., Cell, 72:847-856 (1993); Armitage et al. Nature, 357:80-82 (1992), WO 97/01633 published January 16, 1997; WO 97/25428 published July 17, 1997; Marsters et al., Curr. Biol., 8:525-528 (1998); Chicheportiche et al., J. Biol. Chem., 272:32401-32410 (1997); Hahne et al., J. Exp. Med., 188:1185-1190 (1998); WO98/28426 published July 2, 1998; WO98/46751 published October 22, 1998; WO/98/18921 published May 7, 1998; Moore et al., Science, 285:260-263 (1999); Shu et al., J. Leukocyte Biol., 65:680 (1999); Schneider et al., J. Exp. Med., 189:1747-1756 (1999); Mukhopadhyay et al., J. Biol. Chem., 274:15978-15981 (1999)).

Induction of various cellular responses mediated by such TNF family ligands is typically initiated by their binding to specific cell receptors. Included among the members of the TNF receptor superfamily identified to date are TNFR1, TNFR2, TACI, GITR,, CD27, OX-40, CD30, CD40, HVEM, Fas (also referred to as Apo-1 or CD95), DR4 (also referred to as TRAIL-R1), DR5 (also referred to as Apo-2 or TRAIL-R2), DcR1, DcR2, osteoprotegerin (OPG), RANK and Apo-3 (also referred to as DR3 or TRAMP) (see, e.g., Ashkenazi, Nature Reviews, 2:420-430 (2002); Ashkenazi and Dixit, Science, 281:1305-1308 (1998); Ashkenazi and Dixit, Curr. Opin. Cell Biol., 11:255-260 (2000); Golstein, Curr. Biol., 7:750-753 (1997) Wallach, Cytokine Reference, Academic Press, 2000, pages 377-411; Locksley et al., Cell, 104:487-501 (2001); Gruss and Dower, Blood, 85:3378-3404 (1995); Hohman et al., J. Biol. Chem., 264:14927-14934 (1989); Brockhaus et al., Proc. Natl. Acad. Sci., 87:3127-3131 (1990); EP 417,563, published March 20, 1991; Loetscher et al., Cell, 61:351 (1990); Schall et al., Cell, 61:361 (1990); Smith et al., Science, 248:1019-1023 (1990); Lewis et al., Proc. Natl. Acad. Sci., 88:2830-2834 (1991); Goodwin et al., Mol. Cell. Biol., 11:3020-3026 (1991); Stamenkovic et al., EMBO J., 8:1403-1410 (1989); Mallett et al., EMBO J., 9:1063-1068 (1990); Anderson et al., Nature, 390:175-179 (1997); Chicheportiche et al., J. Biol. Chem., 272:32401-32410 (1997); Pan et al., Science, 276:111-113 (1997); Pan et al., Science, 277:815-818 (1997); Sheridan et al., Science, 277:818-821 (1997); Degli-Esposti et al., J. Exp. Med., 186:1165-1170 (1997); Marsters et al., Curr. Biol., 7:1003-1006 (1997); Tsuda et al., BBRC, 234:137-142 (1997); Nocentini et al., Proc. Natl. Acad. Sci., 94:6216-6221 (1997); vonBulow et al., Science, 278:138-141 (1997)).

Most of these TNF receptor family members share the typical structure of cell surface receptors including extracellular, transmembrane and intracellular regions, while others are found naturally as soluble proteins lacking a transmembrane and intracellular domain. The extracellular portion of typical TNFRs contains a repetitive amino acid sequence pattern of multiple cysteine-rich domains (CRDs), starting from the NH₂-terminus.

The interaction of some TNF ligand family members with their respective receptor(s) can influence a variety of functions within the immune system. Examples of such ligand/receptor interactions include CD40 ligand which binds to the CD40 receptor to, e.g., promote the differentiation of B cells into antibody producing cells (Grewal et al., Immunol. Res., 16:59-70 (1997)), lymphotoxin-beta ligand which binds to the lymphotoxin-beta receptor to, e.g., influence humoral immune responses by regulating the differentiation state of follicular dendritic cells (Mackay and Browning, Nature, 395:26-27 (1998)), and OX40 ligand which binds the OX40 receptor to, e.g., regulate the response of B cells to T cell signals (Flynn et al., J. Exp. Med., 188:297-304 (1998)). Other ligand/receptor pairs which have been reported to play roles in the immune system include TNF-alpha/TNFR-1 and Fas ligand/Fas.

The TNF family ligand referred to as "TWEAK" or "Apo-3 ligand" has been described in the literature (see, e.g., WO98/05783; WO98/35061; WO99/19490; US2002/0015703). The TWEAK ligand was reported in the literature as a relatively weak inducer of apoptosis in transformed cell lines (Chicheportiche et al., J. Biol. Chem., 272:32401-32410 (1997); Marsters et al., Curr. Biol., 8:525-528 (1998)). Purified soluble TWEAK protein was used to induce the differentiation and/or death of some tumor cell lines, including HT29 adenocarcinoma cells, HeLa cervical carcinoma cells, and A375 melanoma cells. TWEAK also induced the HT29 and A375 cell lines to secrete the chemokine IL-8 and had the same effect on a fibroblast cell line, WI-38 (Chicheportiche et al., J. Biol. Chem., 272:32401-32410 (1997)). In addition, TWEAK has been implicated in angiogenic regulation by inducing proliferation of a variety of normal endothelial cell lines and angiogenesis in rat corneas (Lynch et al., J. Interferon Cytokine Res. 18: A-46 (1998)); Jakubowski et al., J. Cell. Sci., 115:267-274 (2002); Lynch et al., J. Biol. Chem., 274:8455-8459 (1999)).

Expression of TWEAK mRNA in mouse and human tissues such as heart, brain, lung, liver, among other tissues, and secondary lymphoid organs such as spleen, and lymph nodes has been described. TWEAK is also expressed on human peripheral blood monocytes and its expression increases following IFN-gamma stimulation (Nakayama et al., J. Exp. Med., 192:1373-1380 (2000)).

A putative receptor for TWEAK was previously described in the literature (Marsters et al., Curr. Biol. 8: 525-528 (1998)). This receptor, referred to as TRAMP, Apo-3, WSL-1, DR3, or LARD, is a member of the TNFR family. Activation of TRAMP was reported to induce apoptosis by engaging either the caspase-dependent cell death signaling pathway or cellular activation via NF-kB signaling pathways (Ashkenazi and Dixit, Science, 281:1305-1308 (1998)). Presently, it is believed that TRAMP/Apo-3/DR3 may indeed not be a physiological, high affinity receptor for TWEAK.

Another receptor which binds TWEAK, called Fn-14, has also been identified. Fn-14 is a fibroblast growth factor-inducible 14-kDa protein (Wiley et al., Immunity, 15:837-846 (2001)), and is a distantly related TNFR family member which contains only one cysteine-rich domain in the extracellular domain along with a TRAF binding motif in the intracellular domain. TWEAK, acting through this receptor, induces NF-KB2 p100 processing and long lasting NF-KB activation (Saitoh et al., J. Biol. Chem., 278:36005-36012 (2003)).

US 6 207 642 B1, Nakayama at al., Journal of Immunology, 2003, 170: 341-348, and US 2004/0253610 A1 describe various TWEAK antagonists for use in the treatment of cancer which either induce apoptosis in tumor cells or inhibit angiogenesis.

### SUMMARY OF THE INVENTION

The TNF ligand family member, TWEAK, is believed to act as a proinflammatory cytokine. As shown in the examples below, TWEAK was found to play an important role in curtailing the innate inflammatory response as well as the transition from innate to T_{H1} adaptive immunity. Accordingly, by modulating such activity(s) in either an agonistic or antagonistic manner, various disorders such as cancer or immune related conditions may be treated.

The present invention provides compositions for use in treating cancer which bind TWEAK and/or TWEAK receptor and modulate the activity or TWEAK and/or TWEAK receptor in, an antagonist manner as defined in the claims. A TWEAK or TWEAK receptor antagonist may be employed, e.g., to block or neutralize the activity of TWEAK and/or TWEAK receptor. Such compositions, and methods using the compositions, can be employed to treat a variety of disorders, including cancer and autoimmune disorders. By way of example, antagonistic antibodies which bind TWEAK and neutralize or block the activity of TWEAK on immune cells, can be used to enhance the effects and numbers of natural killer (NK) cells in a mammal to inhibit pathologies associated with excessive innate and/or adaptive immune system disorders. Compositions for use according to the invention include monoclonal antibodies which bind TWEAK and/or TWEAK receptor and soluble TWEAK-receptor-Ig fusion proteins, which can antagonize the activity of TWEAK and/or TWEAK receptor.

The invention provides a TWEAK antagonist for use in methods for treating a disorder which is cancer, the method comprising administering a composition which comprises a therapeutically effective amount of a TWEAK antagonist and an acceptable carrier as defined in the claims. The TWEAK antagonist may be an antibody directed against a TWEAK ligand; or an antibody directed against a TWEAK receptor. In a preferred embodiment the antibody is a monoclonal antibody. In a more preferred embodiment the monoclonal antibody is directed against the TWEAK ligand. The TWEAK antagonist may be a soluble TWEAK receptor having a ligand binding domain that can selectively bind to a TWEAK ligand. In one embodiment the soluble TWEAK receptor may include a human immunoglobulin IgG domain.

The invention relates to methods for enhancing innate T_{H}1 responses or activity in a mammal, including administering a composition which comprises an effective amount of a TWEAK antagonist, and optionally a pharmaceutically effective carrier.

The invention relates to methods for enhancing NK cell activity in a mammal, including administering a composition which comprises an effective amount of a TWEAK antagonist, and optionally a pharmaceutically effective carrier.

As described herein, a TWEAK or TWEAK receptor agonist may be employed, e.g., to stimulate or enhance the activity of TWEAK and/or TWEAK receptor. Compositions which bind TWEAK and/or TWEAK receptor and stimulate or enhance the activity of TWEAK and/or TWEAK receptor are described herein. Such compositions, and methods using the compositions, can be employed to treat a variety of disorders, including immune-related diseases such as autoimmune diseases. By way of example, agonistic antibodies which bind TWEAK receptor and stimulate or enhance the activity of TWEAK receptor can be used to ameliorate T_{H}1-driven autoimmune diseases such as Crohn's Disease, inflammatory bowel disease, multiple sclerosis, and arthritis.

The disclosure also concerns methods for treating an immune-related condition, comprising administering a composition which comprises a therapeutically effective amount of a TWEAK or TWEAK receptor agonist and an acceptable carrier. The TWEAK agonist may be an antibody directed against a TWEAK receptor. Preferably the antibody is a monoclonal antibody. More preferably, the monoclonal antibody is directed against the TWEAK receptor.

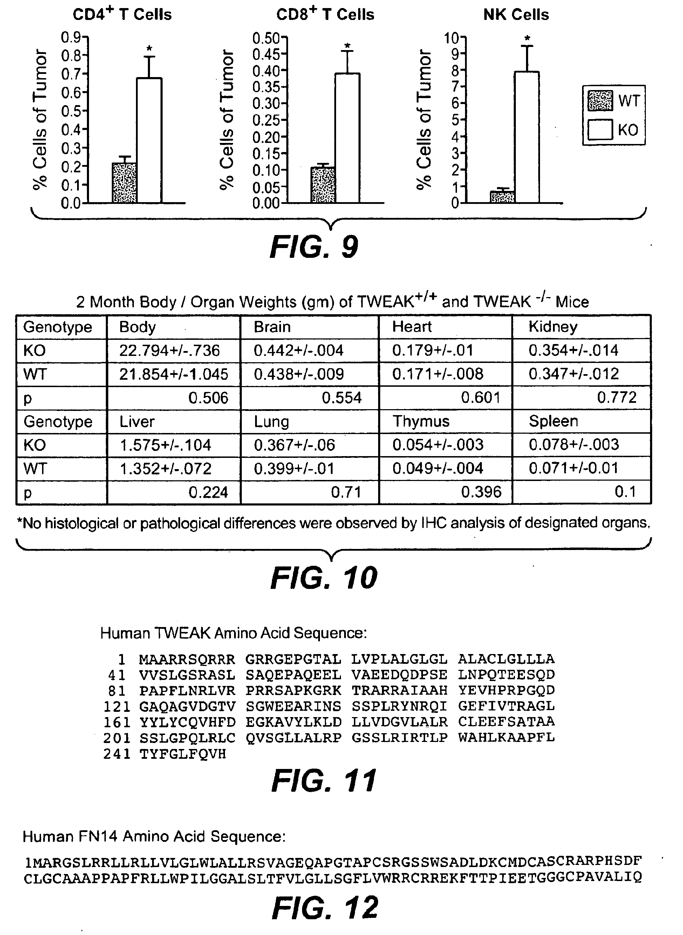

The following subject matter is described herein:
1. A method of treating cancer, comprising exposing mammalian cancer cells to an effective amount of an antagonist molecule, wherein said antagonist is selected from the group consisting of
   a) anti-TWEAK antibody;
   b) anti-TWEAK receptor antibody;
   c) TWEAK receptor immunoadhesin; and
   d) agent or molecule which blocks or interrupts intracellular signaling of TWEAK receptor.
2. The method of para. 1, wherein said TWEAK receptor immunoadhesin comprises a TWEAK receptor sequence fused to a Fc region of an immunoglobulin.
3. The method of para. 2, wherein said TWEAK receptor sequence comprises an extracellular domain sequence of the FN14 receptor.
4. The method of para. 1, wherein said anti-TWEAK antibody binds human TWEAK polypeptide comprising amino acids 94-249 of Figure 11.
5. The method of para. 4, wherein said anti-TWEAK antibody is a chimeric, humanized or human antibody.
6. The method of para. 1, wherein said anti-TWEAK receptor antibody binds the human FN14 receptor polypeptide comprising the amino acid sequence of Figure 12.
7. The method of para. 6, wherein said anti-TWEAK receptor antibody is a chimeric, humanized or human antibody.
8. The method of para. 1, wherein said mammalian cancer cells are also exposed to chemotherapy, radiation, prodrug, cytotoxic agent, or growth inhibitory agent.
9. A method of enhancing NK cell activity in a mammal, comprising administering to said mammal to an effective amount of an antagonist molecule, wherein said antagonist is selected from the group consisting of
   e) anti-TWEAK antibody;
   f) anti-TWEAK receptor antibody;
   g) TWEAK receptor immunoadhesin; and
   h) agent or molecule which blocks or interrupts intracellular signaling of TWEAK receptor.
10. The method of para. 9, wherein said TWEAK receptor immunoadhesin comprises a TWEAK receptor sequence fused to a Fc region of an immunoglobulin.
11. The method of para. 10, wherein said TWEAK receptor sequence comprises an extracellular domain sequence of the FN14 receptor.
12. The method of para. 9, wherein said anti-TWEAK antibody binds to human TWEAK polypeptide comprising amino acids 94-249 of Figure 11.
13. The method of para. 12, wherein said anti-TWEAK antibody is a chimeric, humanized or human antibody.
14. The method of para. 9, wherein said anti-TWEAK receptor antibody binds the human FN14 receptor polypeptide comprising the amino acid sequence of Figure 12.
15.The method of para. 14, wherein said anti-TWEAK receptor antibody is a chimeric, humanized or human antibody.
16. A method of enhancing innate T_{H}1 responses or activity in a mammal, comprising administering to said mammal an effective amount of an antagonist molecule, wherein said antagonist is selected from the group consisting of
   i) anti-TWEAK antibody;
   j) anti-TWEAK receptor antibody;
   k) TWEAK receptor immunoadhesin; and
   1) agent or molecule which blocks or interrupts intracellular signaling of TWEAK receptor.
17. The method of para. 16, wherein said TWEAK receptor immunoadhesin comprises a TWEAK receptor sequence fused to a Fc region of an immunoglobulin.
18. The method of para. 17, wherein said TWEAK receptor sequence comprises an extracellular domain sequence of the FN14 receptor.
19. The method of para. 16, wherein said anti-TWEAK antibody binds the human TWEAK polypeptide comprising amino acids 94-249 of Figure 11.
20. The method of para. 19, wherein said anti-TWEAK antibody is a chimeric, humanized or human antibody.
21. The method of para. 16, wherein said anti-TWEAK receptor antibody binds the human FN14 receptor polypeptide comprising the amino acid sequence of Figure 12.
22. The method of para. 21, wherein said anti-TWEAK receptor antibody is a chimeric, humanized or human antibody.
23. A method of treating a T_{H}2 mediated disorder in a mammal, comprising administering to said mammal an effective amount of an antagonist molecule, wherein said antagonist is selected from the group consisting of
   m) anti-TWEAK antibody;
   n) anti-TWEAK receptor antibody;
   o) TWEAK receptor immunoadhesin; and
   p) agent or molecule which blocks or interrupts intracellular signaling of TWEAK receptor.
24. The method of para. 23, wherein said TWEAK receptor immunoadhesin comprises a TWEAK receptor sequence fused to a Fc region of an immunoglobulin.
25. The method of para. 24, wherein said TWEAK receptor sequence comprises an extracellular domain sequence of the FN14 receptor.
26. The method of para. 23, wherein said anti-TWEAK antibody binds the human TWEAK polypeptide comprising amino acids 94-249 of Figure 11.
27. The method of para. 26, wherein said anti-TWEAK antibody is a chimeric, humanized or human antibody.
28. The method of para. 23, wherein said anti-TWEAK receptor antibody binds the human FN14 receptor polypeptide comprising the amino acid sequence of Figure 12.
29. The method of para. 28, wherein said anti-TWEAK receptor antibody is a chimeric, humanized or human antibody.
30. The method of para. 23, wherein said T_{H}2 mediated disorder is allergy or asthma.
31. A method of treating an immune-related disorder, comprising administering to a mammal an effective amount of an agonist molecule, wherein said agonist is selected from the group consisting of:
   a) anti-TWEAK receptor antibody;
   b) TWEAK polypeptide; and
   c) TWEAK polypeptide variant.
32. The method of para. 31, wherein said anti-TWEAK receptor antibody binds the human FN14 receptor polypeptide comprising the amino acid sequence of Figure 12.
33. The method of para. 32, wherein said anti-TWEAK receptor antibody is a chimeric, humanized or human antibody.
34. The method of para. 31, wherein said immune-related disorder is an auto-immune disease.
35. The method of para. 34, wherein said auto-immune disease is Crohn's disease, inflammatory bowel disease, multiple sclerosis, or arthritis.

### BRIEF DESCRIPTION OF THE FIGURES

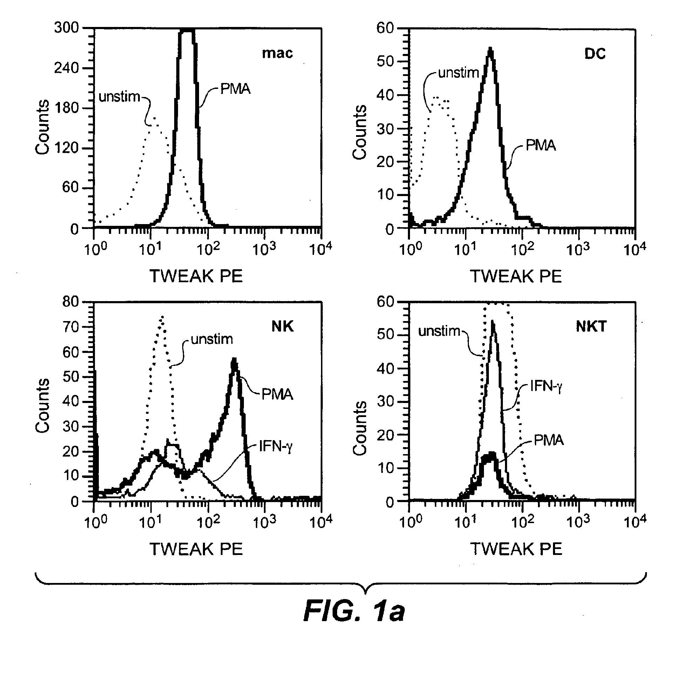

Figures 1A-1B. TWEAK, and its receptor FN14, are expressed on cells of the innate immune system. (1A) Human PBMCs, both resting ("unstim"), and activated for 12 hours with either IFN-gamma or PMA, were surface-stained with antibodies to lymphocyte lineage markers, permeabilized, stained with TWEAK antibody and analyzed by FACS. (macrophages ("mac"), dendritic cells ("DC"), NK cells, and NKT cells). (1B) Human PBMC, both resting and activated, were surfaced stained for the TWEAK receptor, FN14.

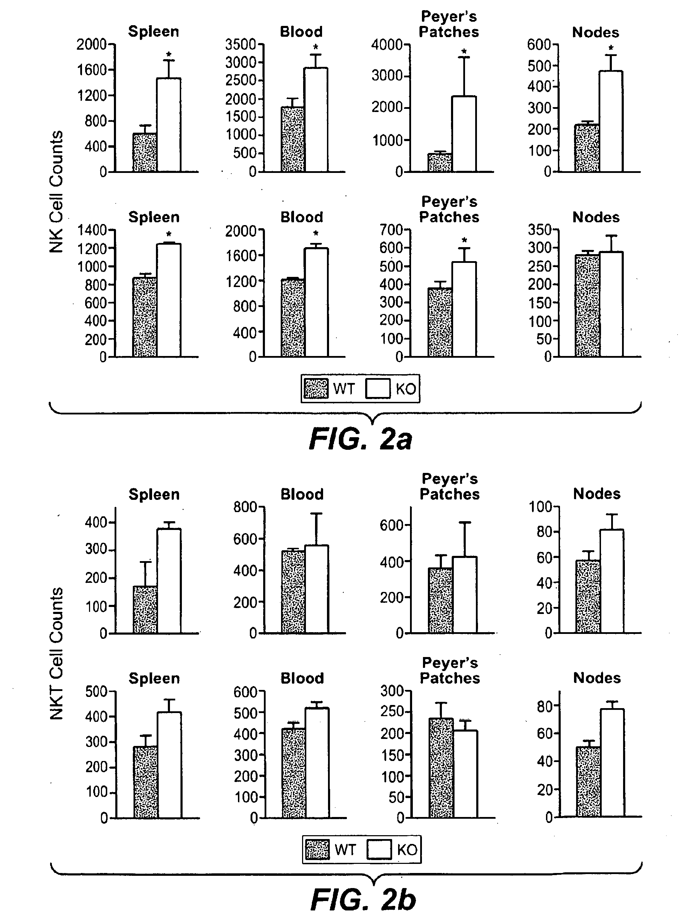

Figures 2A-2D. TWEAK KO mice have greater numbers of NK cells in secondary hematopoietic tissues. (2A, 2B) The spleen, peripheral blood, Peyer's patches, and lymph nodes were isolated from two-month old *TWEAK*^{+/+} mice (black bars) or *TWEAK*^{*-*/*-*} mice (white bars) (n=6 per group), dissociated, NK cells (a) or NKT cells (b) were quantified by FACS analysis. (Top graphs: males; bottom graphs: females). (2C) The bone marrow (0.5 mL) was aspirated from the right femurs of *TWEAK*^{+/+} mice (black bars) or *TWEAK*^{-/-} mice (white bars) (n=6 per group) (left graph: males; right graph: females) and NK cells were quantified. (2D) Human PBMCs were isolated from whole blood and subjected to activation-induced cell death by stimulation with TNF-alpha, LPS, or IFN-gamma, in the presence of various concentrations of FN14 Fc (closed squares), anti-TWEAK mAb (open squares), EDAR Fc (closed circles), or anti-CD4 mAb (open circles). NK cells were then isolated and stained for their sub-G1 content.

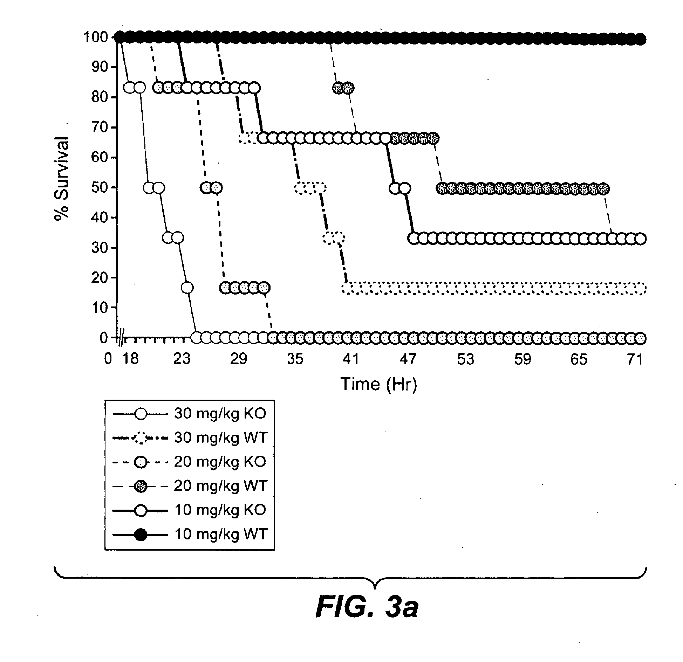

Figures 3A-3C. TWEAK ablation or inhibition augments the innate inflammatory response to endotoxin. (3A) *TWEAK*^{*+*/*+*} and *TWEAK*^{*-*/*-*} mice (n=10 per group) were injected i.p. with the indicated doses of LPS and viability was monitored over a 5 day period. (3B) NK cells and macrophages were isolated from the peripheral blood and spleen of *TWEAK*^{*+*/*+*} and *TWEAK*^{*-*/*-*} mice 24 hours after *in vivo* challenge with LPS (30 mg/kg) and stained for intracellular levels of IFN-gamma, IL-12, and IL-10. (3C) PBMCs from four human donors were stimulated for 24 hours with LPS. Subsequently, NK cells or macrophages (identified by lineage markers) were stained for intracellular levels of IFN-gamma and IL-12, respectively.

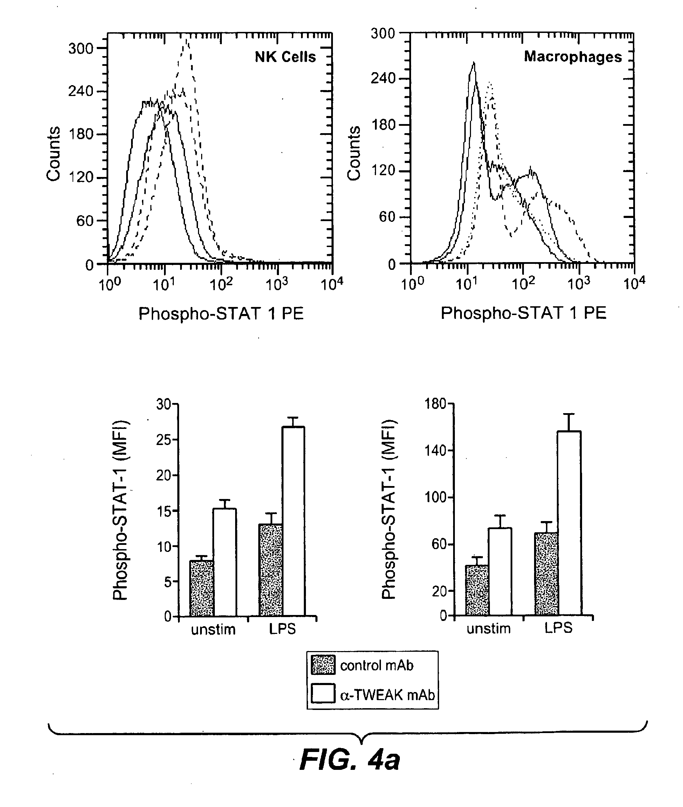

Figures 4A-4C. Involvement of TWEAK in modulation of STAT-1 and NF-κB1. (4A) Analysis of STAT-1 activation. Human NK cells and macrophages were stimulated for 12 hours *in vitro* with LPS (1 µg/mL), surface-stained for lineage markers, permeabilized, and stained for intracellular levels of phosphorylated STAT-1. The top panels depict NK cells and the bottom panels depict macrophages (with the FACS histograms summarized as bar graphs on the right). (4B) Analysis of NF-κB1 phosphorylation. Splenic human NK cells and macrophages were stimulated with TWEAK or TNF-alpha (100 ng/mL) over 24 hours. Cell lysates were prepared at the indicated time points and analyzed for phosphorylated p65 NF-κB1 by immunoblot. (4C) Analysis of NF-κB1 interactions. NF-κB1 was immunoprecipitated through p65 from lysates of TWEAK- or TNF-alpha-stimulated cells and the immunoprecipitates were analyzed by immunoblot for the presence of p300 and HDAC-1.

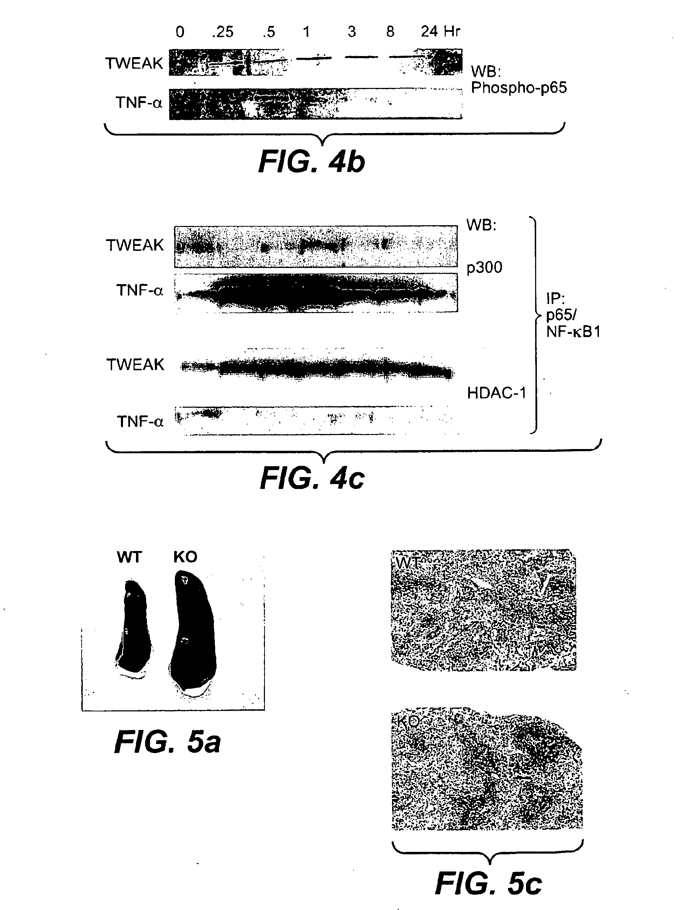

Figures 5A-5E. Aged *TWEAK*^{*-*/*-*} mice have larger spleens with expanded memory and T_{H}1 cell compartments. *TWEAK*^{*-*/*+*} and *TWEAK*^{*-*/*-*} male mouse littermates were grown to 3-, 6-, or 12 months of age, and their spleens and lymph nodes were examined. (5A) Representative images of a spleen from a *TWEAK*^{*+*/*+*} and a *TWEAK*^{*-*/*-*} mouse. (5B) Mean spleen weights as a function of age (n=6 per group). (5C) Representative images of spleen sections from a 12-month old *TWEAK*^{*+*/*+*} and *TWEAK*^{*-*/*-*} mouse stained with CD3 antibody. (5D,5E) Splenocytes from 12-month old wild type mice and *TWEAK* KO littermates were analyzed by FACS to determine the numbers of CD3⁺, CD4⁺, and CD8⁺ T cells (5D) and of memory and T_{H}1 T cells (5E).

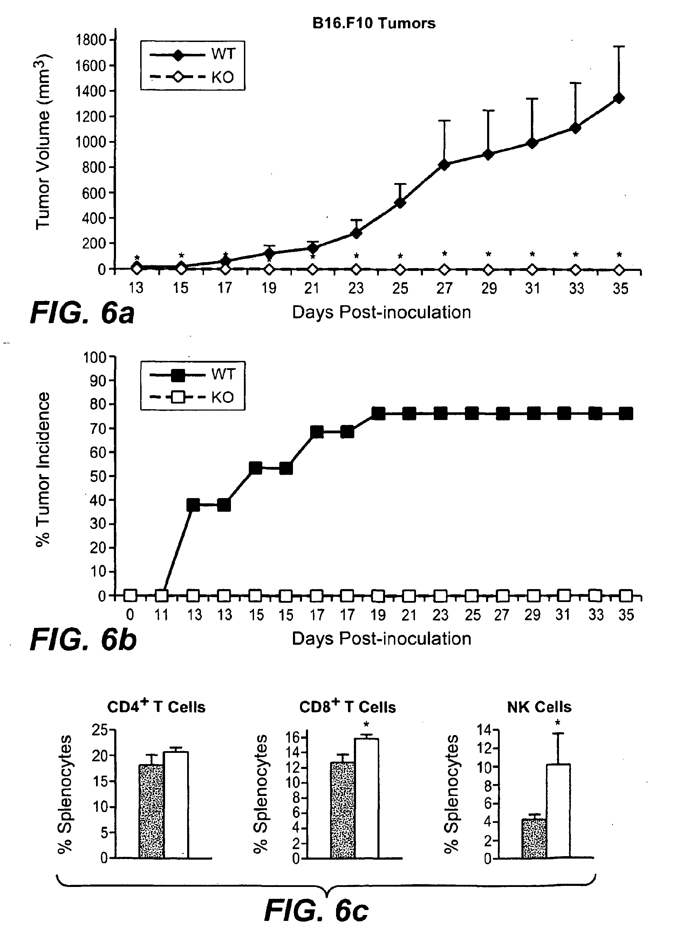

Figures 6A-6C. *TWEAK* deletion inhibits establishment and growth of B16.F10 melanomas and promotes expansion of adaptive CD8⁺ T cells. *TWEAK*^{*+*/*+*} and *TWEAK*^{*-*/*-*} mice were injected s.c. with 100,000 B16.F10 cells and tumor growth (A) or incidence (B) were monitored over 6 weeks (6A, 6B). At study termination, spleens were harvested from the injected mice and analyzed for the indicated lymphocyte subsets (6C).

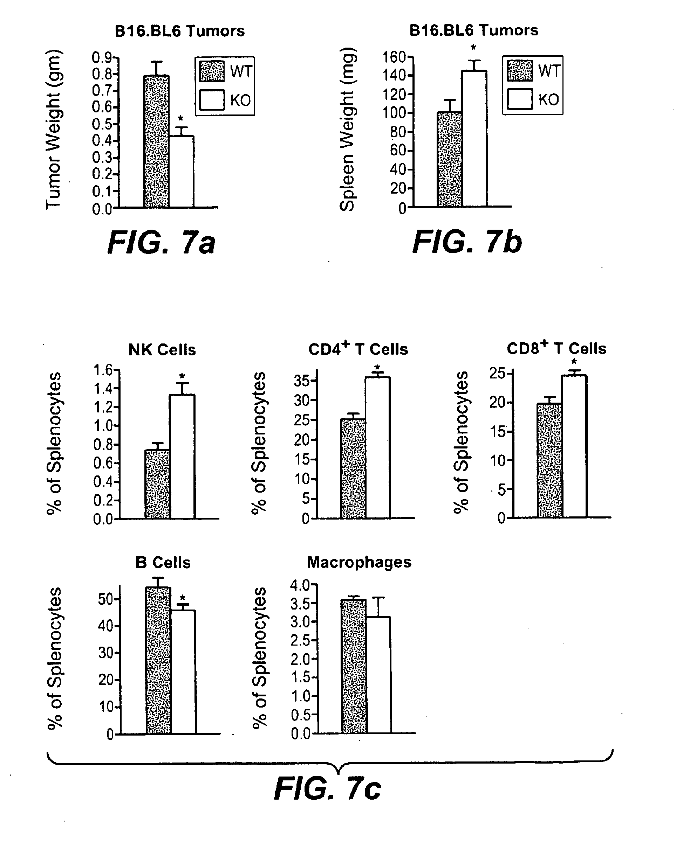

Figures 7A-7E. *TWEAK* deletion inhibits B16.BL6 tumor growth and promotes innate to adaptive priming of an anti-tumor immune response. TWEAK^{+/+} and *TWEAK*^{*-*/}*⁻mice* were injected s.c. with 500,000 B16.BL6 cells and tumor weights (7A) or spleen weights (7B) were determined at one month. (7C) Splenocytes from tumor-bearing mice were stained for various lineage populations and analyzed by FACS. (7D) NK cells and macrophages isolated from tumor-bearing mice were analyzed for cytokine production by intracellular staining and FACS. (7E) CD4⁺ and CD8⁺ T cells from tumor-bearing mice were similarly analyzed for IFN-gamma production. (*) denotes basal cytokine statistical significance (p<0.01); (**) denotes tumor-induced cytokine statistical significance (p<0.01).

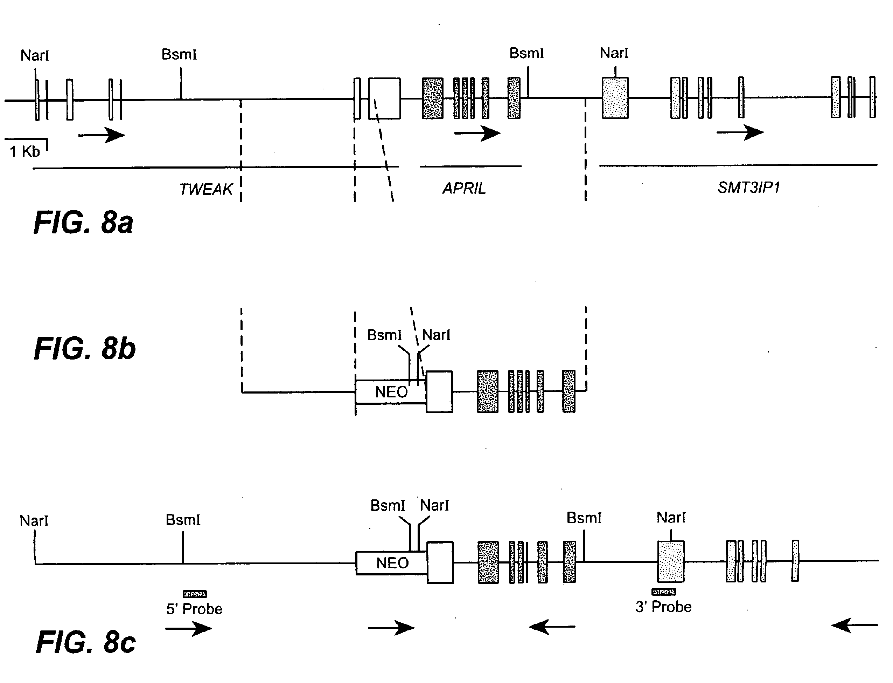

Figures 8A-8G. Characterization of TWEAK^{-/-} mouse. (8A) Structure of the mouse TWEAK genomic locus. Boxes correspond to the genomic regions containing the TWEAK (white bars), APRIL (black bars) and SMT3IP1 (grey bars) genes. The orientations of the three genes are marked by arrows. (8B) Schematic representation of the targeting construct designed to replace the coding sequences of exons six and seven of the TWEAK gene with a neo cassette. (8C) Structure of the mutated region in the TWEAK gene. The positions of the 5' and 3' external probes used for Southern blot analysis of ES cells are indicated by bars. The positions of the primer sets used for genotype analysis of mouse tail DNA are indicated by black (external) and grey (internal) arrowheads. (8D) Southern blot analysis of recombination of the TWEAK gene. Analysis of BsmI (DI) and NarI (DII) digested DNA derived from several ES cell clones. DNA was digested and fractionated on a 0.7% agarose gel, blotted onto a nylon membrane, and hybridized with 5' (DI) and 3' (DII) probes. (8E) Genotyping of TWEAK^{-/-} mice by PCR. Tail-derived genomic DNA was subjected to PCR amplification with nested external and internal sets of primers to visualize wild-type or deletion-mutant TWEAK genes as 4.3 kB or 5.3 kB fragments, respectively. (8F) Expression of TWEAK in total splenocytes derived from TWEAK^{+/+} and TWEAK^{-/-} mice as determined by FACS using anti-mouse TWEAK monoclonal antibody (black), or an isotype control (grey line and filled area). (8G) Quantitative real-time PCR analysis of TWEAK (white bars), APRIL (black bars), and SMT3IP1 (grey bars) mRNA expression in spleens of TWEAK^{+/+}, TWEAK^{+/-}, and TWEAK^{-/-} mice. All values were normalized to an RPL19 RNA internal control. Standard deviations were calculated from triplicate reactions.
Figure 9. TWEAK ^{-/-} mice have greater tumor lymphocytic infiltrate. B16.BL6 tumors were collected from TWEAK^{+/+} and TWFAK^{-/-} mice at 1 month, dissociated, and RBCs were lysed. After Fc blocking, dissociated tumor cells were stained for lymphocyte lineage markers and analyzed by FACS. Black bars represent the designated tumor lymphocyte infiltrate of TWEAK^{+/+} mice; white bars represent the designated tumor lymphocyte infiltrate of TWEAK^{-/-} mice.
Figure 10. Table showing 2 month old body/organ weights (gm) of TWEAK +/+ and TWEAK -/- mice.
Figure 11. Amino acid sequence of human TWEAK ligand (SEQ ID NO:1).
Figure 12. Amino acid sequence of human FN14 receptor (SEQ ID NO:2).

### DETAILED DESCRIPTION OF THE INVENTION

The techniques and procedures described or referenced herein are generally well understood and commonly employed using conventional methodology by those skilled in the art, such as, for example, the widely utilized molecular cloning methodologies described in Sambrook et al., Molecular Cloning: A Laboratory Manual 2nd. edition (1989) Cold Spring Harbor Laboratory Press, Cold Spring Harbor, N.Y. As appropriate, procedures involving the use of commercially available kits and reagents are generally carried out in accordance with manufacturer defined protocols and/or parameters unless otherwise noted.

Before the present methods and assays are described, it is to be understood that this invention is not limited to the particular methodology, protocols, cell lines, animal species or genera, constructs, and reagents described as such may, of course, vary. It is also to be understood that the terminology used herein is for the purpose of describing particular embodiments only, and is not intended to limit the scope of the present invention which will be limited only by the appended claims.

It must be noted that as used herein and in the appended claims, the singular forms "a", "an", and "the" include plural referents unless the context clearly dictates otherwise. Thus, for example, reference to "a genetic alteration" includes a plurality of such alterations and reference to "a probe" includes reference to one or more probes and equivalents thereof known to those skilled in the art, and so forth.

All publications mentioned herein disclose and describe the methods and/or materials in connection with which the publications are cited. Publications cited herein are cited for their disclosure prior to the filing date of the present application. Nothing here is to be construed as an admission that the inventors are not entitled to antedate the publications by virtue of an earlier priority date or prior date of invention. Further the actual publication dates may be different from those shown and require independent verification.

### I. DEFINITIONS

The terms "TWEAK" or "TWEAK ligand" are used herein to refer to a polypeptide sequence which includes amino acid residues 1-249 of Fig. 11, 47-249 of Fig. 11, or 94-249 of Fig. 11, inclusive, as well as biologically active fragments, deletional, insertional, or substitutional variants of the above sequences. In one embodiment, the polypeptide sequence comprises residues 47-249 of Figure 11, and optionally, consists of residues 94-249 of Figure 11. In other embodiments, the fragments or variants are biologically active and have at least about 80% amino acid sequence identity, more preferably at least about 90% sequence identity, and even more preferably, at least 95%, 96%, 97%, 98%, or 99% sequence identity with any one of the above recited sequences. Optionally, the TWEAK polypeptide is encoded by a nucleotide sequence which hybridizes under stringent conditions with the TWEAK encoding polynucleotide sequence. The definition also encompasses a native sequence TWEAK isolated from a TWEAK source or prepared by recombinant or synthetic methods. All numbering of amino acid residues referred to in the TWEAK sequence use the numbering according to Figure 11, unless specifically stated otherwise.

The term "extracellular domain" or "ECD" refers to a form of a protein, such as TWEAK, which is essentially free of transmembrane and cytoplasmic domains. Ordinarily, the ECD will have less than 1% of such transmembrane and cytoplasmic domains, and preferably, will have less than 0.5% of such domains. It will be understood that any transmembrane domain(s) identified for the polypeptides of the present invention are identified pursuant to criteria routinely employed in the art for identifying that type of hydrophobic domain. The exact boundaries of a transmembrane domain may vary but most likely by no more than about 5 amino acids at either end of the domain as initially identified. In preferred embodiments, the ECD will consist of a soluble, extracellular domain sequence of the polypeptide which is free of the transmembrane and cytoplasmic or intracellular domains (and is not membrane bound). Particular extracellular domain sequences of TWEAK are described in Marsters et. al., Curr. Biol., 8:525-528 (1998), Chicheportiche et al., JBC, 272:32401-32410 (1997).

The terms "TWEAK ligand" or "TWEAK" refers to any TWEAK monomeric, polymeric, or heteromeric complex or derivative thereof.

"TWEAK receptor" refers to one or more receptors which are capable of binding the TWEAK ligand described above. "TWEAK receptor" herein includes the receptor referred to in the art as "Fn-14" or "FN14" and its polypeptide sequence comprising amino acids 1-129 shown in Figure 12. The Fn14 receptor is also described in Wiley et al., Immunity, 15:837-846 (2001). The term "TWEAK receptor" when used herein encompasses native sequence receptor and receptor variants. These terms encompass TWEAK receptor expressed in a variety of mammals, including humans. TWEAK receptor may be endogenously expressed as occurs naturally in a variety of human tissue lineages, or may be expressed by recombinant or synthetic methods. A "native sequence TWEAK receptor" comprises a polypeptide having the same amino acid sequence as a TWEAK receptor derived from nature. Thus, a native sequence TWEAK receptor can have the amino acid sequence of naturally-occurring TWEAK receptor from any mammal. Such native sequence TWEAK receptor can be isolated from nature or can be produced by recombinant or synthetic means. The term "native sequence TWEAK receptor" specifically encompasses naturally-occurring truncated or secreted forms of the receptor (*e.g.,* a soluble form containing, for instance, an extracellular domain sequence), naturally-occurring variant forms (*e.g.,* alternatively spliced forms) and naturally-occurring allelic variants. Receptor variants may include fragments or deletion mutants of the native sequence TWEAK receptor.

The term "anti-TWEAK antibody" refers to any antibody that binds to at least one epitope of the TWEAK ligand. Optionally the TWEAK antibody is fused or linked to a heterologous sequence or molecule. Preferably the heterologous sequence allows or assists the antibody to form higher order or oligomeric complexes. Optionally, the TWEAK antibody binds to TWEAK but does not bind or cross-react with any additional TNF family ligands (e.g., Fas ligand, Apo2L/TRAIL, TNF-alpha, etc.). Optionally the antibody is an agonist or antagonist of TWEAK and/or TWEAK receptor activity.

Optionally, the TWEAK antibody of the invention binds to a TWEAK ligand at a concentration range of about 0.1 nM to about 20 mM as measured in a BIAcore binding assay. Optionally, the TWEAK antibodies of the invention exhibit an Ic 50 value of about 0.6 nM to about 18 mM as measured in a BIAcore binding assay.

The term "anti-TWEAK receptor antibody" refers to any antibody that binds to at least one epitope of a TWEAK receptor. Optionally the TWEAK receptor antibody is fused or linked to a heterologous sequence or molecule. Preferably the heterologous sequence allows or assists the antibody to form higher order or oligomeric complexes. Optionally, the TWEAK receptor antibody binds to TWEAK receptor but does not bind or cross-react with any additional TNF family receptors (e.g. FAS, DR4, DR5, TNFRI, TNFRII, etc.). Optionally the antibody is an agonist or antagonist of TWEAK receptor activity.

Optionally, the TWEAK receptor antibody of the invention binds to a TWEAK receptor at a concentration range of about 0.1 nM to about 20 mM as measured in a BIAcore binding assay. Optionally, the TWEAK receptor antibodies of the invention exhibit an Ic 50 value of about 0.6 nM to about 18 mM as measured in a BIAcore binding assay.

The term "antagonist" is used in the broadest sense, and includes any molecule that partially or fully blocks, inhibits, or neutralizes one or more biological activities of TWEAK or TWEAK receptor, *in vitro, in situ,* or *in vivo.* Examples of such biological activities of TWEAK or TWEAK receptor include binding of TWEAK to TWEAK receptor, activation of NF-kB phosphorylation or inhibition of STAT-1 phosphorylation, IL-8 production, inhibition of IFN-γ and IL-12 secretion, promotion of NK cell AICD, promotion of angiogenesis, or promotion of tumor growth. An antagonist may function in a direct or indirect manner. For instance, the antagonist may function to partially or fully block, inhibit or neutralize one or more biological activities of TWEAK or TWEAK receptor, *in vitro,* in *situ,* or in *vivo* as a result of TWEAK direct binding to TWEAK receptor. The antagonist may also function indirectly to partially or fully block, inhibit or neutralize one or more biological activities of TWEAK or TWEAK receptor, *in vitro*, *in situ*, or *in vivo* as a result of, e.g., blocking or inhibiting another effector molecule. The antagonist molecule may comprise a "dual" antagonist activity wherein the molecule is capable of partially or fully blocking, inhibiting or neutralizing a biological activity of both TWEAK and TWEAK receptor.

The term "TWEAK antagonist" refers to any molecule that partially or fully blocks, inhibits, or neutralizes a biological activity of TWEAK or TWEAK receptor, respectively, or both TWEAK and TWEAK receptor, and include, but are not limited to, soluble forms of TWEAK receptor such as an extracellular domain sequence of TWEAK receptor, TWEAK receptor immunoadhesins, TWEAK receptor fusion proteins, covalently modified forms of TWEAK receptor, TWEAK receptor antibodies, and TWEAK antibodies. To determine whether a TWEAK antagonist molecule partially or fully blocks, inhibits or neutralizes a biological activity of TWEAK or TWEAK receptor, assays may be conducted to assess the effect(s) of the antagonist molecule on, for example, binding of TWEAK to TWEAK receptor, or activation of NF-kB phosphorylation or inhibition of STAT-1 phosphorylation, or inhibition of IFN-γ or IL-12 production, or activation of cell death. Such assays may be conducted in known *in vitro* or *in vivo* assay formats, for instance, in NK cells, macrophages and dendritic cells. In one embodiment, the TWEAK antagonist will comprise a monoclonal antibody or a soluble TWEAK receptor ECD-Fc fusion protein.

The term "agonist" is used in the broadest sense, and includes any molecule that partially or fully stimulates, enhances, or induces one or more biological activities of TWEAK or TWEAK receptor, *in vitro, in situ,* or *in vivo*. Examples of such biological activities of TWEAK or TWEAK receptor include binding of TWEAK to TWEAK receptor, or activation of NF-kB phosphorylation or inhibition of STAT-1 phosphorylation, or inhibition of IFN-γ or IL-12 production, or activation of cell death. An agonist may function in a direct or indirect manner. The agonist molecule may comprise a "dual" agonist activity wherein the molecule is capable of partially or fully stimulating, enhancing, or inducing a biological activity of both TWEAK and TWEAK receptor.

The term "TWEAK agonist" refers to any molecule that partially or fully stimulates, enhances, or induces a biological activity of TWEAK or TWEAK receptor, respectively, or both TWEAK and TWEAK receptor, and include, but are not limited to, TWEAK polypeptides and variants thereof, and TWEAK receptor antibodies. To determine whether a TWEAK agonist molecule partially or fully stimulates, enhances, or induces a biological activity of TWEAK or TWEAK receptor, assays may be conducted to assess the effect(s) of the agonist molecule on, for example, IL-8 production, NF-kB phosphorylation, or inhibition of IFN-gamma or IL-12 production. Such assays may be conducted in known *in vitro* or *in vivo* assay formats, for instance, ELISA, intracellular cytokine production, or reporter assays. In one embodiment, the TWEAK agonist will comprise recombinant protein.

The term "mammal" as used herein refers to any mammal classified as a mammal, including humans, cows, horses, dogs and cats. In a preferred embodiment of the invention, the mammal is a human.

By "nucleic acid" is meant to include any DNA or RNA. For example, chromosomal, mitochondrial, viral and/or bacterial nucleic acid present in tissue sample. The term "nucleic acid" encompasses either or both strands of a double stranded nucleic acid molecule and includes any fragment or portion of an intact nucleic acid molecule.

By "gene" is meant any nucleic acid sequence or portion thereof with a functional role in encoding or transcribing a protein or regulating other gene expression. The gene may consist of all the nucleic acids responsible for encoding a functional protein or only a portion of the nucleic acids responsible for encoding or expressing a protein. The nucleic acid sequence may contain a genetic abnormality within exons, introns, initiation or termination regions, promoter sequences, other regulatory sequences or unique adjacent regions to the gene.

The word "label" when used herein refers to a compound or composition which is conjugated or fused directly or indirectly to a reagent such as a nucleic acid probe or an antibody and facilitates detection of the reagent to which it is conjugated or fused. The label may itself be detectable (*e.g.,* radioisotope labels or fluorescent labels) or, in the case of an enzymatic label, may catalyze chemical alteration of a substrate compound or composition which is detectable.

The term "antibody" herein is used in the broadest sense and specifically covers intact monoclonal antibodies, polyclonal antibodies, multispecific antibodies (*e.g.* bispecific antibodies) formed from at least two intact antibodies, and antibody fragments so long as they exhibit the desired biological activity.

"Antibody fragments" comprise a portion of an intact antibody, preferably comprising the antigen-binding or variable region thereof. Examples of antibody fragments include Fab, Fab', F(ab')₂, and Fv fragments; diabodies; linear antibodies; single-chain antibody molecules; and multispecific antibodies formed from antibody fragments.

"Native antibodies" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain (V_{H}) followed by a number of constant domains. Each light chain has a variable domain at one end (V_{L}) and a constant domain at its other end; the constant domain of the light chain is aligned with the first constant domain of the heavy chain, and the light-chain variable domain is aligned with the variable domain of the heavy chain. Particular amino acid residues are believed to form an interface between the light chain and heavy chain variable domains.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called hypervariable or complementary determining regions both in the light chain and the heavy chain variable domains. The more highly conserved portions of variable domains are called the framework regions (FRs). The variable domains of native heavy and light chains each comprise four FRs, largely adopting a β-sheet configuration, connected by three hypervariable regions, which form loops connecting, and in some cases forming part of, the β-sheet structure. The hypervariable regions in each chain are held together in close proximity by the FRs and, with the hypervariable regions from the other chain, contribute to the formation of the antigen-binding site of antibodies (see Kabat et al., Sequences of Proteins of Immunological Interest, 5th Ed. Public Health Service, National Institutes of Health, Bethesda, MD. (1991)). The constant domains are not involved directly in binding an antibody to an antigen, but exhibit various effector functions, such as participation of the antibody in antibody-dependent cell-mediated cytotoxicity (ADCC).

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')₂ fragment that has two antigen-binding sites and is still capable of cross-linking antigen.

"Fv" is the minimum antibody fragment which contains a complete antigen-recognition and antigen-binding site. This region consists of a dimer of one heavy chain and one light chain variable domain in tight, noncovalent association. It is in this configuration that the three hypervariable regions of each variable domain interact to define an antigen-binding site on the surface of the V_{H}-V_{L} dimer. Collectively, the six hypervariable regions confer antigen-binding specificity to the antibody. However, even a single variable domain (or half of an Fv comprising only three hypervariable regions specific for an antigen) has the ability to recognize and bind antigen, although at a lower affinity than the entire binding site.

The Fab fragment also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear at least one free thiol group. F(ab')₂ antibody fragments originally were produced as pairs of Fab' fragments which have hinge cysteines between them. Other chemical couplings of antibody fragments are also known.

The "light chains" of antibodies (immunoglobulins) from any vertebrate species can be assigned to one of two clearly distinct types, called kappa (κ) and lambda (λ), based on the amino acid sequences of their constant domains.

Depending on the amino acid sequence of the constant domain of their heavy chains, antibodies can be assigned to different classes. There are five major classes of intact antibodies: IgA, IgD, IgE, IgG, and IgM, and several of these may be further divided into subclasses (isotypes), e.g., IgG1, IgG2, IgG3, IgG4, IgA, and IgA2. The heavy-chain constant domains that correspond to the different classes of antibodies are called α, δ, ε, γ, and µ, respectively. The subunit structures and three-dimensional configurations of different classes of immunoglobulins are well known.

"Single-chain Fv" or "scFv" antibody fragments comprise the V_{H} and V_{L} domains of antibody, wherein these domains are present in a single polypeptide chain. Preferably, the Fv polypeptide further comprises a polypeptide linker between the V_{H} and V_{L} domains which enables the scFv to form the desired structure for antigen binding. For a review of scFv see Plückthun in The Pharmacology of Monoclonal Antibodies, vol. 113, Rosenburg and Moore eds., Springer-Verlag, New York, pp. 269-315 (1994).

The term "diabodies" refers to small antibody fragments with two antigen-binding sites, which fragments comprise a heavy-chain variable domain (V_{H}) connected to a light-chain variable domain (V_{L}) in the same polypeptide chain (V_{H} - V_{L}). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90:6444-6448 (1993).

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., Nature, 256:495 (1975), or may be made by recombinant DNA methods (see, *e.g*., U.S. Patent No. 4,816,567). The "monoclonal antibodies" may also be isolated from phage antibody libraries using the techniques described in Clackson et al., Nature, 352:624-628 (1991) and Marks et al., J. Mol. Biol., 222:581-597 (1991), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; Morrison et al., Proc. Natl. Acad. Sci. USA, 81:6851-6855 (1984)). Chimeric antibodies of interest herein include "primatized" antibodies comprising variable domain antigen-binding sequences derived from a non-human primate (e.g. Old World Monkey, such as baboon, rhesus or cynomolgus monkey) and human constant region sequences (US Pat No. 5,693,780).

"Humanized" forms of non-human (*e.g.,* murine) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992).

The term "hypervariable region" when used herein refers to the amino acid residues of an antibody which are responsible for antigen-binding. The hypervariable region comprises amino acid residues from a "complementarity determining region" or "CDR" (*e.g.* residues 24-34 (L1), 50-56 (L2) and 89-97 (L3) in the light chain variable domain and 31-35 (H1), 50-65 (H2) and 95-102 (H3) in the heavy chain variable domain; Kabat et al., Sequences of Proteins of Immunological Interest, 5th Ed. Public Health Service, National Institutes of Health, Bethesda, MD. (1991)) and/or those residues from a "hypervariable loop" (*e.g.* residues 26-32 (L1), 50-52 (L2) and 91-96 (L3) in the light chain variable domain and 26-32 (H1), 53-55 (H2) and 96-101 (H3) in the heavy chain variable domain; Chothia and Lesk J. Mol. Biol. 196:901-917 (1987)). "Framework" or "FR" residues are those variable domain residues other than the hypervariable region residues as herein defined.

As used herein, the term "immunoadhesin" designates antibody-like molecules which combine the binding specificity of a heterologous protein (an "adhesin") with the effector functions of immunoglobulin constant domains. Structurally, the immunoadhesins comprise a fusion of an amino acid sequence with the desired binding specificity which is other than the antigen recognition and binding site of an antibody (i.e., is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin may be obtained from any immunoglobulin, such as IgG-1, IgG-2, IgG-3, or IgG-4 subtypes, IgA (including IgA-1 and IgA-2), IgE, IgD or IgM.

The term "Fc domain" of an antibody refers to a part of the molecule comprising the hinge, CH2 and CH3 domains, but lacking the antigen binding sites. The term is also meant to include the equivalent regions of an IgM or other antibody isotype.

An antibody "which binds" an antigen of interest is one capable of binding that antigen with sufficient affinity and/or avidity such that the antibody is useful as a therapeutic or diagnostic agent for targeting a cell expressing the antigen.

For the purposes herein, "immunotherapy" will refer to a method of treating a mammal (preferably a human patient) with an antibody, wherein the antibody may be an unconjugated or "naked" antibody, or the antibody may be conjugated or fused with heterologous molecule(s) or agent(s), such as one or more cytotoxic agent(s), thereby generating an "immunoconjugate".

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody *in situ* within recombinant cells since at least one component of the antibody's natural environment will not be present. Ordinarily, however, isolated antibody will be prepared by at least one purification step.

The expression "effective amount" refers to an amount of an agent (e.g. TWEAK antibody etc.) which is effective for preventing, ameliorating or treating the disease or condition in question.

The terms "treating", "treatment" and "therapy" as used herein refer to curative therapy, prophylactic therapy, and preventative therapy. Consecutive treatment or administration refers to treatment on at least a daily basis without interruption in treatment by one or more days. Intermittent treatment or administration, or treatment or administration in an intermittent fashion, refers to treatment that is not consecutive, but rather cyclic in nature.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-α and -β; mullerian-inhibiting substance; mouse gonadotropin-associated peptide; inhibin; activin; vascular endothelial growth factor; integrin; thrombopoietin (TPO); nerve growth factors; platelet-growth factor; transforming growth factors (TGFs) such as TGF-α and TGF-β; insulin-like growth factor-I and -II; erythropoietin (EPO); osteoinductive factors; interferons such as interferon-α, -β, and - gamma; colony stimulating factors (CSFs) such as macrophage-CSF (M-CSF); granulocyte-macrophage-CSF (GM-CSF); and granulocyte-CSF (G-CSF); interleukins (ILs) such as IL-1, IL-2, IL-3, IL-4, IL-5, IL-6, IL-7, IL-8, IL-9, IL-11, IL-12, IL-13, IL-17; and other polypeptide factors including LIF. and kit ligand (KL). As used herein, the term cytokine includes proteins from natural sources or from recombinant cell culture and biologically active equivalents of the native sequence cytokines.

The term "cytotoxic agent" as used herein refers to a substance that inhibits or prevents the function of cells and/or causes destruction of cells. The term is intended to include radioactive isotopes (e.g., I¹³¹, I¹²⁵, Y⁹⁰ and Re¹⁸⁶), chemotherapeutic agents, and toxins such as enzymatically active toxins of bacterial, fungal, plant or animal origin, or fragments thereof.

A "chemotherapeutic agent" is a chemical compound useful in the treatment of cancer. Examples of chemotherapeutic agents include alkylating agents such as thiotepa and cyclosphosphamide (CYTOXAN^{™}); alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CBI-TMI); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics such as the enediyne antibiotics (e.g. calicheamicin, especially calicheamicin gamma1I and calicheamicin phiI1, see, e.g., Agnew, Chem Intl. Ed. Engl., 33:183-186 (1994); dynemicin, including dynemicin A; bisphosphonates, such as clodronate; an esperamicin; as well as neocarzinostatin chromophore and related chromoprotein enediyne antiobiotic chromomophores), aclacinomysins, actinomycin, authramycin, azaserine, bleomycins, cactinomycin, carabicin, carminomycin, carzinophilin, chromomycins, dactinomycin, daunorubicin, detorubicin, 6-diazo-5-oxo-L-norleucine, doxorubicin (Adriamycin^{™}) (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidamine; maytansinoids such as maytansine and ansamitocins; mitoguazone; mitoxantrone; mopidamol; nitracrine; pentostatin; phenamet; pirarubicin; losoxantrone; podophyllinic acid; 2-ethylhydrazide; procarbazine; PSK^{®}; razoxane; rhizoxin; sizofiran; spirogermanium; tenuazonic acid; triaziquone; 2, 2',2''-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g. paclitaxel (TAXOL^{®}, Bristol-Myers Squibb Oncology, Princeton, NJ) and doxetaxel (TAXOTERE^{®}, Rhône-Poulenc Rorer, Antony, France); chlorambucil; gemcitabine (Gemzar^{™}) 6-thioguanine; mercaptopurine; methotrexate; platinum analogs such as cisplatin and carboplatin; vinblastine; platinum; etoposide (VP-16); ifosfamide; mitoxantrone; vincristine; vinorelbine (Navelbine^{™}) ; novantrone; teniposide; edatrexate; daunomycin; aminopterin; xeloda; ibandronate; CPT-11; topoisomerase inhibitor RFS 2000; difluoromethylornithine (DMFO); retinoids such as retinoic acid; capecitabine; and pharmaceutically acceptable salts, acids or derivatives of any of the above. Also included in this definition are anti-hormonal agents that act to regulate or inhibit hormone action on tumors such as antiestrogens and selective estrogen receptor modulators (SERMs), including, for example, tamoxifen (including Nolvadex^{™}), raloxifene, droloxifene, 4-hydroxytamoxifen, trioxifene, keoxifene, LY117018, onapristone, and toremifene (Fareston^{™}) ; aromatase inhibitors that inhibit the enzyme aromatase, which regulates estrogen production in the adrenal glands, such as, for example, 4(5)-imidazoles, aminoglutethimide, megestrol acetate (Megace^{™}), exemestane, formestane, fadrozole, vorozole (Rivisor^{™}), letrozole (Femara^{™}), and anastrozole (Arimidex^{™}) ; and anti-androgens such as flutamide, nilutamide, bicalutamide, leuprolide, and goserelin; and pharmaceutically acceptable salts, acids or derivatives of any of the above.

A "growth inhibitory agent" when used herein refers to a compound or composition which inhibits growth of a cell, especially cancer cell overexpressing any of the genes identified herein, either *in vitro* or *in vivo.* Thus, the growth inhibitory agent is one which significantly reduces the percentage of cells overexpressing such genes in S phase. Examples of growth inhibitory agents include agents that block cell cycle progression (at a place other than S phase), such as agents that induce G1 arrest and M-phase arrest. Classical M-phase blockers include the vincas (vincristine and vinblastine), taxol, and topo II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogens, and antineoplastic drugs" by Murakami et al. (WB Saunders: Philadelphia, 1995), especially p. 13.

The terms "apoptosis" and "apoptotic activity" are used in a broad sense and refer to the orderly or controlled form of cell death in mammals that is typically accompanied by one or more characteristic cell changes, including condensation of cytoplasm, loss of plasma membrane microvilli, segmentation of the nucleus, degradation of chromosomal DNA or loss of mitochondrial function. This activity can be determined and measured, for instance, by cell viability assays (such as Alamar blue assays or MTT assays), FACS analysis, caspase activation, DNA fragmentation (see, for example, Nicoletti et al., J. Immunol. Methods, 139:271-279 (1991), and poly-ADP ribose polymerase, "PARP", cleavage assays known in the art.

As used herein, the term "disorder" in general refers to any condition that would benefit from treatment with the compositions described herein. This includes chronic and acute disorders, as well as those pathological conditions which predispose the mammal to the disorder in question. Non-limiting examples of disorders to be treated herein include benign and malignant cancers; inflammatory, infection, angiogenic, and immunologic disorders, autoimmune disorders, arthritis (including rheumatoid arthritis), multiple sclerosis, and HIV/AIDS.

The terms "cancer", "cancerous", or "malignant" refer to or describe the physiological condition in mammals that is typically characterized by unregulated cell growth. Examples of cancer include but are not limited to, carcinoma, lymphoma, leukemia, blastoma, and sarcoma. More particular examples of such cancers include squamous cell carcinoma, myeloma, small-cell lung cancer, non-small cell lung cancer, glioma, gastrointestinal (tract) cancer, renal cancer, ovarian cancer, liver cancer, lymphoblastic leukemia, lymphocytic leukemia, colorectal cancer, endometrial cancer, kidney cancer, prostate cancer, thyroid cancer, neuroblastoma, pancreatic cancer, glioblastoma multiforme, cervical cancer, brain cancer, stomach cancer, bladder cancer, hepatoma, breast cancer, colon carcinoma, and head and neck cancer.

The terms "humoral response" and "cellular response" as used herein refer to the immunological response of a mammal to an antigen whereby the mammal produces antibodies to an antigen or produces a cytotoxic response to the antigen, or both. The Th1 class of T helper cells plays a role for the induction of the cellular response, and the Th2 class of T helper cells plays a role for the efficient production of high affinity antibodies.

The term "T helper (Th) cells" as used herein, refers to a functional subclass of T cells which help to generate cytotoxic T cells and which cooperate with B cells to stimulate antibody production. Helper T cells recognize antigen in association with class II MHC molecules and provide contact dependent and contact independent (cytokine and chemokine) signals to effector cells.

The term "Th1" refers to a subclass of T helper cells that produce TNF, interferon-gamma and IL-2 (and other cytokines) and which elicit inflammatory reactions associated with a cellular, i.e. non-immunoglobulin, response to a challenge.

The term "Th2" refers to a subclass of T helper cells that produces IL-4, IL-5, IL-6, IL-10, and other cytokines, which are associated with an immunoglobulin (humoral) response to an immune challenge.

The term "immune related disease" means a disease in which a component of the immune system of a mammal causes, mediates or otherwise contributes to morbidity in the mammal. Also included are diseases in which stimulation or intervention of the immune response has an ameliorative effect on progression of the disease. Included within this term are autoimmune diseases, immune-mediated inflammatory diseases, non-immune-mediated inflammatory diseases, infectious diseases, and immunodeficiency diseases. Examples of immune-related and inflammatory diseases, some of which are immune or T cell mediated, which can be treated according to the invention include systemic lupus erythematosis, rheumatoid arthritis, juvenile chronic arthritis, spondyloarthropathies, systemic sclerosis (scleroderma), idiopathic inflammatory myopathies (dermatomyositis, polymyositis), Sjogren's syndrome, systemic vasculitis, sarcoidosis, autoimmune hemolytic anemia (immune pancytopenia, paroxysmal nocturnal hemoglobinuria), autoimmune thrombocytopenia (idiopathic thrombocytopenic purpura, immune-mediated thrombocytopenia), thyroiditis (Grave's disease, Hashimoto's thyroiditis, juvenile lymphocytic thyroiditis, atrophic thyroiditis), diabetes mellitus, immune-mediated renal disease (glomerulonephritis, tubulointerstitial nephritis), demyelinating diseases of the central and peripheral nervous systems such as multiple sclerosis, idiopathic demyelinating polyneuropathy or Guillain-Barré syndrome, and chronic inflammatory demyelinating polyneuropathy, hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis, inflammatory and fibrotic lung diseases such as inflammatory bowel disease (ulcerative colitis: Crohn's disease), gluten-sensitive enteropathy, and Whipple's disease, autoimmune or immune-mediated skin diseases including bullous skin diseases, erythema multiforme and contact dermatitis, psoriasis, allergic diseases such as asthma, allergic rhinitis, atopic dermatitis, food hypersensitivity and urticaria, immunologic diseases of the lung such as eosinophilic pneumonias, idiopathic pulmonary fibrosis and hypersensitivity pneumonitis, transplantation associated diseases including graft rejection and graft-versus-host-disease. Infectious diseases include AIDS (HIV infection), hepatitis A, B, C, D, and E, bacterial infections, fungal infections, protozoal infections and parasitic infections.

"Autoimmune disease" is used herein in a broad, general sense to refer to disorders or conditions in mammals in which destruction of normal or healthy tissue arises from humoral or cellular immune responses of the individual mammal to his or her own tissue constituents. Examples include, but are not limited to, lupus erythematous, thyroiditis, rheumatoid arthritis, psoriasis, multiple sclerosis, autoimmune diabetes, and inflammatory bowel disease (IBD).

The term "tagged" when used herein refers to a chimeric molecule comprising an antibody or polypeptide fused to a "tag polypeptide". The tag polypeptide has enough residues to provide an epitope against which an antibody can be made or to provide some other function, such as the ability to oligomerize (e.g. as occurs with peptides having leucine zipper domains), yet is short enough such that it generally does not interfere with activity of the antibody or polypeptide. The tag polypeptide preferably also is fairly unique so that a tag-specific antibody does not substantially cross-react with other epitopes. Suitable tag polypeptides generally have at least six amino acid residues and usually between about 8 to about 50 amino acid residues (preferably, between about 10 to about 20 residues).

"Isolated," when used to describe the various peptides or proteins disclosed herein, means peptide or protein that has been identified and separated and/or recovered from a component of its natural environment. Contaminant components of its natural environment are materials that would typically interfere with diagnostic or therapeutic uses for the peptide or protein, and may include enzymes, hormones, and other proteinaceous or nonproteinaceous solutes. In preferred embodiments, the peptide or protein will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain, or (3) to homogeneity by mass spectroscopic or peptide mapping techniques. Isolated material includes peptide or protein in situ within recombinant cells, since at least one component of its natural environment will not be present. Ordinarily, however, isolated peptide or protein will be prepared by at least one purification step.

"Percent (%) amino acid sequence identity" with respect to the sequences identified herein is defined as the percentage of amino acid residues in a candidate sequence that are identical with the amino acid residues in the reference sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art can determine appropriate parameters for measuring alignment, including assigning algorithms needed to achieve maximal alignment over the full-length sequences being compared. For purposes herein, percent amino acid identity values can be obtained using the sequence comparison computer program, ALIGN-2, which was authored by Genentech, Inc. and the source code of which has been filed with user documentation in the US Copyright Office, Washington, DC, 20559, registered under the US Copyright Registration No. TXU510087. The ALIGN-2 program is publicly available through Genentech, Inc., South San Francisco, CA. All sequence comparison parameters are set by the ALIGN-2 program and do not vary.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to re-anneal when complementary strands are present in an environment below their melting temperature. The higher the degree of desired identity between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"High stringency conditions", as defined herein, are identified by those that: (1) employ low ionic strength and high temperature for washing; 0.015 M sodium chloride/0.0015 M sodium citrate/0.1% sodium dodecyl sulfate at 50°C; (2) employ during hybridization a denaturing agent; 50% (v/v) formamide with 0.1% bovine serum albumin/0.1% Ficoll/0.1% polyvinylpyrrolidone/50mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 µg/ml), 0.1% SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55°C.

"Moderately stringent conditions" may be identified as described by Sambrook et al., Molecular Cloning: A Laboratory Manual, New York: Cold Spring Harbor Press, 1989, and include overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50°C. The skilled artisan will recognize how to adjust the temperature, ionic strength, etc. as necessary to accommodate factors such as probe length and the like.

The term "primer" or "primers" refers to oligonucleotide sequences that hybridize to a complementary RNA or DNA target polynucleotide and serve as the starting points for the stepwise synthesis of a polynucleotide from mononucleotides by the action of a nucleotidyltransferase, as occurs for example in a polymerase chain reaction.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

"Antibody-dependent cell-mediated cytotoxicity" and "ADCC" refer to a cell-mediated reaction in which nonspecific cytotoxic cells that express Fc receptors (FcRs) (*e.g.* Natural Killer (NK) cells, neutrophils, and macrophages) recognize bound antibody on a target cell and subsequently cause lysis of the target cell. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch and Kinet, Annu. Rev. Immunol 9:457-92 (1991). To assess ADCC activity of a molecule of interest, an *in vitro* ADCC assay, such as that described in US Patent No. 5,500,362 or 5,821,337 may be performed. Useful effector cells for such assays include peripheral blood mononuclear cells (PBMC) and Natural Killer (NK) cells. Alternatively, or additionally, ADCC activity of the molecule of interest may be assessed in *vivo, e.g.,* in a animal model such as that disclosed in Clynes et al. PNAS (USA) 95:652-656 (1998).

"Human effector cells" are leukocytes which express one or more FcRs and perform effector functions. Preferably, the cells express at least FcγRIII and carry out ADCC effector function. Examples of human leukocytes which mediate ADCC include peripheral blood mononuclear cells (PBMC), natural killer (NK) cells, monocytes, cytotoxic T cells and neutrophils; with PBMCs and NK cells being preferred.

The terms "Fc receptor" or "FcR" are used to describe a receptor that binds to the Fc region of an antibody. The preferred FcR is a native sequence human FcR. Moreover, a preferred FcR is one which binds an IgG antibody (a gamma receptor) and includes receptors of the FcγRI, FcγRII, and Fcγ RIII subclasses, including allelic variants and alternatively spliced forms of these receptors. FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain. (see Daëron, Annu. Rev. Immunol. 15:203-234 (1997)). FcRs are reviewed in Ravetch and Kinet, Annu. Rev. Immunol 9:457-92 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein. The term also includes the neonatal receptor, FcRn, which is responsible for the transfer of maternal IgGs to the fetus (Guyer et al., J. Immunol. 117:587 (1976) and Kim et al., J. Immunol. 24:249 (1994)). FcRs herein include polymorphisms such as the genetic dimorphism in the gene that encodes FcγRIIIa resulting in either a phenylalanine (F) or a valine (V) at amino acid position 158, located in the region of the receptor that binds to IgG1. The homozygous valine FcγRIIIa (FcγRIIIa-158V) has been shown to have a higher affinity for human IgG1 and mediate increased ADCC *in vitro* relative to homozygous phenylalanine FcγRIIIa (FcγRIIIa-158F) or heterozygous (FcγRIIIa-158F/V) receptors.

"Complement dependent cytotoxicity" or "CDC" refer to the ability of a molecule to lyse a target in the presence of complement. The complement activation pathway is initiated by the binding of the first component of the complement system (C1q) to a molecule (*e.g.* an antibody) complexed with a cognate antigen. To assess complement activation, a CDC assay, *e.g.* as described in Gazzano-Santoro et al., J. Immunol. Methods 202:163 (1996), may be performed.

### II. VARIOUS METHODS AND MATERIALS OF THE INVENTION

Host defense against infection involves coordinated function of the innate and adaptive immune systems in mammals. The innate immune system, which includes NK cells, dendritic cells, macrophages, and neutrophils, plays a crucial role not only in the early response to infection, but also in guiding the transition to a T and B cell-based adaptive immunity (Diefenbach and Raulet, Immunol. Rev., 188:9-21 (2002)). Innate immune cells mediate the direct killing and elimination of infected cells; subsequently, they actively support the development of adaptive functions through physical interactions with dendritic cells and consequent secretion of specific cytokines (Diefenbach and Raulet, Immunol. Rev., 181:170-184 (2001); Fernandez et al., Eur. Cytokine Netw., 13:17-27 (2002); Ikeda et al., Cytokine Growth Factor Rev., 13:95-109 (2002)). INF-gamma and IL-12 polarize the development of helper CD4⁺ T cells toward the T_{H}1 subtype, which activates CD8⁺ effector T cell responses, while IL-4 induces the T_{H}2 class, which stimulates B cell-mediated antibody responses (Diefenbach and Raulet, 2002, supra; Fernandez et al., 2002, supra; Ikeda et al., 2002, supra).

Innate immunity is important not only as the first line of defense against infection but also for protecting the host during the time period that is required for the development of adaptive immunity. Furthermore, the innate response critically influences the nature of adaptive mechanisms that develop in response to an infectious challenge (Castriconi et al., C R Biol., 327:533-537 (2004); Lo et al., Immunol. Rev., 169:225-239 (1999); Palucka and Banchereau, J. Clin. Immunol., 19:12-25 (1999); Palucka and Banchereau, Nat. Med., 5:868-870 (1999)). Interactions of NK cells with macrophages and dendritic cells stimulate the secretion of specific cytokines that support the development of particular T and/or B cell responses (Palucka and Banchereau, J. Clin. Immunol., 19:12-25 (1999); Palucka and Banchereau, Nat. Med., 5:868-870 (1999); Trinchieri, Semin. Immunol., 7:83-88 (1995)). IFN-gamma secretion by NK cells and IL-12 production by macrophages and dendritic cells promotes the development of an adaptive T_{H}1 response, leading to cytotoxic T cell effector function (Coudert et al., J. Immunol., 169:2979-2987 (2002); Fujii et al., J. Exp. Med., 198:267-279 (2003); Gerosa et al., J. Exp. Med., 195:327-333 (2002); Pan et al., Immunol. Letters, 94:141-151 (2004); Varma et al., Clin. Diag. Lab Immunol., 9:530-543 (2002)). In contrast, IL-4 production by NKT cells promotes adaptive T_{H}2 differentiation and B cell activation (Araujo et al., Int. Immunol., 12:1613-1622 (2000); Kaneko et al., J. Exp. Med., 191:105-114 (2000); Leite-De-Moraes et al., J. Immunol., 166:945-951 (2001)).

The experiments disclosed in the present application indicate TWEAK is an important regulator of the innate system and its interface with adaptive immunity. Innate immune cells, namely, NK cells, macrophages and dendritic cells, expressed TWEAK and its receptor FN14 and up-regulated both molecules upon stimulation. In contrast, cells of the adaptive system, including T and B cells, did not express significant levels of TWEAK or FN14. This expression pattern suggests that TWEAK signaling in innate immune cells may modulate innate immune function, and may indirectly influence adaptive immune responses by its regulation of innate activity.

As described in the Examples section below, the TWEAK knockout mice generated were viable and healthy, demonstrating that TWEAK is not crucial for normal development. However, TWEAK^{-/-} mice showed a significant accumulation of NK cells as compared to age-matched, wild type littermates, implicating TWEAK in the control of NK cell generation and/or death. TWEAK gene ablation did not alter the amount of NK cells in the bone marrow, suggesting unabated NK cell formation in TWEAK's absence. Conversely, neutralization of TWEAK protected human NK cells from apoptosis induction by TNF-alpha, LPS, or IFN-gamma. These findings suggest that impaired AICD rather than increased generation causes NK cell accumulation in TWEAK^{-/-} mice. Thus, one immunomodulatory role of TWEAK may be to help prevent the potentially harmful development of an excessive innate response, by supporting the deletion of activated NK cells upon immunological resolution.

In Applicants' experiments, TWEAK deficiency in mice substantially increased the sensitivity of mice to systemic LPS injection, further implicating TWEAK in curbing the innate response. Given that NK cell activity is an important component of the systemic inflammatory reaction to LPS (Emoto et al., J. Immunol., 169:1426-1432 (2002); Heremans et al., Eur. J. Immunol., 24:1155-1160 (1994)), one explanation for the hypersensitivity of TWEAK^{-/-} mice could be their elevated NK cell numbers. However, Applicants found, in addition, that TWEAK-deficient NK cells produced more IFN-gamma while TWEAK^{-/-} macrophages generated more IL-12 and less IL-10 after exposure to LPS *in vivo*. Furthermore, TWEAK neutralization enhanced the production of IFN-gamma and IL-12 by LPS-stimulated NK cells and macrophages. These results suggest that the increased sensitivity of TWEAK^{-/-} mice to LPS stems not only from their elevated NK cell numbers but also from greater innate immune cell production of IFN-gamma and IL-12. Thus, in addition to supporting NK AICD, TWEAK may curtail the innate response by repressing secretion of key pro-inflammatory cytokines. In this regard, TWEAK differs strikingly from its relative TNF-alpha, which stimulates the secretion of IL-12 and IFN-gamma, thus augmenting the innate inflammatory response (D'Andrea et al., J. Exp. Med., 178:1041-1048 (1993); Oswald et al., Eur. Cytokine Netw., 10:533-540 (1999); Wilhelm et al., J. Immunol., 166:4012-4019 (2001); Zhan and Cheers, J. Immunol., 161:1447-1453 (1998)). Indeed, contrary to the LPS hypersensitivity of the TWEAK knockouts, TNF-alpha or TNFR1 knockout mice are resistant to LPS-induced lethality (Pasparakis et al., J. Exp. Med., 184:1397-1411 (1996); Rothe et al., Circ. Shock, 44:51-56 (1994)).

STAT-1 is a key signal-transducer involved in the production of IFN-gamma and IL-12 in response to infection (Dupuis et al., Immunol. Rev., 178:129-137 (2000); Feinberg et al., Eur. J. Immunol., 34:3276-3284 (2004)). Comparison of phospho-STAT-1 in NK cells and macrophages from TWEAK^{-/-} and wild type mice revealed elevated basal activity and enhanced stimulation in response to LPS. This result suggests that TWEAK inhibits STAT-1 activity, in contrast to TNF-alpha, which enhances this function (Chen et al., Immunology, 107:199-208 (2002)). Thus, one mechanism that may contribute to TWEAK's suppression of the production of IFN-gamma and IL-12 is inhibition of STAT-1. Like STAT-1, NF-κB1 also plays an important role in controlling cytokine gene transcription (Feinberg et al., Eur. J. Immunol., 34:3276-3284 (2004); Zhan and Cheers, J. Immunol., 161:1447-1453 (1998)). In human NK cells and macrophages, TWEAK stimulated prolonged phosphorylation of NF-κB1, inducing the association of this factor with the transcriptional repressor HDAC-1. In contrast, TNF-alpha induced transient NF-κB1 phosphorylation and binding to the transcriptional co-activator p300. Thus, a second mechanism contributing to TWEAK's repression of the synthesis of IFN-gamma and IL-12 may be the induction of an association between NF-κB1 and HDAC-1. The difference between TWEAK and TNF-alpha in regard to the modulation of NF-κB1 may be due to the kinetics of NF-κB1 phosphorylation which influence the association of this factor with other transcriptional regulators, such that transient phosphorylation favors interaction with p300 while sustained modification promotes binding to HDAC-1. There appears to be a parallel between this observation and the control of the c-Jun N-terminal kinase (JNK) pathway by TNF-alpha, where transient versus sustained JNK phosphorylation correlates with promotion of cell survival *versus* cell death (Varfolomeev and Ashkenazi, Mol. Cell Biol., 24:997-1006 (2004)).

Applicants' findings suggest that the expression of TWEAK by NK cells and macrophages in response to infection helps to curtail the innate inflammatory response by promoting NK AICD as well as by repressing the production of IFN-gamma and IL-12 by NK cells and macrophages. IFN-gamma and IL-12 do not only enhance the innate inflammatory response; they also promote the transition to adaptive immunity in favor of a cellular T_{H}1-type response. Applicants' observed that in the absence of TWEAK, aged mice developed enlarged spleens with increased numbers not only of NK cells (which constitute a very small fraction of splenocytes) but also of T cells of the T_{H}1 phenotype. Further experimental evidence supports TWEAK's regulation of the adaptive transition. In the mouse B16 melanoma model, TWEAK^{-/-} mice rejected growth of the moderately aggressive B16.F10 sub-clone, while wild type littermates failed to combat tumor growth. While the elevated numbers of NK cells in TWEAK^{-/-} mice could explain their ability to reject the tumors, the anti-tumor response in these mice was associated also with an expansion of CD8⁺ T cells) consistent with an augmented T_{H}1 response. TWEAK^{-/-} mice also resisted growth of the more aggressive B16.BL6 sub-clone better than did wild type controls, and upon re-challenge with tumor cells *ex vivo,* their CD8⁺ T cells and NK cells produced significantly more IFN-gamma while their macrophages generated more IL-12 than did corresponding controls.

Accordingly, the findings suggest that TWEAK modulates the innate-to-adaptive immune interface by suppressing the production of IFN-gamma and IL-12 and hence keeping in check the consequent development of a T_{H1}-mediated cellular response. Applicants have found an important role for TWEAK in immune modulation, which markedly differs from the function of its structural relative, TNF-alpha. TNF-alpha plays a key role in supporting the innate inflammatory response by promoting innate cell stimulation and pro-inflammatory cytokine secretion. In contrast, TWEAK seems to be crucial for curtailing the innate response, through mediating NK AICD as well as repressing the production of IFN-gamma and IL-12 by NK cells and macrophages. Whereas TNF-alpha activates transcription of immunomodulatory genes by promoting STAT-1 activation and NF-κB1 association with p300, TWEAK represses STAT-1 activity and induces binding of NF-κB1 to HDAC-1, which inhibits gene transcription. Importantly, TWEAK also has a critical role in attenuating the transition from an innate to an adaptive T_{H}1 immune response. Thus, TWEAK's function may assist in curbing the host mammal's innate and adaptive responses, ensuring against the development of excessive inflammation and autoimmunity. This finding suggests that TWEAK inhibition may be useful clinically for augmenting anti-infective and anti-tumor immunity, while TWEAK receptor activation might be useful for controlling acute and chronic autoimmune diseases.

In accordance with the disclosure herein, compositions comprising one or more molecules which modulate TWEAK or TWEAK receptor activity may be employed for treatment of various disorders. For instance, TWEAK antagonists may be employed in treating cancer. Such TWEAK antagonists include TWEAK antibodies, TWEAK variants, TWEAK receptor immunoadhesins, and TWEAK receptor antibodies. The TWEAK antagonists may be used *in vivo* as well as *ex vivo.* Optionally, the TWEAK antagonists are used in the form of pharmaceutical compositions, described in further detail below.

In further examples, TWEAK agonists may be employed in treating various immune-related conditions. Such TWEAK agonists include TWEAK receptor antibodies and TWEAK polypeptides. The TWEAK agonists may be used *in vivo* as well as *ex vivo*. Optionally, the TWEAK agonists are used in the form of pharmaceutical compositions, described in further detail below.

In the description below, various methods and techniques are described. It is contemplated that these methods and techniques may be similarly employed for preparing a variety of TWEAK agonists and antagonists.

By way of example, it is contemplated that TWEAK polypeptides and TWEAK polypeptide variants can be prepared. TWEAK variants can be prepared by introducing appropriate nucleotide changes into the encoding DNA, and/or by synthesis of the desired polypeptide. Those skilled in the art will appreciate that amino acid changes may alter post-translational processes of the TWEAK polypeptide, such as changing the number or position of glycosylation sites or altering the membrane anchoring characteristics.

Variations in the TWEAK polypeptides described herein, can be made, for example, using any of the techniques and guidelines for conservative and non-conservative mutations set forth, for instance, in U.S. Patent No. 5,364,934. Variations may be a substitution, deletion or insertion of one or more codons encoding the polypeptide that results in a change in the amino acid sequence as compared with the native sequence polypeptide. Optionally the variation is by substitution of at least one amino acid with any other amino acid in one or more of the domains of the TWEAK polypeptide. Guidance in determining which amino acid residue may be inserted, substituted or deleted without adversely affecting the desired activity may be found by comparing the sequence of the TWEAK polypeptide with that of homologous known protein molecules and minimizing the number of amino acid sequence changes made in regions of high homology. Amino acid substitutions can be the result of replacing one amino acid with another amino acid having similar structural and/or chemical properties, such as the replacement of a leucine with a serine, i.e., conservative amino acid replacements. Insertions or deletions may optionally be in the range of about 1 to 5 amino acids. The variation allowed may be determined by systematically making insertions, deletions or substitutions of amino acids in the sequence and testing the resulting variants for activity exhibited by the full-length or mature native sequence.

TWEAK polypeptide fragments are provided herein. Such fragments may be truncated at the N-terminus or C-terminus, or may lack internal residues, for example, when compared with a full length native protein. Certain fragments lack amino acid residues that are not essential for a desired biological activity of the TWEAK polypeptide.

TWEAK polypeptide fragments may be prepared by any of a number of conventional techniques. Desired peptide fragments may be chemically synthesized. An alternative approach involves generating polypeptide fragments by enzymatic digestion, e.g., by treating the protein with an enzyme known to cleave proteins at sites defined by particular amino acid residues, or by digesting the DNA with suitable restriction enzymes and isolating the desired fragment. Yet another suitable technique involves isolating and amplifying a DNA fragment encoding a desired polypeptide fragment, by polymerase chain reaction (PCR). Oligonucleotides that define the desired termini of the DNA fragment are employed at the 5' and 3' primers in the PCR.

In particular examples, conservative substitutions of interest are shown in the Table below under the heading of preferred substitutions. If such substitutions result in a change in biological activity, then more substantial changes, denominated exemplary substitutions in the Table, or as further described below in reference to amino acid classes, are introduced and the products screened.

**Table**

| Original Residue | Exemplary Substitutions | Preferred Substitutions |
|---|---|---|
| Ala (A) | val; leu; ile | val |
| Arg (R) | lys; gln; asn | lys |
| Asn (N) | gln; his; lys; arg | gln |
| Asp (D) | glu | glu |
| Cys (C) | ser | ser |
| Gln (Q) | asn | asn |
| Glu (E) | asp | asp |
| Gly (G) | pro; ala | ala |
| His (H) | asn; gln; lys; arg | arg |
| Ile (I) | leu; val; met; ala; phe; norleucine | leu |
| Leu (L) | norleucine; ile; val; met; ala; phe | ile |
| Lys (K) | arg; gln; asn | arg |
| Met (M) | leu; phe; ile | leu |
| Phe (F) | leu; val; ile; ala; tyr | leu |
| Pro (P) | ala | ala |
| Ser (S) | thr | thr |
| Thr (T) | ser | ser |
| Trp (W) | tyr; phe | tyr |
| Tyr (Y) | trp; phe; thr; ser | phe |
| Val (V) | ile; leu; met; phe; ala; norleucine | leu |

Substantial modifications in function or immunological identity of the TWEAK polypeptide are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:
(1) hydrophobic: norleucine, met, ala, val, leu, ile;
(2) neutral hydrophilic: cys, ser, thr;
(3) acidic: asp, glu;
(4) basic: asn, gln, his, lys, arg;
(5) residues that influence chain orientation: gly, pro; and
(6) aromatic: trp, tyr, phe.

Non-conservative substitutions will entail exchanging a member of one of these classes for another class. Such substituted residues also may be introduced into the conservative substitution sites or, more preferably, into the remaining (non-conserved) sites.

The variations can be made using methods known in the art such as oligonucleotide-mediated (site-directed) mutagenesis, alanine scanning, and PCR mutagenesis. Site-directed mutagenesis [Carter et al., Nucl. Acids Res., 13:4331 (1986); Zoller et al., Nucl. Acids Res., 10:6487 (1987)], cassette mutagenesis [Wells et al., Gene, 34:315 (1985)], restriction selection mutagenesis [Wells et al., Philos. Trans. R. Soc. London SerA, 317:415 (1986)] or other known techniques can be performed on the cloned DNA to produce the TWEAK polypeptide variant DNA.

Scanning amino acid analysis can also be employed to identify one or more amino acids along a contiguous sequence. Among the preferred scanning amino acids are relatively small, neutral amino acids. Such amino acids include alanine, glycine, serine, and cysteine. Alanine is typically a preferred scanning amino acid among this group because it eliminates the side-chain beyond the beta-carbon and is less likely to alter the main-chain conformation of the variant [Cunningham and Wells, Science, 244:1081-1085 (1989)]. Alanine is also typically preferred because it is the most common amino acid. Further, it is frequently found in both buried and exposed positions [Creighton, The Proteins, (W.H. Freeman & Co., N.Y.); Chothia, J. Mol. Biol., 150:1 (1976)]. If alanine substitution does not yield adequate amounts of variant, an isoteric amino acid can be used.

Any cysteine residue not involved in maintaining the proper conformation of the TWEAK polypeptide also may be substituted, generally with serine, to improve the oxidative stability of the molecule and prevent aberrant crosslinking. Conversely, cysteine bond(s) may be added to the TWEAK polypeptide to improve its stability.

The description below relates primarily to production of TWEAK polypeptides by culturing cells transformed or transfected with a vector containing TWEAK polypeptide-encoding nucleic acid. It is, of course, contemplated that alternative methods, which are well known in the art, may be employed to prepare various TWEAK agonists and TWEAK antagonists contemplated herein. For instance, the appropriate amino acid sequence, or portions thereof, may be produced by direct peptide synthesis using solid-phase techniques [see, e.g., Stewart et al., Solid-Phase Peptide Synthesis, W.H. Freeman Co., San Francisco, CA (1969); Merrifield, J. Am. Chem. Soc., 85:2149-2154 (1963)]. *In vitro* protein synthesis may be performed using manual techniques or by automation. Automated synthesis may be accomplished, for instance, using an Applied Biosystems Peptide Synthesizer (Foster City, CA) using manufacturer's instructions. Various portions of the TWEAK polypeptide may be chemically synthesized separately and combined using chemical or enzymatic methods to produce the desired TWEAK polypeptide. The methods and techniques described are similarly applicable to production of TWEAK variants, modified forms of TWEAK and TWEAK antibodies.

### 1. Isolation of DNA Encoding TWEAK Polypeptide

DNA encoding TWEAK polypeptide may be obtained from a cDNA library prepared from tissue believed to possess the TWEAK polypeptide mRNA and to express it at a detectable level. Accordingly, human TWEAK polypeptide DNA can be conveniently obtained from a cDNA library prepared from human tissue. The TWEAK polypeptide-encoding gene may also be obtained from a genomic library or by known synthetic procedures (e.g., automated nucleic acid synthesis).

Libraries can be screened with probes (such as oligonucleotides of at least about 20-80 bases) designed to identify the gene of interest or the protein encoded by it. Screening the cDNA or genomic library with the selected probe may be conducted using standard procedures, such as described in Sambrook et al., Molecular Cloning: A Laboratory Manual (New York: Cold Spring Harbor Laboratory Press, 1989). An alternative means to isolate the gene encoding TWEAK polypeptide is to use PCR methodology [Sambrook et al., supra; Dieffenbach et al., PCR Primer: A Laboratory Manual (Cold Spring Harbor Laboratory Press, 1995)].

Techniques for screening a cDNA library are well known in the art. The oligonucleotide sequences selected as probes should be of sufficient length and sufficiently unambiguous that false positives are minimized. The oligonucleotide is preferably labeled such that it can be detected upon hybridization to DNA in the library being screened. Methods of labeling are well known in the art, and include the use of radiolabels like ³²P-labeled ATP, biotinylation or enzyme labelling. Hybridization conditions, including moderate stringency and high stringency, are provided in Sambrook et al., supra.

Sequences identified in such library screening methods can be compared and aligned to other known sequences deposited and available in public databases such as GenBank or other private sequence databases. Sequence identity (at either the amino acid or nucleotide level) within defined regions of the molecule or across the full-length sequence can be determined using methods known in the art and as described herein.

Nucleic acid having protein coding sequence may be obtained by screening selected cDNA or genomic libraries using the deduced amino acid sequence disclosed herein for the first time, and, if necessary, using conventional primer extension procedures as described in Sambrook et al., supra, to detect precursors and processing intermediates of mRNA that may not have been reverse-transcribed into cDNA.

### 2. Selection and Transformation of Host Cells

Host cells are transfected or transformed with expression or cloning vectors described herein for TWEAK polypeptide production and cultured in conventional nutrient media modified as appropriate for inducing promoters, selecting transformants, or amplifying the genes encoding the desired sequences. The culture conditions, such as media, temperature, pH and the like, can be selected by the skilled artisan without undue experimentation. In general, principles, protocols, and practical techniques for maximizing the productivity of cell cultures can be found in Mammalian Cell Biotechnology: a Practical Approach, M. Butler, ed. (IRL Press, 1991) and Sambrook et al., supra.

Methods of eukaryotic cell transfection and prokaryotic cell transformation are known to the ordinarily skilled artisan, for example, CaCl₂, CaPO₄, liposome-mediated and electroporation. Depending on the host cell used, transformation is performed using standard techniques appropriate to such cells. The calcium treatment employing calcium chloride, as described in Sambrook et al., supra, or electroporation is generally used for prokaryotes. Infection with *Agrobacterium tumefaciens* is used for transformation of certain plant cells, as described by Shaw et al., Gene, 23:315 (1983) and WO 89/05859 published 29 June 1989. For mammalian cells without such cell walls, the calcium phosphate precipitation method of Graham and van der Eb, Virology, 52:456-457 (1978) can be employed. General aspects of mammalian cell host system transfections have been described in U.S. Patent No. 4,399,216. Transformations into yeast are typically carried out according to the method of Van Solingen et al., J. Bact., 130:946 (1977) and Hsiao et al., Proc. Natl. Acad. Sci. (USA), 76:3829 (1979). However, other methods for introducing DNA into cells, such as by nuclear microinjection, electroporation, bacterial protoplast fusion with intact cells, or polycations, e.g., polybrene, polyornithine, may also be used. For various techniques for transforming mammalian cells, see Keown et al., Methods in Enzymology, 185:527-537 (1990) and Mansour et al., Nature, 336:348-352 (1988).

Suitable host cells for cloning or expressing the DNA in the vectors herein include prokaryote, yeast, or higher eukaryote cells. Suitable prokaryotes include but are not limited to eubacteria, such as Gram-negative or Gram-positive organisms, for example, Enterobacteriaceae such as *E. coli.* Various *E. coli* strains are publicly available, such as *E. coli* K12 strain MM294 (ATCC 31,446); *E. coli* X1776 (ATCC 31,537); *E. coli* strain W3110 (ATCC 27,325) and K5 772 (ATCC 53,635). Other suitable prokaryotic host cells include Enterobacteriaceae such as *Escherichia,* e.g., *E. coli, Enterobacter, Erwinia, Klebsiella, Proteus, Salmonella,* e.g., *Salmonella typhimurium, Serratia,* e.g., *Serratia marcescans,* and *Shigella,* as well as *Bacilli* such as *B. subtilis* and *B. licheniformis* (e.g., *B. licheniformis* 41P disclosed in DD 266,710 published 12 April 1989), *Pseudomonas* such as *P. aeruginosa,* and *Streptomyces.* These examples are illustrative rather than limiting. Strain W3110 is one particularly preferred host or parent host because it is a common host strain for recombinant DNA product fermentations. Preferably, the host cell secretes minimal amounts of proteolytic enzymes. For example, strain W3110 may be modified to effect a genetic mutation in the genes encoding proteins endogenous to the host, with examples of such hosts including *E. coli* W3110 strain 1A2, which has the complete genotype *tonA ; E. coli* W3110 strain 9E4, which has the complete genotype *tonA ptr3; E. coli* W3110 strain 27C7 (ATCC 55,244), which has the complete genotype *tonA ptr3 phoA E15 (argF-lac) 169 degP ompT kan^{r}; E. coli* W3110 strain 37D6, which has the complete genotype tonA *ptr3 phoA E15 (argF-lac)169 degP ompT rbs7 ilvG kan^{r}; E. coli* W3110 strain 40B4, which is strain 37D6 with a non-kanamycin resistant *degP* deletion mutation; and an *E. coli* strain having mutant periplasmic protease disclosed in U.S. Patent No. 4,946,783 issued 7 August 1990. Alternatively, *in vitro* methods of cloning, e.g., PCR or other nucleic acid polymerase reactions, are suitable.

In addition to prokaryotes, eukaryotic microbes such as filamentous fungi or yeast are suitable cloning or expression hosts for TWEAK polypeptide-encoding vectors. *Saccharomyces cerevisiae* is a commonly used lower eukaryotic host microorganism. Others include *Schizosaccharomyces* pombe (Beach and Nurse, Nature, 290: 140 [1981]; EP 139,383 published 2 May 1985); *Kluyveromyces* hosts (U.S. Patent No. 4,943,529; Fleer et al., Bio/Technology, 9:968-975 (1991)) such as, e.g., *K. lactis* (MW98-8C, CBS683, CBS4574; Louvencourt et al., J. Bacteriol., 154(2):737-742 [1983]), *K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906; Van den Berg et al., Bio/Technology, 8:135 (1990)), *K. thermotolerans,* and *K. marxianus; yarrowia* (EP 402,226); *Pichia pastoris* (EP 183,070; Sreekrishna et al., J. Basic Microbiol., 28:265-278 [1988]); *Candida; Trichoderma reesia* (EP 244,234); *Neurospora crassa* (Case et al., Proc. Natl. Acad. Sci. USA, 76:5259-5263 [1979]); *Schwanniomyces* such as *Schwanniomyces occidentalis* (EP 394,538 published 31 October 1990); and filamentous fungi such as, e.g., *Neurospora, Penicillium, Tolypocladium* (WO 91/00357 published 10 January 1991), and *Aspergillus* hosts such as *A. nidulans* (Ballance et al., Biochem. Biophys. Res. Commun., 112:284-289 [1983]; Tilburn et al., Gene, 26:205-221 [1983]; Yelton et al., Proc. Natl. Acad. Sci. USA, 81: 1470-1474 [1984]) and *A. niger* (Kelly and Hynes, EMBO J., 4:475-479 [1985]). Methylotropic yeasts are suitable herein and include, but are not limited to, yeast capable of growth on methanol selected from the genera consisting of *Hansenula, Candida, Kloeckera, Pichia, Saccharomyces, Torulopsis,* and *Rhodotorula*. A list of specific species that are exemplary of this class of yeasts may be found in C. Anthony, The Biochemistry of Methylotrophs, 269 (1982).

Suitable host cells for the expression of glycosylated TWEAK polypeptide are derived from multicellular organisms. Examples of invertebrate cells include insect cells such as Drosophila S2 and Spodoptera Sf9, as well as plant cells, such as cell cultures of cotton, corn, potato, soybean, petunia, tomato, and tobacco. Numerous baculoviral strains and variants and corresponding permissive insect host cells from hosts such as *Spodoptera frugiperda* (caterpillar), *Aedes aegypti* (mosquito), *Aedes albopictus* (mosquito), *Drosophila melanogaster* (fruitfly), and *Bombyx mori* have been identified. A variety of viral strains for transfection are publicly available, e.g., the L-1 variant of *Autographa californica* NPV and the Bm-5 strain of *Bombyx mori* NPV, and such viruses may be used as the virus herein according to the present invention, particularly for transfection of *Spodoptera frugiperda* cells.

However, interest has been greatest in vertebrate cells, and propagation of vertebrate cells in culture (tissue culture) has become a routine procedure. Examples of useful mammalian host cell lines are monkey kidney CV1 line transformed by SV40 (COS-7, ATCC CRL 1651); human embryonic kidney line (293 or 293 cells subcloned for growth in suspension culture, Graham et al., J. Gen Virol. 36:59 (1977)); baby hamster kidney cells (BHK, ATCC CCL 10); Chinese hamster ovary cells/-DHFR (CHO, Urlaub et al., Proc. Natl. Acad. Sci. USA 77:4216 (1980)); mouse sertoli cells (TM4, Mather, Biol. Reprod. 23:243-251 (1980)); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci. 383:44-68 (1982)); MRC 5 cells; FS4 cells; and a human hepatoma line (Hep G2).

Host cells are transformed with the above-described expression or cloning vectors for TWEAK polypeptide production and cultured in conventional nutrient media modified as appropriate for inducing promoters, selecting transformants, or amplifying the genes encoding the desired sequences.

### 3. Selection and Use of a Replicable Vector

The nucleic acid (e.g., cDNA or genomic DNA) encoding TWEAK polypeptide may be inserted into a replicable vector for cloning (amplification of the DNA) or for expression. Various vectors are publicly available. The vector may, for example, be in the form of a plasmid, cosmid, viral particle, or phage. The appropriate nucleic acid sequence may be inserted into the vector by a variety of procedures. In general, DNA is inserted into an appropriate restriction endonuclease site(s) using techniques known in the art. Vector components generally include, but are not limited to, one or more of a signal sequence, an origin of replication, one or more marker genes, an enhancer element, a promoter, and a transcription termination sequence. Construction of suitable vectors containing one or more of these components employs standard ligation techniques which are known to the skilled artisan.

The TWEAK polypeptide may be produced recombinantly not only directly, but also as a fusion polypeptide with a heterologous polypeptide, which may be a signal sequence or other polypeptide having a specific cleavage site at the N-terminus of the mature protein or polypeptide. In general, the signal sequence may be a component of the vector, or it may be a part of the TWEAK polypeptide-encoding DNA that is inserted into the vector. The signal sequence may be a prokaryotic signal sequence selected, for example, from the group of the alkaline phosphatase, penicillinase, lpp, or heat-stable enterotoxin II leaders. For yeast secretion the signal sequence may be, e.g., the yeast invertase leader, alpha factor leader (including *Saccharomyces* and *Kluyveromyces* α-factor leaders, the latter described in U.S. Patent No. 5,010,182), or acid phosphatase leader, the *C*. *albicans* glucoamylase leader (EP 362,179 published 4 April 1990), or the signal described in WO 90/13646 published 15 November 1990. In mammalian cell expression, mammalian signal sequences may be used to direct secretion of the protein, such as signal sequences from secreted polypeptides of the same or related species, as well as viral secretory leaders.

Both expression and cloning vectors contain a nucleic acid sequence that enables the vector to replicate in one or more selected host cells. Such sequences are well known for a variety of bacteria, yeast, and viruses. The origin of replication from the plasmid pBR322 is suitable for most Gram-negative bacteria, the 2µ plasmid origin is suitable for yeast, and various viral origins (SV40, polyoma, adenovirus, VSV or BPV) are useful for cloning vectors in mammalian cells.

Expression and cloning vectors will typically contain a selection gene, also termed a selectable marker. Typical selection genes encode proteins that (a) confer resistance to antibiotics or other toxins, e.g., ampicillin, neomycin, methotrexate, or tetracycline, (b) complement auxotrophic deficiencies, or (c) supply critical nutrients not available from complex media, e.g., the gene encoding D-alanine racemase for *Bacilli.*

An example of suitable selectable markers for mammalian cells are those that enable the identification of cells competent to take up the TWEAK polypeptide-encoding nucleic acid, such as DHFR or thymidine kinase. An appropriate host cell when wild-type DHFR is employed is the CHO cell line deficient in DHFR activity, prepared and propagated as described by Urlaub et al., Proc. Natl. Acad. Sci. USA, 77:4216 (1980). A suitable selection gene for use in yeast is the *trp*1 gene present in the yeast plasmid YRp7 [Stinchcomb et al., Nature, 282:39 (1979); Kingsman et al., Gene, 7:141 (1979); Tschemper et al., Gene, 10:157 (1980)]. The trp1 gene provides a selection marker for a mutant strain of yeast lacking the ability to grow in tryptophan, for example, ATCC No. 44076 or PEP4-1 [Jones, Genetics, 85:12 (1977)].

Expression and cloning vectors usually contain a promoter operably linked to the TWEAK polypeptide-encoding nucleic acid sequence to direct mRNA synthesis. Promoters recognized by a variety of potential host cells are well known. Promoters suitable for use with prokaryotic hosts include the β-lactamase and lactose promoter systems [Chang et al., Nature, 275:615 (1978); Goeddel et al., Nature, 281:544 (1979)], alkaline phosphatase, a tryptophan (trp) promoter system [Goeddel, Nucleic Acids Res., 8:4057 (1980); EP 36,776], and hybrid promoters such as the tac promoter [deBoer et al., Proc. Natl. Acad. Sci. USA, 80:21-25 (1983)]. Promoters for use in bacterial systems also will contain a Shine-Dalgarno (S.D.) sequence operably linked to the DNA encoding TWEAK polypeptide.

Examples of suitable promoting sequences for use with yeast hosts include the promoters for 3-phosphoglycerate kinase [Hitzeman et al., J. Biol. Chem., 255:2073 (1980)] or other glycolytic enzymes [Hess et al., J. Adv. Enzyme Reg., 7:149 (1968); Holland, Biochemistry, 17:4900 (1978)], such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657.

TWEAK polypeptide transcription from vectors in mammalian host cells is controlled, for example, by promoters obtained from the genomes of viruses such as polyoma virus, fowlpox virus (UK 2,211,504 published 5 July 1989), adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, e.g., the actin promoter or an immunoglobulin promoter, and from heat-shock promoters, provided such promoters are compatible with the host cell systems.

Transcription of a DNA encoding the TWEAK polypeptide by higher eukaryotes may be increased by inserting an enhancer sequence into the vector. Enhancers are cis-acting elements of DNA, usually about from 10 to 300 bp, that act on a promoter to increase its transcription. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. The enhancer may be spliced into the vector at a position 5' or 3' to the TWEAK polypeptide coding sequence, but is preferably located at a site 5' from the promoter.

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding TWEAK polypeptide.

Still other methods, vectors, and host cells suitable for adaptation to the synthesis of TWEAK polypeptide in recombinant vertebrate cell culture are described in Gething et al., Nature, 293:620-625 (1981); Mantei et al., Nature, 281:40-46 (1979); EP 117,060; and EP 117,058.

### 4. Culturing the Host Cells

The host cells used to produce the TWEAK polypeptide of this invention may be cultured in a variety of media. Commercially available media such as Ham's F10 (Sigma), Minimal Essential Medium ((MEM), (Sigma), RPMI-1640 (Sigma), and Dulbecco's Modified Eagle's Medium ((DMEM), Sigma) are suitable for culturing the host cells. In addition, any of the media described in Ham et al., Meth. Enz. 58:44 (1979), Barnes et al., Anal. Biochem. 102:255 (1980), U.S. Pat. Nos. 4,767,704; 4,657,866; 4,927,762; 4,560,655; or 5,122,469; WO 90/03430; WO 87/00195; or U.S. Patent Re. 30,985 may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleotides (such as adenosine and thymidine), antibiotics (such as GENTAMYCIN^{™} drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

### 5. Detecting Gene Amplification/Expression

Gene amplification and/or expression may be measured in a sample directly, for example, by conventional Southern blotting, Northern blotting to quantitate the transcription of mRNA [Thomas, Proc. Natl. Acad. Sci. USA, 77:5201-5205 (1980)], dot blotting (DNA analysis), semi-quantitative PCR, DNA array gene expression analysis, or in situ hybridization, using an appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

Gene expression, alternatively, may be measured by immunological methods, such as immunohistochemical staining of cells or tissue sections and assay of cell culture or body fluids, to quantitate directly the expression of gene product. Antibodies useful for immunohistochemical staining and/or assay of sample fluids may be either monoclonal or polyclonal, and may be prepared in any mammal. Conveniently, the antibodies may be prepared against a native sequence TWEAK polypeptide or against a synthetic peptide based on the DNA sequences provided herein or against exogenous sequence fused to TWEAK DNA and encoding a specific antibody epitope.

### 6. Purification of TWEAK Polypeptide

Forms of TWEAK polypeptide may be recovered from culture medium or from host cell lysates. If membrane-bound, it can be released from the membrane using a suitable detergent solution (e.g. Triton-X 100) or by enzymatic cleavage. Cells employed in expression of TWEAK polypeptide can be disrupted by various physical or chemical means, such as freeze-thaw cycling, sonication, mechanical disruption, or cell lysing agents.

It may be desired to purify TWEAK polypeptide from recombinant cell proteins or polypeptides. The following procedures are exemplary of suitable purification procedures: by fractionation on an ion-exchange column; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; protein A Sepharose columns to remove contaminants such as IgG; and metal chelating columns to bind epitope-tagged forms of the TWEAK polypeptide. Various methods of protein purification may be employed and such methods are known in the art and described for example in Deutscher, Methods in Enzymology, 182 (1990); Scopes, Protein Purification: Principles and Practice, Springer-Verlag, New York (1982). The purification step(s) selected will depend, for example, on the nature of the production process used and the particular TWEAK polypeptide produced.

Soluble forms of TWEAK may be employed in the methods of the invention. Such soluble forms of TWEAK may comprise modifications, as described below (such as by fusing to an immunoglobulin, epitope tag or leucine zipper). Immunoadhesin molecules are further contemplated for use in the methods herein. TWEAK receptor immunoadhesins may comprise various forms of TWEAK receptor, such as the full length polypeptide as well as soluble forms of the TWEAK receptor or a fragment thereof. In particular embodiments, the molecule may comprise a fusion of the TWEAK receptor polypeptide with an immunoglobulin or a particular region of an immunoglobulin. For a bivalent form of the immunoadhesin, such a fusion could be to the Fc region of an IgG molecule. The Ig fusions preferably include the substitution of a soluble (transmembrane domain deleted or inactivated) form of the polypeptide in place of at least one variable region within an Ig molecule. In a particularly preferred embodiment, the immunoglobulin fusion includes the hinge, CH2 and CH3, or the hinge, CH1, CH2 and CH3 regions of an IgG1 molecule. For the production of immunoglobulin fusions, see also US Patent No. 5,428,130 issued June 27, 1995 and Chamow et al., TIBTECH, 14:52-60 (1996).

The simplest and most straightforward immunoadhesin design combines the binding domain(s) of the adhesin (e.g. the TWEAK or TWEAK receptor) with the Fc region of an immunoglobulin heavy chain. Ordinarily, when preparing the immunoadhesins of the present invention, nucleic acid encoding the binding domain of the adhesin will be fused C-terminally to nucleic acid encoding the N-terminus of an immunoglobulin constant domain sequence, however N-terminal fusions are also possible.

Typically, in such fusions the encoded chimeric polypeptide will retain at least functionally active hinge, C_{H}2 and C_{H}3 domains of the constant region of an immunoglobulin heavy chain. Fusions are also made to the C-terminus of the Fc portion of a constant domain, or immediately N-terminal to the C_{H}1 of the heavy chain or the corresponding region of the light chain. The precise site at which the fusion is made is not critical; particular sites are well known and may be selected in order to optimize the biological activity, secretion, or binding characteristics of the immunoadhesin.

In a preferred embodiment, the adhesin sequence is fused to the N-terminus of the Fc region of immunoglobulin G₁ (IgG₁). It is possible to fuse the entire heavy chain constant region to the adhesin sequence. However, more preferably, a sequence beginning in the hinge region just upstream of the papain cleavage site which defines IgG Fc chemically (i.e. residue 216, taking the first residue of heavy chain constant region to be 114), or analogous sites of other immunoglobulins is used in the fusion. In a particularly preferred embodiment, the adhesin amino acid sequence is fused to (a) the hinge region and C_{H}2 and C_{H}3 or (b) the C_{H}1, hinge, C_{H}2 and C_{H}3 domains, of an IgG heavy chain.

For bispecific immunoadhesins, the immunoadhesins are assembled as multimers, and particularly as heterodimers or heterotetramers. Generally, these assembled immunoglobulins will have known unit structures. A basic four chain structural unit is the form in which IgG, IgD, and IgE exist. A four chain unit is repeated in the higher molecular weight immunoglobulins; IgM generally exists as a pentamer of four basic units held together by disulfide bonds. IgA globulin, and occasionally IgG globulin, may also exist in multimeric form in serum. In the case of multimer, each of the four units may be the same or different.

Various exemplary assembled immunoadhesins within the scope herein are schematically diagrammed below:
(a) AC_{L}-AC_{L};
(b) AC_{H}- (AC_{H}, AC_{L}-AC_{H}, AC_{L}-V_{H}C_{H}, or V_{L}C_{L}-AC_{H}) ;
(c) AC_{L}-AC_{H}-(AC_{L}-AC_{H}, AC_{L}-V_{H}C_{H}, V_{L}C_{L}-AC_{H}, or V_{L}C_{L}-V_{H}C_{H})
(d) AC_{L}-V_{H}C_{H}- (AC_{H}, or AC_{L}-V_{H}C_{H}, or V_{L}C_{L}-AC_{H});
(e) V_{L}C_{L}-AC_{H}- (AC_{L}-V_{H}C_{H}, or V_{L}C_{L}-AC_{H}) ; and
(f) (A-Y)ₙ-(V_{L}C_{L}-V_{H}C_{H})₂,
wherein each A represents identical or different adhesin amino acid sequences;
V_{L} is an immunoglobulin light chain variable domain;
V_{H} is an immunoglobulin heavy chain variable domain;
C_{L} is an immunoglobulin light chain constant domain;
C_{H} is an immunoglobulin heavy chain constant domain;
n is an integer greater than 1;
Y designates the residue of a covalent cross-linking agent.

In the interests of brevity, the foregoing structures only show key features; they do not indicate joining (J) or other domains of the immunoglobulins, nor are disulfide bonds shown. However, where such domains are required for binding activity, they shall be constructed to be present in the ordinary locations which they occupy in the immunoglobulin molecules.

Alternatively, the adhesin sequences can be inserted between immunoglobulin heavy chain and light chain sequences, such that an immunoglobulin comprising a chimeric heavy chain is obtained. In this embodiment, the adhesin sequences are fused to the 3' end of an immunoglobulin heavy chain in each arm of an immunoglobulin, either between the hinge and the C_{H}2 domain, or between the C_{H}2 and C_{H}3 domains. Similar constructs have been reported by Hoogenboom et al., Mol. Immunol., 28:1027-1037 (1991).

Although the presence of an immunoglobulin light chain is not required in the immunoadhesins of the present invention, an immunoglobulin light chain might be present either covalently associated to an adhesin-immunoglobulin heavy chain fusion polypeptide, or directly fused to the adhesin. In the former case, DNA encoding an immunoglobulin light chain is typically coexpressed with the DNA encoding the adhesin-immunoglobulin heavy chain fusion protein. Upon secretion, the hybrid heavy chain and the light chain will be covalently associated to provide an immunoglobulin-like structure comprising two disulfide-linked immunoglobulin heavy chain-light chain pairs. Methods suitable for the preparation of such structures are, for example, disclosed in U.S. Patent No. 4,816,567, issued 28 March 1989.

Immunoadhesins are most conveniently constructed by fusing the cDNA sequence encoding the adhesin portion in-frame to an immunoglobulin cDNA sequence. However, fusion to genomic immunoglobulin fragments can also be used (see, e.g. Aruffo et al., Cell, 61:1303-1313 (1990); and Stamenkovic et al., Cell, 66:1133-1144 (1991)). The latter type of fusion requires the presence of Ig regulatory sequences for expression. cDNAs encoding IgG heavy-chain constant regions can be isolated based on published sequences from cDNA libraries derived from spleen or peripheral blood lymphocytes, by hybridization or by polymerase chain reaction (PCR) techniques. The cDNAs encoding the "adhesin" and the immunoglobulin parts of the immunoadhesin are inserted in tandem into a plasmid vector that directs efficient expression in the chosen host cells.

In other embodiments, the TWEAK agonist or TWEAK antagonist may be covalently modified by linking the molecule to one of a variety of nonproteinaceous polymers, e.g., polyethylene glycol (PEG), polypropylene glycol, or polyoxyalkylenes, in the manner set forth in U.S. Patent Nos. 4,640,835; 4,496,689; 4,301,144; 4,670,417; 4,791,192 or 4,179,337, or other like molecules such as polyglutamate. Such pegylated forms may be prepared using techniques known in the art.

Leucine zipper forms of these molecules are also contemplated by the invention. "Leucine zipper" is a term in the art used to refer to a leucine rich sequence that enhances, promotes, or drives dimerization or trimerization of its fusion partner (e.g., the sequence or molecule to which the leucine zipper is fused or linked to). Various leucine zipper polypeptides have been described in the art. See, e.g., Landschulz et al., Science, 240:1759 (1988); US Patent 5,716,805; WO 94/10308; Hoppe et al., FEBS Letters, 344:1991 (1994); Maniatis et al., Nature, 341:24 (1989). Those skilled in the art will appreciate that a leucine zipper sequence may be fused at either the 5' or 3' end of the molecule.

The TWEAK agonists and TWEAK antagonists of the present invention may also be modified in a way to form chimeric molecules by fusing the polypeptide to another, heterologous polypeptide or amino acid sequence. Preferably, such heterologous polypeptide or amino acid sequence is one which acts to oligimerize the chimeric molecule. In one embodiment, such a chimeric molecule comprises a fusion of the polypeptide with a tag which provides an epitope to which an anti-tag antibody can selectively bind.
The epitope tag is generally placed at the amino- or carboxyl- terminus of the polypeptide. The presence of such epitope-tagged forms of the polypeptide can be detected using an antibody against the tag polypeptide. Also, provision of the epitope tag enables the polypeptide to be readily purified by affinity purification using an anti-tag antibody or another type of affinity matrix that binds to the epitope tag. Various tag polypeptides and their respective antibodies are well known in the art. Examples include poly-histidine (poly-his) or poly-histidine-glycine (poly-his-gly) tags; the flu HA tag polypeptide and its antibody 12CA5 [Field et al., Mol. Cell. Biol., 8:2159-2165 (1988)]; the c-myc tag and the 8F9, 3C7, 6E10, G4, B7 and 9E10 antibodies thereto [Evan et al., Molecular and Cellular Biology, 5:3610-3616 (1985)]; and the Herpes Simplex virus glycoprotein D (gD) tag and its antibody [Paborsky et al., Protein Engineering, 3(6):547-553 (1990)]. Other tag polypeptides include the Flag-peptide [Hopp et al., BioTechnology, 6:1204-1210 (1988)]; the KT3 epitope peptide [Martin et al., Science, 255:192-194 (1992)]; an α-tubulin epitope peptide [Skinner et al., J. Biol. Chem., 266:15163-15166 (1991)]; and the T7 gene 10 protein peptide tag [Lutz-Freyermuth et al., Proc. Natl. Acad. Sci. USA, 87:6393-6397 (1990)].

It is contemplated that anti-TWEAK or anti-TWEAK receptor antibodies may also be employed in the presently disclosed methods. These antibodies may be monoclonal antibodies. One skilled in the art can utilize methods known in the art, and described herein, to identify TWEAK antibodies or TWEAK receptor antibodies which act as agonists or antagonists of TWEAK or TWEAK receptor activity(s).

Monoclonal antibodies may be prepared using hybridoma methods, such as those described by Kohler and Milstein, Nature, 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes may be immunized *in vitro.*

The immunizing agent will typically include a TWEAK polypeptide or TWEAK receptor or a fusion protein thereof, such as a TWEAK-IgG fusion protein. Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell [Goding, Monoclonal Antibodies: Principles and Practice, Academic Press, (1986) pp. 59-103]. Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection, Manassas, Virginia. Human myeloma and mouse-human heteromyeloma cell lines also have been described for the production of human monoclonal antibodies [Kozbor, J. Immunol., 133:3001 (1984); Brodeur et al., Monoclonal Antibody Production Techniques and Applications, Marcel Dekker, Inc., New York, (1987) pp. 51-63].

The culture medium in which the hybridoma cells are cultured can then be assayed for the presence of monoclonal antibodies directed against TWEAK or TWEAK receptor. Optionally, the binding specificity of monoclonal antibodies produced by the hybridoma cells is determined by immunoprecipitation or by an in vitro binding assay, such as radioimmunoassay (RIA) or enzyme-linked immunoabsorbent assay (ELISA), and preferably by way of BIAcore assay. Such techniques and assays are known in the art. The binding affinity of the monoclonal antibody can, for example, be determined by the Scatchard analysis of Munson and Pollard, Anal. Biochem., 107:220 (1980).

After the desired hybridoma cells are identified, the clones may be subcloned by limiting dilution procedures and grown by standard methods [Goding, supra]. Suitable culture media for this purpose include, for example, Dulbecco's Modified Eagle's Medium or RPMI-1640 medium. Alternatively, the hybridoma cells may be grown *in vivo* as ascites in a mammal.

The monoclonal antibodies secreted by the subclones may be isolated or purified from the culture medium or ascites fluid by conventional immunoglobulin purification procedures such as, for example, protein A-Sepharose, hydroxylapatite chromatography, gel electrophoresis, dialysis, or affinity chromatography.

The monoclonal antibodies may also be made by recombinant DNA methods, such as those described in U.S. Patent No. 4,816,567. DNA encoding the monoclonal antibodies is readily isolated and sequenced using conventional procedures (*e.g.*, by using oligonucleotide probes that are capable of binding specifically to genes encoding the heavy and light chains of the monoclonal antibodies). The hybridoma cells serve as a preferred source of such DNA. Once isolated, the DNA may be placed into expression vectors, which are then transfected into host cells such as *E. coli* cells, simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for human heavy and light chain constant domains in place of the homologous murine sequences, Morrison, et al., Proc. Nat. Acad. Sci. 81, 6851 (1984), or by covalently joining to the immunoglobulin coding sequence all or part of the coding sequence for a non-immunoglobulin polypeptide.

Typically such non-immunoglobulin polypeptides are substituted for the constant domains of an antibody of the invention, or they are substituted for the variable domains of one antigen-combining site of an antibody of the invention to create a chimeric bivalent antibody comprising one antigen-combining site having specificity for TWEAK or TWEAK receptor and another antigen-combining site having specificity for a different antigen.

Chimeric or hybrid antibodies also may be prepared in vitro using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate.

Single chain Fv fragments may also be produced, such as described in Iliades et al., FEBS Letters, 409:437-441 (1997). Coupling of such single chain fragments using various linkers is described in Kortt et al., Protein Engineering, 10:423-433 (1997). A variety of techniques for the recombinant production and manipulation of antibodies are well known in the art. Illustrative examples of such techniques that are typically utilized by skilled artisans are described in greater detail below.

### (i) Humanized antibodies

Generally, a humanized antibody has one or more amino acid residues introduced into it from a non-human source. These non-human amino acid residues are often referred to as "import" residues, which are typically taken from an "import" variable domain. Humanization can be essentially performed following the method of Winter and co-workers [Jones et al., Nature, 321:522-525 (1986); Riechmann et al., Nature, 332:323-327 (1988); Verhoeyen et al., Science, 239:1534-1536 (1988)], by substituting rodent CDRs or CDR sequences for the corresponding sequences of a human antibody.

Accordingly, such "humanized" antibodies are chimeric antibodies wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

It is important that antibodies be humanized with retention of high affinity for the antigen and other favorable biological properties. To achieve this goal, according to a preferred method, humanized antibodies are prepared by a process of analysis of the parental sequences and various conceptual humanized products using three dimensional models of the parental and humanized sequences. Three dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i.e. the analysis of residues that influence the ability of the candidate immunoglobulin to bind its antigen. In this way, FR residues can be selected and combined from the consensus and import sequence so that the desired antibody characteristic, such as increased affinity for the target antigen(s), is achieved. In general, the CDR residues are directly and most substantially involved in influencing antigen binding.

### (ii) Human antibodies

Human monoclonal antibodies can be made by the hybridoma method. Human myeloma and mouse-human heteromyeloma cell lines for the production of human monoclonal antibodies have been described, for example, by Kozbor, J. Immunol. 133, 3001 (1984), and Brodeur, et al., Monoclonal Antibody Production Techniques and Applications, pp.51-63 (Marcel Dekker, Inc., New York, 1987).

It is now possible to produce transgenic animals (e.g. mice) that are capable, upon immunization, of producing a repertoire of human antibodies in the absence of endogenous immunoglobulin production. For example, it has been described that the homozygous deletion of the antibody heavy chain joining region (J_{H}) gene in chimeric and germ-line mutant mice results in complete inhibition of endogenous antibody production. Transfer of the human germ-line immunoglobulin gene array in such germ-line mutant mice will result in the production of human antibodies upon antigen challenge. See, e.g. Jakobovits et al., Proc. Natl. Acad. Sci. USA 90, 2551-255 (1993); Jakobovits et al., Nature 362, 255-258 (1993).

Mendez et al. (Nature Genetics 15: 146-156 [1997]) have further improved the technology and have generated a line of transgenic mice designated as "Xenomouse II" that, when challenged with an antigen, generates high affinity fully human antibodies. This was achieved by germ-line integration of megabase human heavy chain and light chain loci into mice with deletion into endogenous J_{H} segment as described above. The Xenomouse II harbors 1,020 kb of human heavy chain locus containing approximately 66 V_{H} genes, complete D_{H} and J_{H} regions and three different constant regions (µ, δ and χ), and also harbors 800 kb of human κ locus containing 32 Vκ genes, Jκ segments and Cκ genes. The antibodies produced in these mice closely resemble that seen in humans in all respects, including gene rearrangement, assembly, and repertoire. The human antibodies are preferentially expressed over endogenous antibodies due to deletion in endogenous J_{H} segment that prevents gene rearrangement in the murine locus.

Alternatively, the phage display technology (McCafferty et al., Nature 348, 552-553 [1990]) can be used to produce human antibodies and antibody fragments in vitro, from immunoglobulin variable (V) domain gene repertoires from unimmunized donors. According to this technique, antibody V domain genes are cloned in-frame into either a major or minor coat protein gene of a filamentous bacteriophage, such as M13 or fd, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. Thus, the phage mimicks some of the properties of the B-cell. Phage display can be performed in a variety of formats; for their review see, e.g. Johnson, Kevin S. and Chiswell, David J., Current Opinion in Structural Biology 3, 564-571 (1993). Several sources of V-gene segments can be used for phage display. Clackson et al., Nature 352, 624-628 (1991) isolated a diverse array of anti-oxazolone antibodies from a small random combinatorial library of V genes derived from the spleens of immunized mice. A repertoire of V genes from unimmunized human donors can be constructed and antibodies to a diverse array of antigens (including self-antigens) can be isolated essentially following the techniques described by Marks et al., J. Mol. Biol. 222, 581-597 (1991), or Griffith et al., EMBO J. 12, 725-734 (1993). In a natural immune response, antibody genes accumulate mutations at a high rate (somatic hypermutation). Some of the changes introduced will confer higher affinity, and B cells displaying high-affinity surface immunoglobulin are preferentially replicated and differentiated during subsequent antigen challenge. This natural process can be mimicked by employing the technique known as "chain shuffling" (Marks et al., Bio/Technol. 10, 779-783 [1992]). In this method, the affinity of "primary" human antibodies obtained by phage display can be improved by sequentially replacing the heavy and light chain V region genes with repertoires of naturally occurring variants (repertoires) of V domain genes obtained from unimmunized donors. This technique allows the production of antibodies and antibody fragments with affinities in the nM range. A strategy for making very large phage antibody repertoires (also known as "the mother-of-all libraries") has been described by Waterhouse et al., Nucl. Acids Res. 21, 2265-2266 (1993). Gene shuffling can also be used to derive human antibodies from rodent antibodies, where the human antibody has similar affinities and specificities to the starting rodent antibody. According to this method, which is also referred to as "epitope imprinting", the heavy or light chain V domain gene of rodent antibodies obtained by phage display technique is replaced with a repertoire of human V domain genes, creating rodent-human chimeras. Selection on antigen results in isolation of human variable capable of restoring a functional antigen-binding site, i.e. the epitope governs (imprints) the choice of partner. When the process is repeated in order to replace the remaining rodent V domain, a human antibody is obtained (see PCT patent application WO 93/06213, published 1 April 1993). Unlike traditional humanization of rodent antibodies by CDR grafting, this technique provides completely human antibodies, which have no framework or CDR residues of rodent origin.

As discussed below, the antibodies of the invention may optionally comprise monomeric antibodies, dimeric antibodies, as well as multivalent forms of antibodies. Those skilled in the art may construct such dimers or multivalent forms by techniques known in the art. Methods for preparing monovalent antibodies are also well known in the art. For example, one method involves recombinant expression of immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking.

### (iii) Bispecific antibodies

Bispecific antibodies are monoclonal, preferably human or humanized, antibodies that have binding specificities for at least two different antigens. In the present case, one of the binding specificities is for TWEAK or TWEAK receptor.

Methods for making bispecific antibodies are known in the art. Traditionally, the recombinant production of bispecific antibodies is based on the coexpression of two immunoglobulin heavy chain-light chain pairs, where the two heavy chains have different specificities (Millstein and Cuello, Nature 305, 537-539 (1983)). Because of the random assortment of immunoglobulin heavy and light chains, these hybridomas (quadromas) produce a potential mixture of 10 different antibody molecules, of which only one has the correct bispecific structure. The purification of the correct molecule, which is usually done by affinity chromatography steps, is rather cumbersome, and the product yields are low. Similar procedures are disclosed in PCT application publication No. WO 93/08829 (published 13 May 1993), and in Traunecker et al., EMBO 10, 3655-3659 (1991).

According to a different and more preferred approach, antibody variable domains with the desired binding specificities (antibody-antigen combining sites) are fused to immunoglobulin constant domain sequences. The fusion preferably is with an immunoglobulin heavy chain constant domain, comprising at least part of the hinge, CH2 and CH3 regions. It is preferred to have the first heavy chain constant region (CH1) containing the site necessary for light chain binding, present in at least one of the fusions. DNAs encoding the immunoglobulin heavy chain fusions and, if desired, the immunoglobulin light chain, are inserted into separate expression vectors, and are cotransfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance. In a preferred embodiment of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in PCT Publication No. WO 94/04690, published on March 3, 1994.

For further details of generating bispecific antibodies see, for example, Suresh et al., Methods in Enzymology 121, 210 (1986).

### (iv) Heteroconjugate antibodies

Heteroconjugate antibodies are also within the scope of the present invention. Heteroconjugate antibodies are composed of two covalently joined antibodies. Such antibodies have, for example, been proposed to target immune system cells to unwanted cells (U.S. Patent No. 4,676,980), and for treatment of HIV infection (PCT application publication Nos. WO 91/00360 and WO 92/200373; EP 03089). Heteroconjugate antibodies may be made using any convenient cross-linking methods. Suitable cross-linking agents are well known in the art, and are disclosed in U.S. Patent No. 4,676,980, along with a number of cross-linking techniques.

### (v) Antibody fragments

In certain embodiments, the anti-TWEAK or anti-TWEAK receptor antibody (including murine, human and humanized antibodies, and antibody variants) is an antibody fragment. Various techniques have been developed for the production of antibody fragments. Traditionally, these fragments were derived via proteolytic digestion of intact antibodies (see, *e.g.*, Morimoto et al., J. Biochem. Biophys. Methods 24:107-117 (1992) and Brennan et al., Science 229:81 (1985)). However, these fragments can now be produced directly by recombinant host cells. For example, Fab'-SH fragments can be directly recovered from *E. coli* and chemically coupled to form F(ab')₂ fragments (Carter et al., Bio/Technology 10:163-167 (1992)). In another embodiment, the F(ab')₂ is formed using the leucine zipper GCN4 to promote assembly of the F(ab')₂ molecule. According to another approach, Fv, Fab or F(ab')₂ fragments can be isolated directly from recombinant host cell culture. A variety of techniques for the production of antibody fragments will be apparent to the skilled practitioner. For instance, digestion can be performed using papain. Examples of papain digestion are described in WO 94/29348 published 12/22/94 and U.S. Patent No. 4,342,566. Papain digestion of antibodies typically produces two identical antigen binding fragments, called Fab fragments, each with a single antigen binding site, and a residual Fc fragment. Pepsin treatment yields an F(ab')₂ fragment that has two antigen combining sites and is still capable of cross-linking antigen.

The Fab fragments produced in the antibody digestion also contain the constant domains of the light chain and the first constant domain (CH₁) of the heavy chain. Fab' fragments differ from Fab fragments by the addition of a few residues at the carboxy terminus of the heavy chain CH₁ domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments which have hinge cysteines between them. Other chemical couplings of antibody fragments are also known.

Antibodies are glycosylated at conserved positions in their constant regions (Jefferis and Lund, Chem. Immunol. 65:111-128 [1997]; Wright and Morrison, TibTECH 15:26-32 [1997]). The oligosaccharide side chains of the immunoglobulins affect the protein's function (Boyd et al., Mol. Immunol. 32:1311-1318 [1996]; Wittwe and Howard, Biochem. 29:4175-4180 [1990]), and the intramolecular interaction between portions of the glycoprotein which can affect the conformation and presented three-dimensional surface of the glycoprotein (Hefferis and Lund, *supra;* Wyss and Wagner, Current Opin. Biotech. 7:409-416 [1996]). Oligosaccharides may also serve to target a given glycoprotein to certain molecules based upon specific recognition structures. For example, it has been reported that in a galactosylated IgG, the oligosaccharide moiety 'flips' out of the inter-CH2 space and terminal N-acetylglucosamine residues become available to bind mannose binding protein (Malhotra et al., Nature Med. 1:237-243 [1995]). Removal by glycopeptidase of the oligosaccharides from CAMPATH-1H (a recombinant humanized murine monoclonal IgG1 antibody which recognizes the CDw52 antigen of human lymphocytes) produced in Chinese Hamster Ovary (CHO) cells resulted in a complete reduction in complement mediated lysis (CMCL) (Boyd et al., Mol. Immunol. 32:1311-1318 [1996]), while selective removal of sialic acid residues using neuraminidase resulted in no loss of DMCL. Glycosylation of antibodies has also been reported to affect antibody-dependent cellular cytotoxicity (ADCC). In particular, CHO cells with tetracycline-regulated expression of β(1,4)-N-acetylglucosaminyltransferase III (GnTIII), a glycosyltransferase catalyzing formation of bisecting GlcNAc, was reported to have improved ADCC activity (Umana et al., Mature Biotech. 17:176-180 [1999]).

Glycosylation variants of antibodies are variants in which the glycosylation pattern of an antibody is altered. By altering is meant deleting one or more carbohydrate moieties found in the antibody, adding one or more carbohydrate moieties to the antibody, changing the composition of glycosylation (glycosylation pattern), the extent of glycosylation, etc. Glycosylation variants may, for example, be prepared by removing, changing and/or adding one or more glycosylation sites in the nucleic acid sequence encoding the antibody.

Glycosylation of antibodies is typically either N-linked or O-linked. N-linked refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-aceylgalactosamine, galactose, or xylose to a hydroxyamino acid, most commonly serine or threonine, although 5-hydroxyproline or 5-hydroxylysine may also be used.

Addition of glycosylation sites to the antibody is conveniently accomplished by altering the amino acid sequence such that it contains one or more of the above-described tripeptide sequences (for N-linked glycosylation sites). The alteration may also be made by the addition of, or substitution by, one or more serine or threonine residues to the sequence of the original antibody (for O-linked glycosylation sites).

The glycosylation (including glycosylation pattern) of antibodies may also be altered without altering the underlying nucleotide sequence. Glycosylation largely depends on the host cell used to express the antibody. Since the cell type used for expression of recombinant glycoproteins, e.g. antibodies, as potential therapeutics is rarely the native cell, significant variations in the glycosylation pattern of the antibodies can be expected (see, e.g. Hse et al., J. Biol. Chem. 272:9062-9070 [1997]). In addition to the choice of host cells, factors which affect glycosylation during recombinant production of antibodies include growth mode, media formulation, culture density, oxygenation, pH, purification schemes and the like. Various methods have been proposed to alter the glycosylation pattern achieved in a particular host organism including introducing or overexpressing certain enzymes involved in oligosaccharide production (U. S. Patent Nos. 5,047,335; 5,510,261 and 5.278,299). Glycosylation, or certain types of glycosylation, can be enzymatically removed from the glycoprotein, for example using endoglycosidase H (Endo H). In addition, the recombinant host cell can be genetically engineered, e.g. make defective in processing certain types of polysaccharides. These and similar techniques are well known in the art.

The glycosylation structure of antibodies can be readily analyzed by conventional techniques of carbohydrate analysis, including lectin chromatography, NMR, Mass spectrometry, HPLC, GPC, monosaccharide compositional analysis, sequential enzymatic digestion, and HPAEC-PAD, which uses high pH anion exchange chromatography to separate oligosaccharides based on charge. Methods for releasing oligosaccharides for analytical purposes are also known, and include, without limitation, enzymatic treatment (commonly performed using peptide-N-glycosidase F/endo-β-galactosidase), elimination using harsh alkaline environment to release mainly O-linked structures, and chemical methods using anhydrous hydrazine to release both N- and O-linked oligosaccharides.

Triabodies are also within the scope of the invention. Such antibodies are described for instance in Iliades et al., supra and Kortt et al., supra.

The antibodies of the present invention may be modified by conjugating the antibody to a cytotoxic agent (like a toxin molecule) or a prodrug-activating enzyme which converts a prodrug (e.g. a peptidyl chemotherapeutic agent, see WO81/01145) to an active anti-cancer drug. See, for example, WO 88/07378 and U.S. Patent No. 4,975,278. This technology is also referred to as "Antibody Dependent Enzyme Mediated Prodrug Therapy" (ADEPT).

The enzyme component of the immunoconjugate useful for ADEPT includes any enzyme capable of acting on a prodrug in such a way so as to covert it into its more active, cytotoxic form. Enzymes that are useful in the method of this invention include, but are not limited to, alkaline phosphatase useful for converting phosphate-containing prodrugs into free drugs; arylsulfatase useful for converting sulfate-containing prodrugs into free drugs; cytosine deaminase useful for converting non-toxic 5-fluorocytosine into the anti-cancer drug, 5-fluorouracil; proteases, such as serratia protease, thermolysin, subtilisin, carboxypeptidases and cathepsins (such as cathepsins B and L), that are useful for converting peptide-containing prodrugs into free drugs; caspases such as caspase-3; D-alanylcarboxypeptidases, useful for converting prodrugs that contain D-amino acid substituents; carbohydrate-cleaving enzymes such as beta-galactosidase and neuraminidase useful for converting glycosylated prodrugs into free drugs; beta-lactamase useful for converting drugs derivatized with beta-lactams into free drugs; and penicillin amidases, such as penicillin V amidase or penicillin G amidase, useful for converting drugs derivatized at their amine nitrogens with phenoxyacetyl or phenylacetyl groups, respectively, into free drugs. Alternatively, antibodies with enzymatic activity, also known in the art as "abzymes", can be used to convert the prodrugs of the invention into free active drugs (see, e.g., Massey, Nature 328: 457-458 (1987)). Antibody-abzyme conjugates can be prepared as described herein for delivery of the abzyme to a tumor cell population.

The enzymes can be covalently bound to the antibodies by techniques well known in the art such as the use of heterobifunctional crosslinking reagents. Alternatively, fusion proteins comprising at least the antigen binding region of an antibody of the invention linked to at least a functionally active portion of an enzyme of the invention can be constructed using recombinant DNA techniques well known in the art (see, *e.g.*, Neuberger et al., Nature, 312: 604-608 (1984).

Further antibody modifications are contemplated. For example, the antibody may be linked to one of a variety of nonproteinaceous polymers, *e.g.*, polyethylene glycol, polypropylene glycol, polyoxyalkylenes, or copolymers of polyethylene glycol and polypropylene glycol, or other molecules such as polyglutamate. The antibody also may be entrapped in microcapsules prepared, for example, by coacervation techniques or by interfacial polymerization (for example, hydroxymethylcellulose or gelatin-microcapsules and poly-(methylmethacylate) microcapsules, respectively), in colloidal drug delivery systems (for example, liposomes, albumin microspheres, microemulsions, nano-particles and nanocapsules), or in macroemulsions. Such techniques are disclosed in Remington's Pharmaceutical Sciences, 16th edition, Osol, A., Ed., (1980). To increase the serum half life of the antibody, one may incorporate a salvage receptor binding epitope into the antibody (especially an antibody fragment) as described in U.S. Patent 5,739,277, for example. As used herein, the term "salvage receptor binding epitope" refers to an epitope of the Fc region of an IgG molecule (*e.g.,* IgG₁, IgG₂, IgG₃, or IgG₄) that is responsible for increasing the *in vivo* serum half-life of the IgG molecule.

Alternatively, or additionally, one may increase, or decrease, serum half-life by altering the amino acid sequence of the Fc region of an antibody to generate variants with altered FcRn binding. Antibodies with altered FcRn binding and/or serum half life are described in WO00/42072 (Presta, L.).

The antibodies of the invention may be stabilized by polymerization. This may be accomplished by crosslinking monomer chains with polyfunctional crosslinking agents, either directly or indirectly, through multi-functional polymers. Ordinarily, two substantially identical polypeptides are crosslinked at their C- or N-termini using a bifunctional crosslinking agent. The agent is used to crosslink the terminal amino and/or carboxyl groups. Generally, both terminal carboxyl groups or both terminal amino groups are crosslinked to one another, although by selection of the appropriate crosslinking agent the alpha amino of one polypeptide is crosslinked to the terminal carboxyl group of the other polypeptide. Preferably, the polypeptides are substituted at their C-termini with cysteine. Under conditions well known in the art a disulfide bond can be formed between the terminal cysteines, thereby crosslinking the polypeptide chains. For example, disulfide bridges are conveniently formed by metal-catalyzed oxidation of the free cysteines or by nucleophilic substitution of a suitably modified cysteine residue. Selection of the crosslinking agent will depend upon the identities of the reactive side chains of the amino acids present in the polypeptides. For example, disulfide crosslinking would not be preferred if cysteine was present in the polypeptide at additional sites other than the C-terminus. Also within the scope hereof are peptides crosslinked with methylene bridges.

Suitable crosslinking sites on the antibodies, aside from the N-terminal amino and C-terminal carboxyl groups, include epsilon amino groups found on lysine residues, as well as amino, imino, carboxyl, sulfhydryl and hydroxyl groups located on the side chains of internal residues of the peptides or residues introduced into flanking sequences. Crosslinking through externally added crosslinking agents is suitably achieved, *e.g.*, using any of a number of reagents familiar to those skilled in the art, for example, via carbodiimide treatment of the polypeptide. Other examples of suitable multi-functional (ordinarily bifunctional) crosslinking agents are found in the literature.

In the preparation of typical formulations herein, it is noted that the recommended quality or "grade" of the components employed will depend on the ultimate use of the formulation. For therapeutic uses, it is preferred that the component(s) are of an allowable grade (such as "GRAS") as an additive to pharmaceutical products.

In certain embodiments, there are provided compositions comprising antagonists or agonists and one or more excipients which provide sufficient ionic strength to enhance solubility and/or stability, wherein the composition has a pH of 6 (or about 6) to 9 (or about 9). The antagonist or agonist may be prepared by any suitable method to achieve the desired purity, for example, according to the above methods. In certain embodiments, the antagonist or agonist is recombinantly expressed in host cells or prepared by chemical synthesis. The concentration of the antagonist or agonist in the formulation may vary depending, for instance, on the intended use of the formulation. Those skilled in the art can determine without undue experimentation the desired concentration of the antagonist or agonist.

The one or more excipients in the formulations which provide sufficient ionic strength to enhance solubility and/or stability of the antagonist or agonist is optionally a polyionic organic or inorganic acid, aspartate, sodium sulfate, sodium succinate, sodium acetate, sodium chloride, Captisol^{™}, Tris, arginine salt or other amino acids, sugars and polyols such as trehalose and sucrose. Preferably the one or more excipients in the formulations which provide sufficient ionic strength is a salt. Salts which may be employed include but are not limited to sodium salts and arginine salts. The type of salt employed and the concentration of the salt are preferably such that the formulation has a relatively high ionic strength which allows the antagonist or agonist in the formulation to be stable. Optionally, the salt is present in the formulation at a concentration of about 20 mM to about 0.5 M.

The composition preferably has a pH of 6 (or about 6) to 9 (or about 9), more preferably about 6.5 to about 8.5, and even more preferably about 7 to about 7.5. In a preferred aspect of this embodiment, the composition will further comprise a buffer to maintain the pH of the composition at least about 6 to about 8. Examples of buffers which may be employed include but are not limited to Tris, HEPES, and histidine. When employing Tris, the pH may optionally be adjusted to about 7 to 8.5. When employing Hepes or histidine, the pH may optionally be adjusted to about 6.5 to 7. Optionally, the buffer is employed at a concentration of about 5 mM to about 50 mM in the formulation.

Particularly for liquid formulations (or reconstituted lyophilized formulations), it may be desirable to include one or more surfactants in the composition. Such surfactants may, for instance, comprise a non-ionic surfactant like TWEEN^{™} or PLURONICS^{™} (e.g., polysorbate or poloxamer). Preferably, the surfactant comprises polysorbate 20 ("Tween 20"). The surfactant will optionally be employed at a concentration of about 0.005% to about 0.2%.

The formulations of the present invention may include, in addition to antagonist or agonist and those components described above, further various other excipients or components. Optionally, the formulation may contain, for parenteral administration, a pharmaceutically or parenterally acceptable carrier, i.e., one that is non-toxic to recipients at the dosages and concentrations employed and is compatible with other ingredients of the formulation. Optionally, the carrier is a parenteral carrier, such as a solution that is isotonic with the blood of the recipient. Examples of such carrier vehicles include water, saline or a buffered solution such as phosphate-buffered saline (PBS), Ringer's solution, and dextrose solution. Various optional pharmaceutically acceptable carriers, excipients, or stabilizers are described further in Remington's Pharmaceutical Sciences, 16th edition, Osol, A. ed. (1980).

The formulations herein also may contain one or more preservatives. Examples include octadecyldimethylbenzyl ammonium chloride, hexamethonium chloride, benzalkonium chloride (a mixture of alkylbenzyldimethylammonium chlorides in which the alkyl groups are long-chain compounds), and benzethonium chloride. Other types of preservatives include aromatic alcohols, alkyl parabens such as methyl or propyl paraben, and m-cresol. Antioxidants include ascorbic acid and methionine; preservatives (such as octadecyldimethylbenzyl ammonium chloride; hexamethonium chloride; benzalkonium chloride, benzethonium chloride; butyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; sugars such as sucrose, mannitol, trehalose or sorbitol; or polyethylene glycol (PEG).

Additional examples of such carriers include lecithin, serum proteins, such as human serum albumin, buffer substances such as glycine, sorbic acid, potassium sorbate, partial glyceride mixtures of saturated vegetable fatty acids, water, salts, or electrolytes such as protamine sulfate, sodium chloride, polyvinyl pyrrolidone, and cellulose-based substances. Carriers for gel-based forms include polysaccharides such as sodium carboxymethylcellulose or methylcellulose, polyvinylpyrrolidone, polyacrylates, polyoxyethylene-polyoxypropylene-block polymers, polyethylene glycol, and wood wax alcohols. Conventional depot forms include, for example, microcapsules, nano-capsules, liposomes, plasters, inhalation forms, nose sprays, and sustained-release preparations.

The compositions of the invention may comprise liquid formulations (liquid solutions or liquid suspensions), and lyophilized formulations, as well as suspension formulations in which the TWEAK antagonist or TWEAK agonist is in the form of crystals or amorphous precipitate.

The final formulation, if a liquid, is preferably stored frozen at ≤ 20° C. Alternatively, the formulation can be lyophilized and provided as a powder for reconstitution with water for injection that optionally may be stored at 2-30° C.

The formulation to be used for therapeutic administration must be sterile. Sterility is readily accomplished by filtration through sterile filtration membranes (*e.g.,* 0.2 micron membranes). Therapeutic compositions generally are placed into a container having a sterile access port, for example, an intravenous solution bag or vial having a stopper pierceable by a hypodermic injection needle.

The composition ordinarily will be stored in single unit or multidose containers, for example, sealed ampules or vials, as an aqueous solution or as a lyophilized formulation for reconstitution. The containers may any available containers in the art and filled using conventional methods. Optionally, the formulation may be included in an injection pen device (or a cartridge which fits into a pen device), such as those available in the art (see, e.g., US Patent 5,370,629), which are suitable for therapeutic delivery of the formulation. An injection solution can be prepared by reconstituting the lyophilized antagonist or agonist formulation using, for example, Water-for-Injection.

The compositions described herein which modulate TWEAK or TWEAK receptor activity(s) can be employed in a variety of therapeutic applications. TWEAK antagonists may for instance, be employed in methods of treating cancer while TWEAK agonists find utility in treating a variety of immune related conditions.

In the methods of the invention for treating such a disorder, a formulation of antagonist or agonist can be directly administered to the mammal by any suitable technique, including infusion or injection. The specific route of administration will depend, e.g., on the medical history of the patient, including any perceived or anticipated side effects using antagonist or agonist and the particular disorder to be corrected. Examples of parenteral administration include subcutaneous, intramuscular, intravenous, intraarterial, and intraperitoneal administration of the composition. The formulations are preferably administered as repeated intravenous (i.v.), subcutaneous (s.c.), intramuscular (i.m.) injections or infusions, intracranial infusions or as aerosol formulations suitable for intranasal or intrapulmonary delivery (for intrapulmonary delivery see, *e.g.*, EP 257,956).

It is noted that osmotic pressure of injections may be important in subcutaneous and intramuscular injection. Injectable solutions, when hypotonic or hypertonic, may cause pain to a patient upon infusion. Usually, for the therapeutic, injectable formulations herein, it is preferred that the relative osmolarity of the injectable solution be about 300 mosm to about 600 mosm.

The formulations can also be administered in the form of oral or sustained-release preparations. Suitable examples of sustained-release preparations include semipermeable matrices of solid hydrophobic polymers containing the protein, which matrices are in the form of shaped articles, *e.g.*, films, or microcapsules. Examples of sustained-release matrices include cellulose derivatives (e.g., carboxymethylcellulose), sucrose-acetate isobutyrate (SABER^{™}) in non-aqueous media, polyesters, hydrogels (*e.g.*, poly(2-hydroxyethyl-methacrylate) (Langer et al., J. Biomed. Mater. Res. 1981, 15: 167-277; Langer, Chem. Tech. 1982, 12: 98-105 or poly(vinylalcohol)), polylactides (U.S. Patent No. 3,773,919, EP 58,481), copolymers of L-glutamic acid and gamma ethyl-L-glutamate (Sidman et al., Biopolymers 1983, 22: 547-556), non-degradable ethylene-vinyl acetate (Langer *et al.*, *supra*), degradable lactic acid-glycolic acid copolymers such as the Lupron Depot (injectable microspheres composed of lactic acid-glycolic acid copolymer and leuprolide acetate), and poly-D-(-)-3-hydroxybutyric acid (EP 133,988). One optional method of delivery for systemic-acting drugs involves administration by continuous infusion (using, *e.g.*, slow-release devices or minipumps such as osmotic pumps or skin patches), or by injection (using, *e.g.*, intravenous or subcutaneous means, including single-bolus administration).

The composition to be used in the therapy will be formulated and dosed in a fashion consistent with good medical practice, taking into account the clinical condition of the individual patient, the site of delivery of the composition, the method of administration, the scheduling of administration, and other factors known to practitioners.

It is contemplated that yet additional therapies may be employed in the methods. The one or more other therapies may include but are not limited to, administration of radiation therapy, cytokine(s), growth inhibitory agent(s), chemotherapeutic agent(s), cytotoxic agent(s), tyrosine kinase inhibitors, ras farnesyl transferase inhibitors, angiogenesis inhibitors, cyclin-dependent kinase inhibitors, and chromatin remodeling agents such as histone acetylase inhibitors and/or methylation inhibitors which are known in the art and defined further with particularity above, and may be administered in combination (e.g., concurrently or sequentially) with TWEAK antagonist or TWEAK agonist. In addition, therapies based on therapeutic antibodies that target tumor or other cell antigens such as CD20 antibodies (including Rituxan^{™}) or Her receptor antibodies (including Herceptin^{™}) as well as anti-angiogenic antibodies such as anti-VEGF, or antibodies that target other receptors, such as EGFR (such as Tarceva^{™}), or to be used in conjunction with tumor vaccination.

In methods for treating conditions such as cancer, preparation and dosing schedules for chemotherapeutic agents may be used according to manufacturers' instructions or as determined empirically by the skilled practitioner. Preparation and dosing schedules for such chemotherapy are also described in Chemotherapy Service Ed., M.C. Perry, Williams & Wilkins, Baltimore, MD (1992). In some instances, it may be beneficial to expose cells to one or more chemotherapeutic agents prior to administering, e.g., TWEAK antagonist.

It may be desirable to also administer antibodies against other antigens, such as antibodies which bind to CD20, CD11a, CD18, CD40, ErbB2, EGFR, ErbB3, ErbB4, vascular endothelial factor (VEGF), or other TNFR family members (such as OPG, DR4, DR5, TNFR1, TNFR2). Alternatively, or in addition, two or more antibodies binding the same or two or more different antigens disclosed herein may be co-administered to the patient. Sometimes, it may be beneficial to also administer one or more cytokines to the patient.

The antagonist or agonist formulation may be administered in any of the therapeutic methods described in this application in combination with, e.g., concurrently or sequentially, with other agents, cytokines, chemotherapies, antibodies, etc. that are for example, specifically provided in the Definition section of the application above. For example, the TWEAK antagonist formulation may be administered as a pre-treatment (prior to administration of any such other agents), such as a pre-treatment of cancer cells which may otherwise be resistant to the apoptotic effects of other therapeutic agents.

As noted above, antagonists and agonists of the invention have various utilities. For example, TWEAK antagonists may be employed in methods for treating pathological conditions in mammals such as cancer. TWEAK agonists may be employed to treat immune-related diseases in mammals. Diagnosis in mammals of the various pathological conditions described herein can be made by the skilled practitioner. Diagnostic techniques are available in the art which allow, e.g., for the diagnosis or detection of cancer or immune related disease in a mammal. For instance, cancers may be identified through techniques, including but not limited to, palpation, blood analysis, x-ray, NMR and the like. Immune related diseases can also be readily identified. In systemic lupus erythematosus, the central mediator of disease is the production of auto-reactive antibodies to self proteins/tissues and the subsequent generation of immune-mediated inflammation. Multiple organs and systems are affected clinically including kidney, lung, musculoskeletal system, mucocutaneous, eye, central nervous system, cardiovascular system, gastrointestinal tract, bone marrow and blood.

Medical practitioners are familiar with a number diseases in which intervention of the immune and/or inflammatory response have benefit. For example, rheumatoid arthritis (RA) is a chronic systemic autoimmune inflammatory disease that mainly involves the synovial membrane of multiple joints with resultant injury to the articular cartilage. The pathogenesis is T lymphocyte dependent and is associated with the production of rheumatoid factors, auto-antibodies directed against self IgG, with the resultant formation of immune complexes that attain high levels in joint fluid and blood. These complexes in the joint may induce the marked infiltrate of lymphocytes and monocytes into the synovium and subsequent marked synovial changes; the joint space/fluid if infiltrated by similar cells with the addition of numerous neutrophils. Tissues affected are primarily the joints, often in symmetrical pattern. However, extra-articular disease also occurs in two major forms. One form is the development of extra-articular lesions with ongoing progressive joint disease and typical lesions of pulmonary fibrosis, vasculitis, and cutaneous ulcers. The second form of extra-articular disease is the so called Felty's syndrome which occurs late in the RA disease course, sometimes after joint disease has become quiescent, and involves the presence of neutropenia, thrombocytopenia and splenomegaly. This can be accompanied by vasculitis in multiple organs with formations of infarcts, skin ulcers and gangrene. Patients often also develop rheumatoid nodules in the subcutis tissue overlying affected joints; the nodules late stage have necrotic centers surrounded by a mixed inflammatory cell infiltrate. Other manifestations which can occur in RA include: pericarditis, pleuritis, coronary arteritis, interstitial pneumonitis with pulmonary fibrosis, keratoconjunctivitis sicca, and rheumatoid nodules.

Juvenile chronic arthritis is a chronic idiopathic inflammatory disease which begins often at less than 16 years of age. Its phenotype has some similarities to RA; some patients which are rheumatoid factor positive are classified as juvenile rheumatoid arthritis. The disease is subclassified into three major categories: pauciarticular, polyarticular, and systemic. The arthritis can be severe and is typically destructive and leads to joint ankylosis and retarded growth. Other manifestations can include chronic anterior uveitis and systemic amyloidosis.

Spondyloarthropathies are a group of disorders with some common clinical features and the common association with the expression of HLA-B27 gene product. The disorders include: ankylosing spondylitis, Reiter's syndrome (reactive arthritis), arthritis associated with inflammatory bowel disease, spondylitis associated with psoriasis, juvenile onset spondyloarthropathy and undifferentiated spondyloarthropathy. Distinguishing features include sacroileitis with or without spondylitis; inflammatory asymmetric arthritis; association with HLA-B27 (a serologically defined allele of the HLA-B locus of class I MHC); ocular inflammation, and absence of autoantibodies associated with other rheumatoid disease. The cell most implicated as key to induction of the disease is the CD8+ T lymphocyte, a cell which targets antigen presented by class I MHC molecules. CD8+ T cells may react against the class I MHC allele HLA-B27 as if it were a foreign peptide expressed by MHC class I molecules. It has been hypothesized that an epitope of HLA-B27 may mimic a bacterial or other microbial antigenic epitope and thus induce a CD8+ T cells response.

Systemic sclerosis (scleroderma) has an unknown etiology. A hallmark of the disease is induration of the skin; likely this is induced by an active inflammatory process. Scleroderma can be localized or systemic; vascular lesions are common and endothelial cell injury in the microvasculature is an early and important event in the development of systemic sclerosis; the vascular injury may be immune mediated. An immunologic basis is implied by the presence of mononuclear cell infiltrates in the cutaneous lesions and the presence of anti-nuclear antibodies in many patients. ICAM-1 is often upregulated on the cell surface of fibroblasts in skin lesions suggesting that T cell interaction with these cells may have a role in the pathogenesis of the disease. Other organs involved include: the gastrointestinal tract: smooth muscle atrophy and fibrosis resulting in abnormal peristalsis/motility; kidney: concentric subendothelial intimal proliferation affecting small arcuate and interlobular arteries with resultant reduced renal cortical blood flow, results in proteinuria, azotemia and hypertension; skeletal muscle: atrophy, interstitial fibrosis; inflammation; lung: interstitial pneumonitis and interstitial fibrosis; and heart: contraction band necrosis, scarring/fibrosis.

Idiopathic inflammatory myopathies including dermatomyositis, polymyositis and others are disorders of chronic muscle inflammation of unknown etiology resulting in muscle weakness. Muscle injury/inflammation is often symmetric and progressive. Autoantibodies are associated with most forms. These myositis-specific autoantibodies are directed against and inhibit the function of components, proteins and RNA's, involved in protein synthesis.

Sjogren's syndrome is due to immune-mediated inflammation and subsequent functional destruction of the tear glands and salivary glands. The disease can be associated with or accompanied by inflammatory connective tissue diseases. The disease is associated with autoantibody production against Ro and La antigens, both of which are small RNA-protein complexes. Lesions result in keratoconjunctivitis sicca, xerostomia, with other manifestations or associations including bilary cirrhosis, peripheral or sensory neuropathy, and palpable purpura.

Systemic vasculitis are diseases in which the primary lesion is inflammation and subsequent damage to blood vessels which results in ischemia/necrosis/degeneration to tissues supplied by the affected vessels and eventual end-organ dysfunction in some cases. Vasculitides can also occur as a secondary lesion or sequelae to other immune-inflammatory mediated diseases such as rheumatoid arthritis, systemic sclerosis, etc., particularly in diseases also associated with the formation of immune complexes. Diseases in the primary systemic vasculitis group include: systemic necrotizing vasculitis: polyarteritis nodosa, allergic angiitis and granulomatosis, polyangiitis; Wegener's granulomatosis; lymphomatoid granulomatosis; and giant cell arteritis. Miscellaneous vasculitides include: mucocutaneous lymph node syndrome (MLNS or Kawasaki's disease), isolated CNS vasculitis, Behet's disease, thromboangiitis obliterans (Buerger's disease) and cutaneous necrotizing venulitis. The pathogenic mechanism of most of the types of vasculitis listed is believed to be primarily due to the deposition of immunoglobulin complexes in the vessel wall and subsequent induction of an inflammatory response either via ADCC, complement activation, or both.

Sarcoidosis is a condition of unknown etiology which is characterized by the presence of epithelioid granulomas in nearly any tissue in the body; involvement of the lung is most common. The pathogenesis involves the persistence of activated macrophages and lymphoid cells at sites of the disease with subsequent chronic sequelae resultant from the release of locally and systemically active products released by these cell types.

Autoimmune hemolytic anemia including autoimmune hemolytic anemia, immune pancytopenia, and paroxysmal noctural hemoglobinuria is a result of production of antibodies that react with antigens expressed on the surface of red blood cells (and in some cases other blood cells including platelets as well) and is a reflection of the removal of those antibody coated cells via complement mediated lysis and/or ADCC/Fc-receptor-mediated mechanisms.

In autoimmune thrombocytopenia including thrombocytopenic purpura, and immune-mediated thrombocytopenia in other clinical settings, platelet destruction/removal occurs as a result of either antibody or complement attaching to platelets and subsequent removal by complement lysis, ADCC or FC-receptor mediated mechanisms.

Thyroiditis including Grave's disease, Hashimoto's thyroiditis, juvenile lymphocytic thyroiditis, and atrophic thyroiditis, are the result of an autoimmune response against thyroid antigens with production of antibodies that react with proteins present in and often specific for the thyroid gland. Experimental models exist including spontaneous models: rats (BUF and BB rats) and chickens (obese chicken strain); inducible models: immunization of animals with either thyroglobulin, thyroid microsomal antigen (thyroid peroxidase).

Type I diabetes mellitus or insulin-dependent diabetes is the autoimmune destruction of pancreatic islet β cells; this destruction is mediated by auto-antibodies and auto-reactive T cells. Antibodies to insulin or the insulin receptor can also produce the phenotype of insulin-non-responsiveness.

Immune mediated renal diseases, including glomerulonephritis and tubulointerstitial nephritis, are the result of antibody or T lymphocyte mediated injury to renal tissue either directly as a result of the production of autoreactive antibodies or T cells against renal antigens or indirectly as a result of the deposition of antibodies and/or immune complexes in the kidney that are reactive against other, non-renal antigens. Thus other immune-mediated diseases that result in the formation of immune-complexes can also induce immune mediated renal disease as an indirect sequelae. Both direct and indirect immune mechanisms result in inflammatory response that produces/induces lesion development in renal tissues with resultant organ function impairment and in some cases progression to renal failure. Both humoral and cellular immune mechanisms can be involved in the pathogenesis of lesions.

Demyelinating diseases of the central and peripheral nervous systems, including Multiple Sclerosis; idiopathic demyelinating polyneuropathy or Guillain-Barr syndrome; and Chronic Inflammatory Demyelinating Polyneuropathy, are believed to have an autoimmune basis and result in nerve demyelination as a result of damage caused to oligodendrocytes or to myelin directly. In MS there is evidence to suggest that disease induction and progression is dependent on T lymphocytes. Multiple Sclerosis is a demyelinating disease that is T lymphocyte-dependent and has either a relapsing-remitting course or a chronic progressive course. The etiology is unknown; however, viral infections, genetic predisposition, environment, and autoimmunity all contribute. Lesions contain infiltrates of predominantly T lymphocyte mediated, microglial cells and infiltrating macrophages; CD4+T lymphocytes are the predominant cell type at lesions. The mechanism of oligodendrocyte cell death and subsequent demyelination is not known but is likely T lymphocyte driven.

Inflammatory and Fibrotic Lung Disease, including Eosinophilic Pneumonias; Idiopathic Pulmonary Fibrosis, and Hypersensitivity Pneumonitis may involve a disregulated immune-inflammatory response. Inhibition of that response would be of therapeutic benefit.

Autoimmune or Immune-mediated Skin Disease including Bullous Skin Diseases, Erythema Multiforme, and Contact Dermatitis are mediated by auto-antibodies, the genesis of which is T lymphocyte-dependent.

Psoriasis is a T lymphocyte-mediated inflammatory disease. Lesions contain infiltrates of T lymphocytes, macrophages and antigen processing cells, and some neutrophils.

Allergic diseases, including asthma; allergic rhinitis; atopic dermatitis; food hypersensitivity; and urticaria are T lymphocyte dependent. These diseases are predominantly mediated by T lymphocyte induced inflammation, IgE mediated-inflammation or a combination of both.

Transplantation associated diseases, including Graft rejection and Graft-Versus-Host-Disease (GVHD) are T lymphocyte-dependent; inhibition of T lymphocyte function is ameliorative.

Other diseases in which intervention of the immune and/or inflammatory response have benefit are Infectious disease including but not limited to viral infection (including but not limited to AIDS, hepatitis A, B, C, D, E) bacterial infection, fungal infections, and protozoal and parasitic infections (molecules (or derivatives/agonists) which stimulate the MLR can be utilized therapeutically to enhance the immune response to infectious agents), diseases of immunodeficiency (molecules/derivatives/agonists) which stimulate the MLR can be utilized therapeutically to enhance the immune response for conditions of inherited, acquired, infectious induced (as in HIV infection), or iatrogenic (i.e. as from chemotherapy) immunodeficiency), and neoplasia.

The invention also provides kits which include antagonists or agonists described herein. A typical kit will comprise a container, preferably a vial, for antagonist or agonist in one or more excipients as described above; and instructions, such as a product insert or label, directing the user as to how to employ the antagonist or agonist formulation. This would preferably provide a pharmaceutical formulation. Preferably, the pharmaceutical formulation is for treating cancer or an immune related condition. Suitable containers include, for example, bottles, vials, syringes, and test tubes. The containers may be formed from a variety of materials such as glass or plastic. The container holds an antagonist or agonist formulation that is effective for diagnosing or treating the disorder and may have a sterile access port (for example, the container may be an intravenous solution bag or a vial having a stopper pierceable by a hypodermic injection needle). The label on, or associated with, the container indicates that the formulation is used for diagnosing or treating the disorder of choice. The article of manufacture may further comprise a second container comprising water-for-injection, a pharmaceutically-acceptable solution, saline, Ringer's solution, or dextrose solution. It may further include other materials desirable from a commercial and user standpoint, including other buffers, diluents, filters, needles, syringes, and package inserts with instructions for use.

All patents, patent applications, publications, product descriptions, and protocols are cited throughout this application. The section headings used herein are for organizational purposes only and are not to be construed as limiting the subject matter described.

### EXAMPLES

Various aspects of the invention are further described and illustrated by way of the examples that follow, none of which are intended to limit the scope of the invention.

Commercially available reagents referred to in the examples were used according to manufacturer's instructions unless otherwise indicated. The source of those cells identified in the following examples, and throughout the specification, by ATCC accession numbers is the American Type Culture Collection, Manassas, VA. Unless otherwise noted, the present invention uses standard procedures of recombinant DNA technology, such as those described hereinabove and in the following textbooks: Sambrook et al., Molecular Cloning: A Laboratory Manual, Cold Spring Harbor Press N.Y., 1989; Ausubel et al., Current Protocols in Molecular Biology, Green Publishing Associates and Wiley Interscience, N.Y., 1989; Innis et al., PCR Protocols: A Guide to Methods and Applications, Academic Press, Inc., N.Y., 1990; Harlow et al., Antibodies: A Laboratory Manual, Cold Spring Harbor Press, Cold Spring Harbor, 1988; Gait, M.J., Oligonucleotide Synthesis, IRL Press, Oxford, 1984; R.I. Freshney, Animal Cell Culture, 1987; Coligan et al., Current Protocols in Immunology, 1991.

### MATERIALS AND TECHNIQUES:

**Expression analysis of TWEAK and Fn14 in human PBMCs.** Human peripheral blood mononuclear cells (PBMCs) were isolated from 50 ml human donor whole blood with Lymphocyte Separation Medium (ICN) according to the manufacturer's instructions. Cells were resuspended in complete Iscoves's medium in the presence of Brefeldin A (5 ug/mL) for 24 hours in the presence and absence of inflammatory stimuli. Following stimulation, Fc receptors were blocked with 2 ug/mL Fc Block (Miltenyi Biotec, Auburn, CA) for 20 minutes at room temperature. Cells were then surface stained with fluorescence-conjugated monoclonal antibodies to CD3, CD4, CD8, CD11b, CD11c, CD14, CD20, CD45, CD56, HLA-DR, Lin1 FITC(BD Biosciences, San Jose, CA) and FN14 (e-Biosciences) for 30 minutes at room temperature and then treated with BD FACS Lyse solution according to the manufacturer's instructions and stored at -70° C overnight.

Cells were permeabilized and then stained for TWEAK (e-Biosciences) for 30 minutes at room temperature. Following washing, cells were analyzed on FACS Calibur (BD Biosciences).

**Generation of TWEAK-deficient mice**. A *TWEAK* targeting vector was constructed based on the *TNLOX1-3* vector (Gerber et al., Development, 126:1149-1159(1999)) by replacing 2.5 kb of the *TWEAK* gene, encompassing the first and all five downstream exons, with a *PGK-neo* cassette. The construct contained two DNA stretches derived from the mouse genome: a 3.1-kb fragment encompassing the sixth and the seventh exons of *TWEAK* and part of exon one of *TWEAK,* placed 5' of the neo cassette, and a 4.1-kb fragment encompassing the first and second *SMT3IP1* exons placed 3' of the *PGK-neo* cassette.

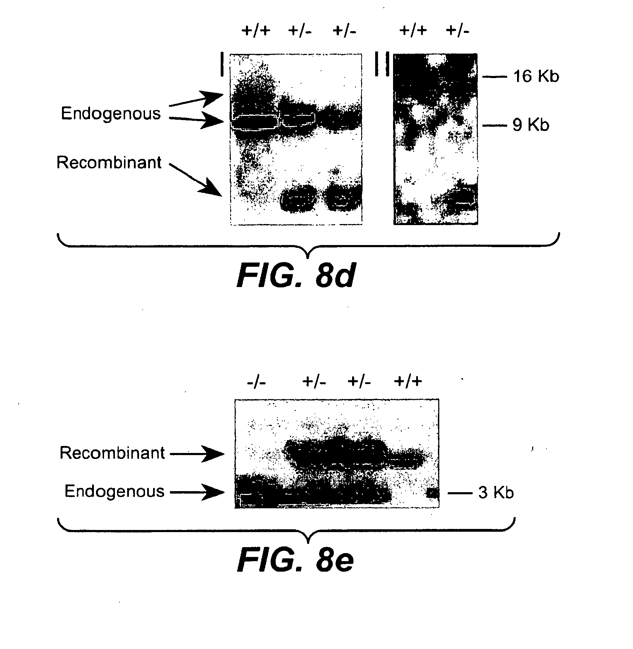

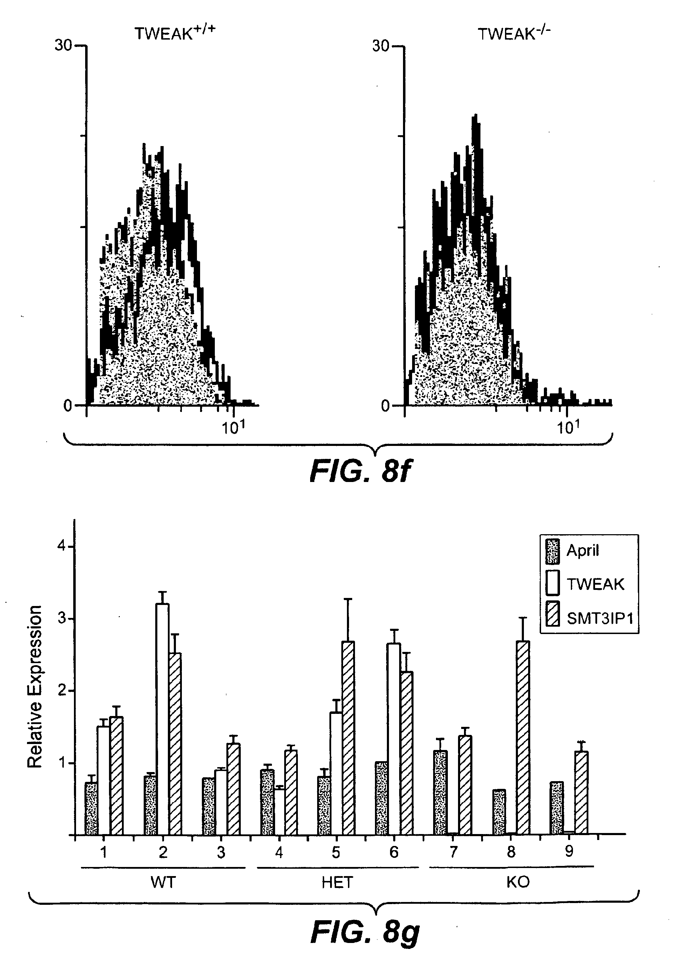

R1 embryonic stem cells (Nagy et al., Gene Targeting:A Practical Approach, A.L. Joyner, ed., Oxford University Press, Oxford, England, pp. 147-179 (1993)) were transfected with the linearized vector by electroporation, and G418-resistant clones were screened for the presence of the expected recombination event by Southern blot analysis with 5'- and 3'-specific DNA probes (as shown in Fig. 8). Two independent *TWEAK* -/cell lines were microinjected into C57BL/6 blastocytes. Germ line transmission in mice generated by crossing chimeric males with C57BL/6 females was detected by coat color and confirmed by two-step genomic PCR (Fig. 8) with the following external (E) and internal (I) primer sets: E forward, TGCCCTAAGCCAGTCTACACCCAGTATTCCTTC (SEQ ID NO:3); E reverse, TGGCCTGAAAGAAATGCTCACACTATCACCAAC (SEQ ID NO:4); I forward, CTTAGAACCAGCCGTAGGAAGGATT (SEQ ID NO:5); and I reverse, GTGCCAGGGCGTCCAGTACATACAA (SEQ ID NO:6).

*TWEAK* knockout animals were backcrossed a minimum of six times onto the C57BL/6 background.

### Examination of APRIL, TWEAK, and SMT3IP1 mRNA expression.

Analysis of several tissues by quantitative RT-PCR demonstrated that TWEAK -/- mice did not express TWEAK transcripts, while mRNA expression of two nearby genes, APRIL and SMT31P1, was unaltered in the knockouts. (Varfolomeev et al., Mol. Cell. Biol., 24:997-1006 (2004)); Fig. 9.

**Flow cytometry analyses**. Single-cell suspensions from hematopoietic organs were obtained from eight week old mice by dissociation of the isolated tissues with wire mesh screens and rubber stoppers from syringes. Single-cell suspensions were incubated with Fc blocking antibodies (2 ug/mL, BD Biosciences) and subsequently stained with lineage-specific conjugated monoclonal antibodies to B220, CD3, CD4, CD8, CD11b, CD11c, CD19, CD45, DX5, Lin1 FITC (BD Biosciences, San Jose, CA), CD161, and F4/80 (e-Biosciences) for 30 minutes at room temperature. Following surface staining, RBCs were lysed with ACK Lysis Buffer (Biosource International) according to the manufacturer's instructions and the remaining cells were fixed. TRUCount Beads (BD Biosciences) were added to the tubes for quantitation. Cell-associated fluorescence was analyzed with a FACS Calibur instrument and associated Cell Quest software (BD Biosciences).

**NK cell AICD Assays.** Human PBMCs were isolated from 100 mL human donor whole blood and stimulated for 24 hours with TNF-α (500 ng/mL), LPS (5 µg/L), or IFN-gamma (500 ng/mL) in the absence or presence of anti-TWEAK mAb (CARL-1, e-Biosciences) or FN14-Fc (fusion protein comprising amino acids 1-129 of Figure 12) (Genentech). Following stimulation, NK cells were isolated using Miltenyi CD56+ beads and stained for sub-G1 DNA content as described in Maecker et al., Cancer Cell, 2:139-148(2002).

**LPS experiments.** Ten *TWEAK*^{-/-} and *TWEAK*^{+/+} mice per group were injected intraperitoneally (i.p.) with LPS (*Escherichia coli* 055:B5; Sigma). LPS doses ranging from 100 mg/kg to 10 mg/kg were dissolved in sterile saline. Mice were monitored for viability every hour over a period of 5 days. Murine cytokine analysis was conducted by injecting ten mice per group i.p. with 30 mg/kg LPS and isolating blood and spleens 24 hours later. Single cell suspensions were incubated for 6 hours in the presence of Brefeldin A (5 µg/mL). Cells were Fc blocked (2 µg/mL, BD Biosciences) for the last 20 minutes of this incubation and subsequently stained with lineage-specific conjugated monoclonal antibodies, DX5 (to identify NK cells), CD11b and F4/80 (to identify macrophages) as well as CD45 (common leukocyte antigen) for 30 minutes at room temperature. Following surface staining, RBCs were lysed as described above. Cells were permeabilized and then stained with antibody to IFN-gamma, IL-12, or IL-10 and analyzed on a FACS Calibur (BD Biosciences). Human cytokine analysis was conducted by isolating PBMCs from four separate human donors. Donor PBMCs were incubated *in* vitro in the presence or absence of 1 µg/mL LPS for 16 hours. During the last 6 hours of stimulation, Brefeldin A was added to the cells at a final concentration of 5 µg/mL. Human PBMCs were Fc blocked (Miltneyi) for 20 minutes at room temperature and then surface stained (CD3, CD56, CD14, CD45; BD Biosciences) for 30 minutes at room temperature. Following surface staining, the RBCs were lysed according to manufacturer's instructions for intracellular staining. Cells were fixed and permeabilized, stained with IFN-gamma or IL-12 antibody, and analyzed on a FACS Calibur.

**STAT-1 Activity Assays**. NK cells and macrophages were isolated from a human donor's spleen using Miltenyi CD56+ and CD11b+ beads, respectively. 1.0 x 10⁶ NK cells/0.5mL were co-incubated with 1.0 x 10⁶ macrophages/0.5 mL Macrophage-SFM Medium (Invitrogen). Cells were rested in serum-free medium for 12 hours and then stimulated with 1 µg/mL LPS. Twelve hours later, cells were surface stained for CD56 and CD11b followed by intracellular staining for phospho-STAT-1 as outlined by Perez and Nolan (Krutzik et al., Clin. Immunol., 110:206-221 (2004); Perez et al., Meth. Mol. Biol., 263:67-94 (2004); Perez and Nolan, Nat. Biotechnol., 20:155-162 (2002)).

**NF-κB Analysis**. NK cells and macrophages were isolated from a donor's human spleen using Miltenyi CD56⁺ and CD11b⁺ beads, respectively. 5.0 x 10⁶ NK cells were co-incubated with 5.0 x 10⁶ macrophages in 5 mL Macrphage-SFM Medium per timepoint. Cells were rested for 12 hours, prior to stimulation with TWEAK (100 ng/mL) or TNF-alpha (100 ng/mL). Lysates (20 µg total protein) and immunoprecipitates (50 µg total protein) were prepared according to manufacturers instructions (Cell Signaling, Beverly, MA). All antibodies for subsequent immunoblots and immunoprecipitations were purchased from Cell Signaling and experiments were conducted according to their protocols.

**Histology and immunohistochemistry.** Tissues of 3, 6, and 12 month old male *TWEAK -*/*-* and *TWEAK +*/*+* mice were weighed, fixed, sectioned, and analyzed for pathological status. Hematoxylin and eosin-stained sections were analyzed for gross histological abnormalities. Peanut agglutinin (Vector Research, Burlingame, CA)-stained frozen sections were analyzed for structure of germinal centers. Five *TWEAK -*/*-* and *TWEAK +*/*+* spleens from 12 month old old male mice were dissociated, stained, and quantitated for lymphocyte cellularity utilizing TruCount beads (BD Biosciences) according to the manufacturer's instruction.

**B16 melanoma experiments**. Ten *TWEAK -*/*-* and *TWEAK +*/*+* mice were injected with either 0.1-0.5 X 10⁶ cells/0.1 mL sterile saline subcutaneously (s.c.) in the right hind flank: Mice were monitored daily and tumor measurements taken every other day for 4 weeks (B16.BL6 study) or 6 weeks (B16.F10 study). At study termination, tumors were removed, weighed and dissociated first through wire mesh screens followed by treatment with non-enzymatic cell dissociation buffer (Sigma) for 5 minutes to create single cell suspensions. Splenocytes generated from tumor-injected mice and were co-incubated with either sterile saline or tumor cell suspensions in the presence of Brefeldin A for 12 hours to measure intracellular cytokine production.

### EXPERIMENTAL RESULTS:

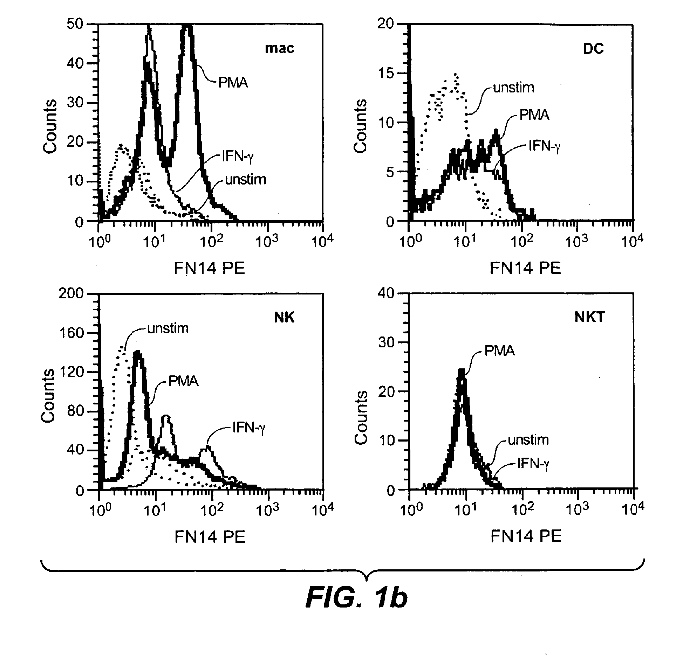

TWEAK expression in various hematopoietic tissues was previously reported (Chicheportiche et al., supra; Marsters et al., supra), but the only lymphoid cells previously reported to express TWEAK are monocytes (Nakayama et al., J. Exp. Med., 192:1373-1380 (2000)). In order to further elucidate immunological targets of TWEAK, numerous lymphoid populations were analyzed for expression of TWEAK and its receptor, FN14, following various inflammatory stimuli (Figs. 1A and 1B).

TWEAK and its receptor, FN14, were shown to be expressed by cells of the innate immune system (see Fig. 1). Only NK cells, macrophages, and dendritic cells were found to express TWEAK (Fig. 1A) and its receptor, FN14 (Fig. 1B). Further, surface expression of both receptor and ligand was upregulated following stimulation with IFN-gamma or PMA. NKT cells expressed TWEAK but not FN14, and neither was upregulated by IFN-gamma or PMA. Other lymphoid cell types, including T and B cells did not express significant levels of TWEAK or FN14 (data not shown.)

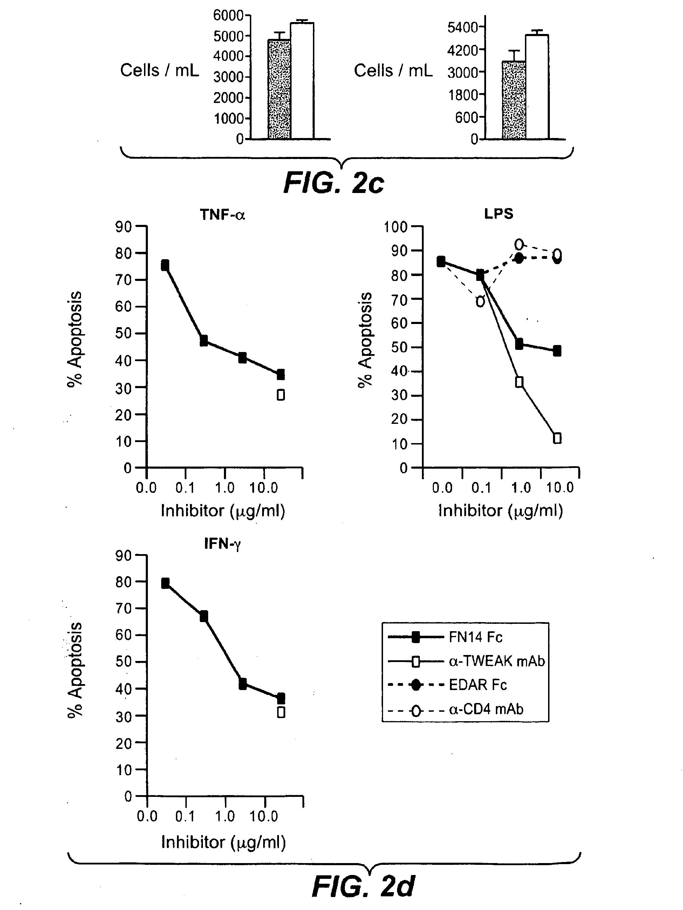

To examine the biological role of TWEAK *in vivo,* TWEAK gene knockout mice were generated (Fig. 8). Detailed anatomical and histological analysis did not suggest any significant abnormality in the non-lymphoid tissues of TWEAK^{-/-} mice. (Fig. 10) However, analysis of hematopoietic tissues revealed that TWEAK^{-/-} mice had significantly more NK cells as compared to wild type, age-matched littermates (Fig. 2A). This increase was apparent in secondary lymphoid organs, including spleen, Peyer's patches, lymph nodes, and peripheral blood (Fig. 2A) and was greater in males (Fig. 2A, top) than females (Fig. 2A, bottom). In contrast to their elevated NK numbers, TWEAK^{-/-} mice displayed normal levels of NKT cells (Fig. 2B), as well as CD4⁺ or CD8⁺ T cells, B cells, macrophages, dendritic cells, granulocytes, and platelets (data not shown). The amount of NK cells in the bone marrow of TWEAK^{-/-} and wild type mice was similar (Fig. 2C), suggesting that the elevation in NK counts may not be caused by changes in NK cell development (Kim et al., Nat. Immunol., 3:523-528 (2002)). Alternatively, the impaired elimination of NK cells by activation-induced cell death (AICD) may lead to NK accumulation in TWEAK's absence. NK cells from human peripheral blood were isolated, and the effect of TWEAK neutralization on their sensitivity to AICD was examined (Fig. 2D). TWEAK inhibition by a soluble FN14-Fc decoy receptor or a TWEAK-neutralizing antibody markedly protected the NK cells from stimulation of AICD by TNF-alpha, LPS, or IFN-gamma, suggesting that NK cells may accumulate in TWEAK^{-/-} mice because of insufficient NK deletion through AICD.

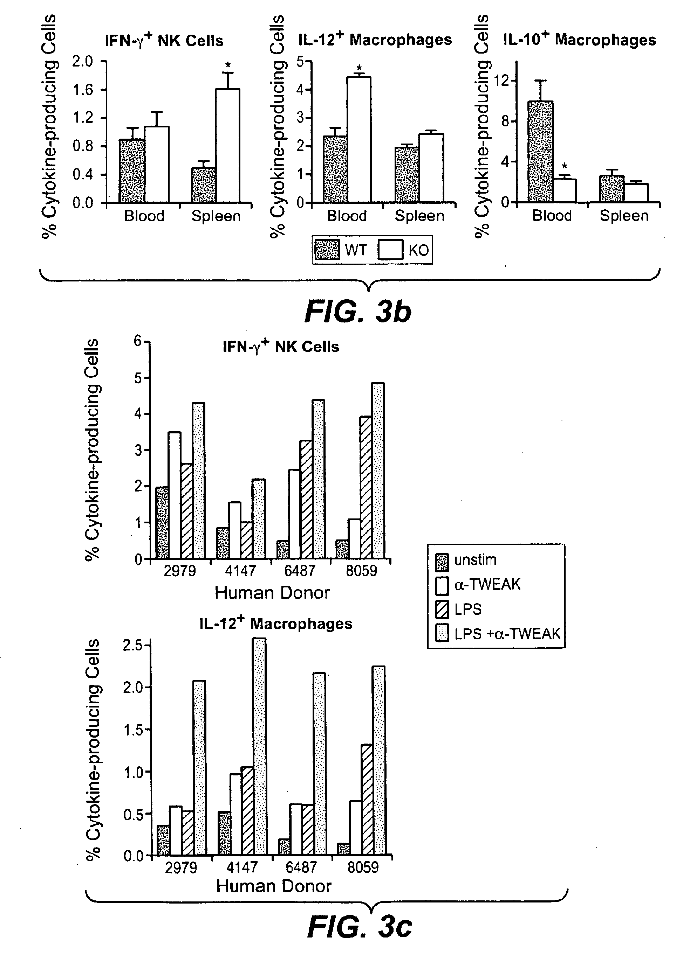

To determine the importance of TWEAK for innate immune responses *in vivo,* an established model of systemic challenge was examined with lethal doses of the gram-negative bacterial endotoxin lipopolysaccharide (LPS) (Fig. 3A). TWEAK^{-/-} mice were more susceptible to LPS-induced death than wild type controls over a wide range of LPS doses, suggesting that a stronger innate inflammatory response develops in the absence of TWEAK. TWEAK^{-/-} NK cells and macrophages, isolated from peripheral blood and spleens of LPS-injected mice, produced more INF-gamma and IL-12 and less IL-10 as compared to wild type cells (Fig. 3B). Similarly, antibody neutralization of TWEAK augmented the production of IFN-gamma and IL-12 by human peripheral blood NK cells and macrophages following LPS stimulation (Fig. 3C). Thus, it is believed that TWEAK^{-/-} mice are hypersensitive to LPS not only because they have elevated NK cell numbers but also because their NK cells and macrophages produce more IFN-gamma and IL-12, which further promote the inflammatory response (D'Andrea et al., J. Exp. Med., 178:1041-1048 (1993); Emoto et al., J. Immunol., 169:1426-1432 (2002); Heremans et al., Eur. J. Immunol., 24:1155-1160 (1994)). These results suggest that TWEAK functions to attenuate the innate inflammatory response.

To investigate how TWEAK's absence might promote the production of IFN-gamma and IL-12 by innate immune cells, the activity of the signal transducer and activator of transcription (STAT-1), which is key to inducing the expression of IFN-gamma in NK cells and of IL-12 in macrophages in response to pathogens, was examined (Marodi et al., Clin. Exp. Immunol., 126:456-460 (2001); Morrison et al., J. Immunol.,172:1825-1832 (2004); Nelson et al., J. Immunol., 156:3711-3720 (1996); Varma et al., Clin. Diag. Lab Immunol., 9:530-543 (2002)). TWEAK neutralization increased basal STAT-1 phosphorylation in NK cells and macrophages and further enhanced the stimulation of STAT-1 by LPS in these cells (Fig. 4A). Thus, one mechanism contributing to TWEAK's repression of IFN-gamma and IL-12 production may be attenuation of STAT-1 activation.

TNF-alpha, a cytokine that plays a crucial role in augmenting the innate inflammatory response, induces the expression of IFN-gamma and IL-12 (as well as of other immunomodulatory genes) through activation of the canonical NF-κB1 pathway (Bonizzi and Karin, Trends Immunol., 25:280-288 (2004); Chen and Greene, Nat. Rev. Mol. Cell Biol., 5:392-401 (2004); Chen et al., J. Immunol., 166:270-276 (2001); D'Andrea et al., J. Exp. Med., 178:1041-1048 (1993); Zhong et al., Mol. Cell, 9:625-636 (2002)). TNF-alpha induces transient phosphorylation of the p65/RelA NF-κB1 subunit, leading to its association with the p50 subunit and to nuclear translocation of the resulting heteromeric complex. In the nucleus, the p65/p50 heterodimer transactivates downstream target genes, such as the IFN-gamma and IL-12, through association with the p300/CBP transcriptional co-activator (Chen and Greene, supra; Chen et al., J. Immunol., 166:270-276 (2001); Chen et al., Immunology, 107:199-208 (2002); Kiernan et al., J. Biol. Chem., 278:2758-2766 (2003); Zhong et al., supra). Alternatively, NF-κB1 may interact with histone deacetylase (HDAC)-1,-2, or -3, which cause transcriptional repression of target genes (Ashburner et al., Mol. Cell Biol., 21:7065-7077 (2001); Kiernan et al., J. Biol. Chem., 278:2758-2766 (2003); Quivy and Van Lint, Biochem. Pharmacol., 68:2507-2515 (2004); Rahman et al., Biochem. Pharmacol., 68:1255-1267 (2004); Zhong et al., supra). Whereas TNF-alpha selectively activates the canonical NF-κB1 pathway, TWEAK appears capable of promoting nuclear translocation of both canonical NF-κB1 (Chicheportiche et al., supra; Marsters et al., supra; Saitoh et al., supra) and non-canonical NF-κB2 subunits (Saitoh et al., supra).

To examine whether TWEAK also might affect gene expression by modulating the transcriptional interactions of NF-κB1, the effects of TWEAK and TNF-alpha on phosphorylation of p65 NF-κB1 in human splenic NK cells and macrophages were compared (Fig. 4B). Unlike TNF-alpha, which caused transient p65 modification detectable at 0.5 hours, TWEAK induced prolonged p65 phosphorylation, starting at 0.25 hours and lasting up to 8 hours. Next, p65 NF-κB1 from stimulated cells was immunoprecipitated and probed for association with p300 or HDAC-1 by immunoblot analysis (Fig. 4C). Whereas TNF-alpha induced strong interaction of p65 with p300 but not with HDAC-1, TWEAK induced robust association of p65 with HDAC-1 but not with p300. Thus, in addition to inhibiting STAT-1 activation, TWEAK may repress the transcription of IFN-gamma and IL-12 by promoting interaction of NF-κB1 with HDAC-1. The inhibitory effect of TWEAK on IFN-gamma production by NK cells and IL-12 production by macrophages was reversed by the HDAC inhibitor Trichostatin A (data not shown).

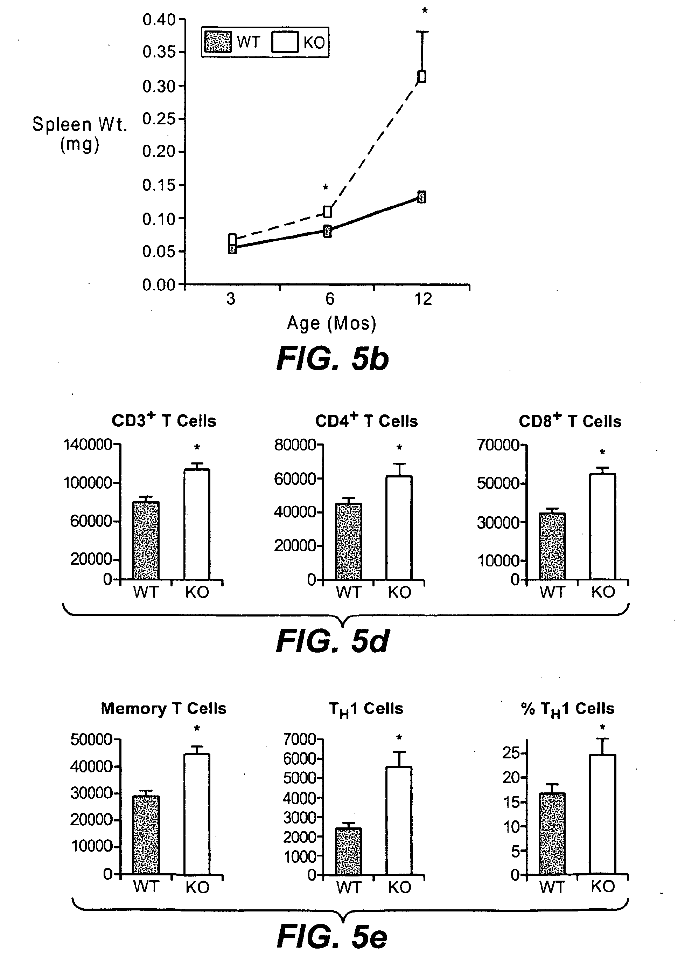

To investigate whether TWEAK deficiency alters immune system development, the lymphoid tissues of TWEAK^{-/-} mice and wild type littermates at 3, 6, and 12 months of age were compared (Fig. 5). By 6 months, TWEAK^{-/-}mice showed notable spleen and lymph node enlargement as compared to controls (Fig. 5A , 5B), while the thymus and liver did not differ (data not shown). Histological evaluation indicated that the TWEAK^{-/-} spleens had normal germinal center formation and were free of malignancy, as were the lymph nodes (Fig. 5C). However, immunohistochemical staining of the spleens showed a stronger signal with anti-CD3 antibody in the 12-month old TWEAK^{-/-} mice as compared to age-matched littermates (Fig. 5C), suggesting an expansion of the T cell compartment. FACS analysis confirmed that both CD4⁺ and CD8⁺ T cells were significantly more abundant in aged TWEAK^{-/-} mice (Fig. 5D). Splenic NK cell numbers also were increased, while the amount of B cells, macrophages, granulocytes, or platelets was similar (data not shown). Given that NK cells comprise only a small percentage of spleen cells, it is likely that the increased spleen size was caused primarily by an expansion of the T cell compartment in TWEAK's absence. Further analysis demonstrated a marked increase in memory T cells and in T cells positive for expression of the T_{H}1-specific transcription factor T-bet in the TWEAK^{-/-}mice (Fig. 5E). These results suggest that TWEAK functions to inhibit the development of an adaptive T_{H}1 immune profile.

To assess further the involvement of TWEAK in modulating the transition to adaptive immunity, an established model of anti-tumor immunity, based on syngeneic mouse C57 Black 6 B16 melanoma cells was examined (Yang et al., Int. J. Cancer, 105:512-519 (2003); Yang et al., Cell. Immunology, 179:84-95 (1997); Yei et al., Gene Ther., 9:1302-1311 (2002)). In this model, both NK cells and effector T cells are important for tumor rejection (Prevost-Blondel et al., Eur. J. Immunol., 30:2507-2515 (2000); Turk et al., J. Exp. Med., 200:771-782 (2004); Yang et al., Int. J. Cancer, 105:512-519 (2003); Yang et al., Cell. Immunol., 179:84-95 (1997); Yei et al., Gene Ther., 9:1302-1311 (2002)). First, mice were challenged with the moderately aggressive B16.F10 sub-clone of the B16 cell line (Fig. 6). TWEAK^{-/-} mice completely resisted the establishment and growth of B16.F10 tumors, while the wild type animals succumbed to tumor growth at a rate comparable to previously reported data (Fig. 6A and 6B) (Yei et al., supra). To define which immunological differences might have caused this marked disparity in tumor rejection, the splenic lymphocyte populations of the B16.F10-injected mice were analyzed (Fig. 6C). Consistent with the other findings, TWEAK-deficient animals had more splenic NK cells than the wild type controls. Surprisingly, despite their lack of detectable tumors and hence absence of abundant tumor-associated antigens, the TWEAK^{-/-} mice displayed a significant expansion of CD8⁺ T cells relative to controls. Taking this finding together with the observation of increased memory T cell numbers in aged TWEAK^{-/-} mice, it is believed the absence of TWEAK may facilitate an enhanced tumor-induced memory response, possibly through stronger T cell priming facilitated by presence of higher levels of IFN-gamma and IL-12.

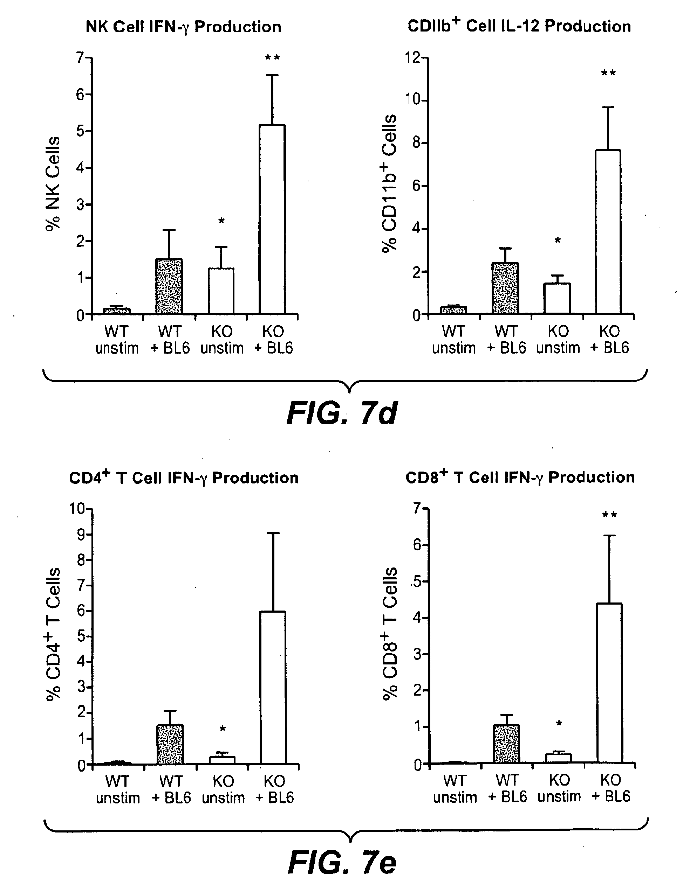

Mice were also challenged with a more aggressive B16 melanoma sub-clone, B16.BL6; this ensured tumor implantation, although tumor growth was significantly attenuated in TWEAK^{-/-} mice compared to wild type controls, as indicated by mean tumor weights at 1 month (Fig. 7A). Tumors isolated from TWEAK^{-/-} mice exhibited greatly increased lymphocytic infiltration, with 2-8 fold more T and NK cells than controls (Fig. 9). Tumor-bearing TWEAK^{-/-} mice also had larger spleens than controls (Fig. 7B), with expanded NK and T cell populations (Fig. 7C). To verify whether the expanded lymphocytic populations harbored specific anti-tumor activity, splenocytes from tumor-bearing mice were isolated, re-challenged *ex vivo* with B16.BL6 tumor cells, and their capacity to produce specific cytokines was determined. TWEAK-deficient CD8⁺ T cells and NK cells produced significantly more IFN-gamma while TWEAK^{-/-} macrophages generated more IL-12 upon tumor re-challenge than did corresponding wild type controls (Fig. 7D, 7E). Together, these studies demonstrate that TWEAK's absence augments innate as well as adaptive anti-tumor immunity, suggesting that TWEAK acts physiologically to repress both responses. Further, the evidence of T cell expansion and enhanced anti-tumor cytokine production in TWEAK^{-/-} mice suggests that TWEAK modulates the innate-to-adaptive immune interface.

## Claims

1. An antagonist molecule for use in a method of treating cancer in mammal,
wherein the antagonist is an anti-TWEAK antibody, an anti-TWEAK receptor antibody and/or a TWEAK receptor immunoadhesin, and
wherein the antagonist molecule is capable of blocking or interrupting intracellular signalling of TWEAK receptor, thereby enhancing innate T_{H}1 responses or activity in a mammal and treating the cancer,
wherein TWEAK receptor is FN14 receptor.

2. Use of an antagonist molecule for the preparation of a medicament for the treatment of cancer in a mammal,
wherein the antagonist is an anti-TWEAK antibody, an anti-TWEAK receptor antibody and/or a TWEAK receptor immunoadhesin, and
wherein the antagonist molecule is capable of blocking or interrupting intracellular signalling of TWEAK receptor, thereby enhancing innate T_{H}1 responses or activity in a mammal and treating the cancer,
wherein TWEAK receptor is FN14 receptor.

3. The antagonist for use in a method of treatment or the use of claim 1 or claim 2, wherein said mammal is also exposed to chemotherapy, radiation, prodrug, cytotoxic agent, or growth inhibitory agent.

4. The antagonist for use in a method of treatment or the use of any one of the preceding claims, wherein said TWEAK receptor immunoadhesin comprises a TWEAK receptor sequence fused to a Fc region of an immunoglobulin.

5. The antagonist for use in a method of treatment or the use of claim 4, wherein said TWEAK receptor sequence comprises an extracellular domain sequence of the FN14 receptor.

6. The antagonist for use in a method of treatment or the use of any one of the preceding claims, wherein said anti-TWEAK antibody binds the human TWEAK polypeptide comprising amino acids 94-249 of Figure 11 (SEQ ID NO:1).

7. The antagonist for use in a method of treatment or the use of claim 6, wherein said anti-TWEAK antibody is a chimeric, humanized or human antibody.

8. The antagonist for use in a method of treatment or the use of any one of the preceding claims, wherein said anti-TWEAK receptor antibody binds the human FN14 receptor polypeptide comprising the amino acid sequence of Figure 12 (SEQ ID NO:2).

9. The antagonist for use in a method of treatment or the use of claim 8, wherein said anti-TWEAK receptor antibody is a chimeric, humanized or human antibody.

## Patentansprüche

1. Antagonistenmolekül zur Verwendung in einem Verfahren zur Behandlung von Krebs in einem Säugetier,
wobei der Antagonist ein Anti-TWEAK-Antikörper, ein Anti-TWEAK-Rezeptor-Antikörper und/oder ein TWEAK-Rezeptor-Immunadhäsin ist und
wobei das Antagonistenmolekül in der Lage ist, intrazelluläre Signalübertragung eines TWEAK-Rezeptors zu blockieren oder zu unterbrechen, wodurch angeborene T_{H}1-Reaktionen oder -Aktivität in einem Säugetier gefördert und der Krebs behandelt wird,
wobei der TWEAK-Rezeptor ein FN14-Rezeptor ist.

2. Verwendung eines Antagonistenmoleküls zur Herstellung eines Medikaments zur Behandlung von Krebs in einem Säugetier,
wobei der Antagonist ein Anti-TWEAK-Antikörper, ein Anti-TWEAK-Rezeptor-Antikörper und/oder ein TWEAK-Rezeptor-Immunadhäsin ist und
wobei das Antagonistenmolekül in der Lage ist, intrazelluläre Signalübertragung eines TWEAK-Rezeptors zu blockieren oder zu unterbrechen, wodurch angeborene T_{H}1-Reaktionen oder -Aktivität in einem Säugetier gefördert und der Krebs behandelt wird,
wobei der TWEAK-Rezeptor ein FN14-Rezeptor ist.

3. Antagonist zur Verwendung in einem Behandlungsverfahren oder Verwendung nach Anspruch 1 oder 2, wobei das Säugetier auch einer Chemotherapie, einer Bestrahlung, einem Prodrug, einem Zytotoxikum oder einem wachstumshemmenden Mittel ausgesetzt wird.

4. Antagonist zur Verwendung in einem Behandlungsverfahren oder Verwendung nach einem der vorangegangenen Ansprüche, wobei das TWEAK-Rezeptor-Immunadhäsin eine TWEAK-Rezeptor-Sequenz an eine Fc-Region eines Immunglobulins fusioniert umfasst.

5. Antagonist zur Verwendung in einem Behandlungsverfahren oder Verwendung nach Anspruch 4, wobei die TWEAK-Rezeptor-Sequenz eine extrazelluläre Domänensequenz des FN14-Rezeptors umfasst.

6. Antagonist zur Verwendung in einem Behandlungsverfahren oder Verwendung nach einem der vorangegangenen Ansprüche, wobei der Anti-TWEAK-Antikörper an das menschliche TWEAK-Polypeptid bindet, das die Aminosäuren 94-249 aus Fig. 11 (Seq.-ID Nr. 1) umfasst.

7. Antagonist zur Verwendung in einem Behandlungsverfahren oder Verwendung nach Anspruch 6, wobei der Anti-TWEAK-Antikörper ein chimärer, humanisierter oder menschlicher Antikörper ist.

8. Antagonist zur Verwendung in einem Behandlungsverfahren oder Verwendung nach einem der vorangegangenen Ansprüche, wobei der Anti-TWEAK-Rezeptor-Antikörper das menschliche FN14-Rezeptor-Polypeptid bindet, das die Aminosäuresequenz aus Fig. 12 (Seq.-ID Nr. 2) umfasst.

9. Antagonist zur Verwendung in einem Behandlungsverfahren oder Verwendung nach Anspruch 8, wobei der Anti-TWEAK-Rezeptor-Antikörper ein chimärer, humanisierter oder menschlicher Antikörper ist.

## Revendications

1. Molécule antagoniste pour une utilisation dans un procédé de traitement d'un cancer chez un mammifère,
l'antagoniste étant un anticorps anti-TWEAK, un anticorps anti-récepteur de TWEAK et/ou une immunoadhésine de récepteur de TWEAK, et
la molécule antagoniste étant capable de bloquer ou d'interrompre la signalisation intracellulaire du récepteur de TWEAK, renforçant ainsi les réponses de T_{H}1 innées ou l'activité chez un mammifère et en traitant le cancer,
le récepteur de TWEAK étant le récepteur FN14.

2. Utilisation d'une molécule antagoniste pour la préparation d'un médicament pour le traitement d'un cancer chez un mammifère,
l'antagoniste étant un anticorps anti-TWEAK, un anticorps anti-récepteur de TWEAK et/ou une immunoadhésine de récepteur de TWEAK, et
la molécule antgoniste étant capable de bloquer ou d'interrompre la signalisation intracellulaire du récepteur de TWEAK, renforçant ainsi les réponses de T_{H}1 innées ou l'activité chez un mammifère et en traitant le cancer,
le récepteur de TWEAK étant le récepteur FN14.

3. Antagoniste pour une utilisation dans un procédé de traitement ou l'utilisation selon la revendication 1 ou la revendication 2, ledit mammifère étant aussi exposé à une chimiothérapie, un rayonnement, un promédicament, un agent cytotoxique ou un agent inhibiteur de croissance.

4. Antagoniste pour une utilisation dans un procédé de traitement ou l'utilisation selon l'une quelconque des revendications précédentes, ladite immunoadhésine de récepteur de TWEAK comprenant une séquence de récepteur de TWEAK fusionnée à une région Fc d'une immunoglobuline.

5. Antagoniste pour une utilisation dans un procédé de traitement ou l'utilisation selon la revendication 4, ladite séquence de récepteur de TWEAK comprenant une séquence de domaine extracellulaire du récepteur FN14.

6. Antagoniste pour une utilisation dans un procédé de traitement ou l'utilisation selon l'une quelconque des revendications précédentes, ledit anticorps anti-TWEAK se liant au polypeptide TWEAK humain comprenant les acides aminés 94-249 de la figure 11 (SEQ ID n° 1).

7. Antagoniste pour une utilisation dans un procédé de traitement ou l'utilisation selon la revendication 6, ledit anticorps anti-TWEAK étant un anticorps chimérique, humanisé ou humain.

8. Antagoniste pour une utilisation dans un procédé de traitement ou l'utilisation de l'une quelconque des revendications précédentes, ledit anticorps anti-récepteur de TWEAK se liant au polypeptide du récepteur FN14 humain comprenant la séquence d'acides aminés de la figure 12 (SEQ ID n° 2).

9. Antagoniste pour une utilisation dans un procédé de traitement ou l'utilisation selon la revendication 8, ledit anticorps anti-récepteur de TWEAK étant un anticorps chimérique, humanisé ou humain.
